# EUROPEAN PATENT APPLICATION

(11) **EP 4 108 263 A2**
(43) Date of publication of application: **28.12.2022**
(21) Application number: 22176836.9
(22) Date of filing: 01.06.2022
(51) Int. Cl.: A61K 48/00, A61P 21/00, C12N 15/864

(54) **RECOMBINANT ADENO-ASSOCIATED VIRUS PRODUCTS AND METHODS FOR TREATING LIMB GIRDLE MUSCULAR DYSTROPHY 2A**

(30) Priority: 02.06.2021 US 202163196108 P
(71) Applicant: Research Institute at Nationwide Children's Hospital, Columbus, Ohio 43205 (US)
(72) Inventor: SAHENK, Zarife, Columbus, OH (US)
(74) Representative: D Young & Co LLP

(57) **Abstract**

Products and methods for treating limb girdle muscular dystrophy 2A are provided. In the methods, recombinant adeno-associated viruses deliver DNA encoding a protein with calpain 3 activity.

## Description

This application claims priority to U.S. provisional application no. 63/196,108 filed June 2, 2021, which is incorporated by reference herein in its entirety.

### Field

Provided herein are products and methods for treating limb girdle muscular dystrophy 2A. In the methods, recombinant adeno-associated viruses deliver DNA encoding a protein with calpain3 (CAPN3) activity.

### Incorporation by Reference of the Sequence Listing

This application contains, as a separate part of disclosure, a Sequence Listing in computer-readable form (filename: 56524_SeqListing.txt; 43,828 bytes - ASCII text file created May 31, 2022) which is incorporated by reference herein in its entirety.

### Background

Muscular dystrophies (MDs) are a group of genetic diseases. The group is characterized by progressive weakness and degeneration of the skeletal muscles that control movement. Some forms of MD develop in infancy or childhood, while others may not appear until middle age or later. The disorders differ in terms of the distribution and extent of muscle weakness (some forms of MD also affect cardiac muscle), the age of onset, the rate of progression, and the pattern of inheritance.

One group of MDs is the limb girdle group (LGMD) of MDs. LGMDs are rare conditions and they present differently in different people with respect to age of onset, areas of muscle weakness, heart and respiratory involvement, rate of progression and severity. LGMDs can begin in childhood, adolescence, young adulthood or even later. Both genders are affected equally. LGMDs cause weakness in the shoulder and pelvic girdle, with nearby muscles in the upper legs and arms sometimes also weakening with time. Weakness of the legs often appears before that of the arms. Facial muscles are usually unaffected. As the condition progresses, people can have problems with walking and may need to use a wheelchair over time. The involvement of shoulder and arm muscles can lead to difficulty in raising arms over head and in lifting objects. In some types of LGMD, the heart and breathing muscles may be involved.

There are at least nineteen forms of LGMD, and the forms are classified by their associated genetic defects.

| Type | Pattern of Inheritance | Gene or Chromosome |
|---|---|---|
| LGMD1A | Autosomal dominant | Myotilin gene |
| LGMD1B | Autosomal dominant | Lamin A/C gene |
| LGMD1C | Autosomal dominant | Caveolin gene |
| LGMD1D | Autosomal dominant | Chromosome 7 |
| LGMD1E | Autosomal dominant | Desmin gene |
| LGMD1F | Autosomal dominant | Chromosome 7 |
| LGMD1G | Autosomal dominant | Chromosome 4 |
| LGMD2A | Autosomal recessive | Calpain-3 gene |
| LGMD2B | Autosomal recessive | Dysferlin gene |
| LGMD2C | Autosomal recessive | Gamma-sarcoglycan gene |
| LGMD2D | Autosomal recessive | Alpha-sarcoglycan gene |
| LGMD2E | Autosomal recessive | Beta-sarcoglycan gene |
| LGMD2F | Autosomal recessive | Delta-sarcoglycan gene |
| LGMD2G | Autosomal recessive | Telethonin gene |
| LGMD2H | Autosomal recessive | TRIM32 |
| LGMD21 | Autosomal recessive | FKRP gene |
| LGMD2J | Autosomal recessive | Titin gene |
| LGMD2K | Autosomal recessive | POMT1 gene |
| LGMD2L | Autosomal recessive | Fukutin gene |

Specialized tests for LGMD are now available through a national scheme for diagnosis, the National Commissioning Group (NCG).

Mutations in calpain3 gene (CAPN3) lead to one of the most common limb-girdle muscular dystrophies worldwide, LGMD2A. At present, there is no treatment for this inherited disease. Previous studies have demonstrated the potential for CAPN3 gene transfer to correct the pathological signs in CAPN3-deficient mice. However expression of CAPN3 driven by the desmin promoter resulted in cardiotoxicity (Bartoli et al., Mol. Ther., 13: 250-259 (2006)). In follow up studies, skeletal muscle expression of the gene was studied (Roudaut et al., Circulation, 128: 1094-1104 (2013)).

There remains a need in the art for treatments for LGMD2A.

### Summary

Methods and products for delivering DNA encoding a protein with calpain3 (CAPN3) activity are provided herein. Such methods and products can be used to treat various diseases, for example, LGMD2A.

In addition, methods of treating muscular dystrophy in a subject are provided, wherein the method comprises administering to the subject a recombinant adeno-associated virus (rAAV) comprising a gene encoding a protein that increases muscular force and/or increases muscular mass, and wherein the treatment results in an increase in the levels of peroxisome proliferator activated receptor γ coactivator 1 alpha (PGC1α) compared to a control level of PGC1α. The method further comprises a step of measuring the level of PGC1α nucleic acid or PGC1α protein. In other embodiments, the level of PGC1α may be detected by measuring expression of PGC1α nucleic acid by PCR, Northern blot or Southern blot or any other method to detect PGC1α nucleic acids. In other embodiments, the level of PGC1α protein may be measured by immunohistochemistry, Western blot, ELISA or any methods of detecting PGC1α protein.

Provided are methods of increasing expression of peroxisome proliferator activated receptor γ coactivator 1 alpha (PGC1α) in a subject comprising administering to the subject a recombinant adeno-associated virus (rAAV), wherein the rAAV comprises a polynucleotide encoding a protein that increases muscular force and/or increases muscular mass, and wherein administration of the rAAV results in an increase in the level of PGC1α compared to a control. The method results in a switch from fast-twitch glycolytic (FTG) fibers to a slow-twitch oxidative (STO) fibers in the subject. The methods may further comprise the step of measuring the level of PGC1α in the subject. The level of PGC1α may be detected by measuring expression of PGC1α nucleic acid by PCR, Northern blot or Southern blot or any other method to detect PGC1α nucleic acids. In other embodiments, the level of PGC1α protein may be measured by immunohistochemistry, Western blot, ELISA or any methods of detecting PGC1α protein.

Also provided are methods of treating muscular dystrophy in a subject, comprising administering to the subject an rAAV comprising a polynucleotide encoding a protein that increases muscular force and/or increases muscular mass, and measuring the level of peroxisome proliferator activated receptor γ coactivator 1 alpha (PGC1α) in a sample obtained from the subject after administration of a rAAV, wherein a change in PGC1α level compared to a control level of PGC1α is indicative of a therapeutic effect of the rAAV and/or the muscle function of the subject In some embodiments, the change in PGC1α is an increase in PGC1α compared to a control level of PGC1α, which is indicative of an therapeutic effect of administering the rAAV. For example, an increase in PGC1α level is indicative of improved muscle function.

In addition, the methods optionally comprise the step of obtaining the sample from the subject before and/or after administration of the rAAV. The control level of PGC1α is the level of PGC1α in the subject prior to administration of the rAAV, or a known standard level of PGC1. The level of PGC1α may be detected by measuring expression of PGC1α nucleic acid by PCR, Northern blot or Southern blot or any other method to detect PGC1α nucleic acids. In other embodiments, the level of PGC1α protein may be measured by immunohistochemistry, Western blot, ELISA or any methods of detecting PGC1α protein.

In any of the methods provided, the control level of PGC1α refers to the level of PGC1α in the subject prior to administration of the rAAV, e.g. the control level may also be referred to as the "basal level". In addition, the control level of PGC1α is a known standard level of PGC1α for a healthy or normal age-matched subject.

In addition, the disclosure provides for a recombinant adeno-associated virus (rAAV) for treating muscular dystrophy in a subject, wherein the rAAV comprises a gene encoding a protein that increases muscular force and/or increases muscular mass, and administration of the rAAV results in an increase in the levels of peroxisome proliferator activated receptor γ coactivator 1 alpha (PGC1α) compared to a control level of PGC1α. The level of PGC1α nucleic acid or PGC1α protein may be measured after administration of the rAAV. For example, the level of PGC1α may be detected by measuring expression of PGC1α nucleic acid by PCR, Northern blot or Southern blot or any other method to detect PGC1α nucleic acids. In other embodiments, the level of PGC1α protein may be measured by immunohistochemistry, Western blot, ELISA or any methods of detecting PGC1α protein.

Also provided are rAAV for increasing expression of peroxisome proliferator activated receptor γ coactivator 1 alpha (PGC1α) in a subject, wherein the rAAV comprises a polynucleotide encoding a protein that increases muscular force and/or increases muscular mass, and wherein administration of the rAAV results in an increase in the level of PGC1α compared to a control. Administration of the rAAV results in a switch from fast-twitch glycolytic (FTG) fibers to a slow-twitch oxidative (STO) fibers in the subject.

The disclosure also provides for a recombinant adeno-associated virus (rAAV) for treating muscular dystrophy in a subject, wherein the rAAV comprises a gene encoding a protein that increases muscular force and/or increases muscular mass, and wherein administration of the rAAV results in an increase in the levels of peroxisome proliferator activated receptor γ coactivator 1 alpha (PGC1α) as compared to a control level of PGC1α, wherein a change in the PGC1α level is indicative of a therapeutic effect of the rAAV and/or the muscle function of the subject. Administration of any of the disclosed rAAV may result in an an increase in PGC1α level, which is indicative of improved muscle function. In addition, administration of any of the disclosed rAAV may result in a change in PGC1α, which is determined in a sample obtained before and/or after administering the rAAV to the subject. In any of the methods provided, the control level of PGC1α refers to the level of PGC1α in the subject prior to administration of the rAAV, e.g. the control level may also be referred to as the "basal level". In addition, the control level of PGC1α is a known standard level of PGC1α for a healthy or normal age-matched subject.

In any of the provided rAAV for treating muscular dystrophy, the control level of PGC1α refers to the level of PGC1α in the subject prior to administration of the rAAV, e.g. the control level may also be referred to as the "basal level". In addition, the control level of PGC1α is a known standard level of PGC1α for a healthy or normal age-matched subject.

In addition, the disclosure provides for a use of a recombinant adeno-associated virus (rAAV) the preparation of a medicament for treating muscular dystrophy in a subject, wherein the rAAV comprises a gene encoding a protein that increases muscular force and/or increases muscular mass, and administration of the rAAV results in an increase in the levels of peroxisome proliferator activated receptor γ coactivator 1 alpha (PGC1α) compared to a control level of PGC1α. The level of PGC1α nucleic acid or PGC1α protein may be measured after administration of the rAAV. For example, the level of PGC1α may be detected by measuring expression of PGC1α nucleic acid by PCR, Northern blot or Southern blot or any other method to detect PGC1α nucleic acids. In other embodiments, the level of PGC1α protein may be measured by immunohistochemistry, Western blot, ELISA or any methods of detecting PGC1α protein.

Also provided is use of an rAAV for the preparation of a medicament for increasing expression of peroxisome proliferator activated receptor γ coactivator 1 alpha (PGC1α) in a subject, wherein the rAAV comprises a polynucleotide encoding a protein that increases muscular force and/or increases muscular mass, and wherein administration of the rAAV results in an increase in the level of PGC1α compared to a control. Administration of the rAAV results in a switch from fast-twitch glycolytic (FTG) fibers to a slow-twitch oxidative (STO) fibers in the subject.

In addition, the disclosure provides for use of a rAAV for the preparation of a medicament for treating muscular dystrophy in a subject, wherein the rAAV comprises a gene encoding a protein that increases muscular force and/or increases muscular mass, and wherein administration of the rAAV results in an increase in the levels of peroxisome proliferator activated receptor γ coactivator 1 alpha (PGC1α) as compared to a control level of PGC1α, wherein a change in the PGC1α level is indicative of a therapeutic effect of the rAAV and/or the muscle function of the subject. In any of the disclosed uses, an increase in PGC1α level is indicative of improved muscle function. In addition, in any of the disclosed uses, the change in PGC1α is determined in a sample obtained before and/or after administering the rAAV to the subject.

In any of the provided uses for the preparation of a medicament for treating muscular dystrophy, the control level of PGC1α refers to the level of PGC1α in the subject prior to administration of the rAAV, e.g. the control level may also be referred to as the "basal level". In addition, the control level of PGC1α is a known standard level of PGC1α for a healthy or normal age-matched subject.

In any of the provided methods, rAAV and uses, the protein that increases muscular force and/or increases muscular mass is microdystrophin, dystrophin, calpain 3 (CAPN3), dysferlin (DYSF), SGCGA (α-sarcoglycan ), SGCB (β-sarcoglycan ), γ-sarcoglycan, delta-sarcoglycan, telethonin ancotamin 5 (ANO5), galgt2, myotilin, lamin A/C, caveolin, desmin, telethonin, FKRP, titin, POMT1 or fukutin.

In any of the provided methods, rAAV and uses, the muscular dystrophy is Duchenne Muscular Dystrophy (DMD); Becker Muscular Dystrophy; Congenital Muscular Dystrophy (MDC) 1A, 1B, 1C and 1D; Limb Girdle Muscular Dystrophy (LGMD) 1A, 1B, 1C, 1D, 1E, 1F, 1G, 1H, 2A, 2B, 2C, 2D, 2E, 2F, 2G 2H, 2I, 2J, 2K, 2L, 2M, 2N, 2O and 2Q; Ullrich Congenital Muscular Dystrophy; Fukuyama Congenital Muscular Dystrophy; Myotonic Dystrophy; Emery Dreifuss Muscular Dystrophy; Distal Muscular Dystrophy; Centronuclear; and Faciosacpulohumeral Muscular Dystrophy.

In addition, in any of the methods, the rAAV are administered by intramuscular injection or intravenous injection, or any of the rAAV are formulated for administration by intramuscular injection or intravenous injection. In addition, any of the rAAV and medicaments are formulated for administration by intramuscular injection or intravenous injection.

The disclosure also provides for a method of increasing expression of peroxisome proliferator activated receptor γ coactivator 1 alpha (PGC1α) in a subject suffering from limb girdle muscular dystrophy 2A comprising administering to the subject a recombinant adeno-associated virus (rAAV), wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein the administration of the rAAV results in an increase in PGC1α compared to a control level of PGC1α. The control level of PGC1α is the level of PGC1α in the subject prior to administration of the rAAV, or a known standard level of PGC1. Optionally, the methods further comprise a step of measuring the level of PGC1α in the subject before and/or after administration of the rAAV. The level of PGC1α may be detected by measuring expression of PGC1α nucleic acid by PCR, Northern blot or Southern blot or any other method to detect PGC1α nucleic acids. In other embodiments, the level of PGC1α protein may be measured by immunohistochemistry, Western blot, ELISA or any methods of detecting PGC1α protein. For example, the method comprises administering a therapeutically effective amount of the rAAV. In addition, the method results in a switch from fast-twitch glycolytic (FTG) fibers to a slow-twitch oxidative (STO) fibers in the subject.

The disclosure also provides for methods of treating limb girdle muscular dystrophy 2A in a subject comprising administering to the subject a recombinant adeno-associated virus (rAAV), wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein the administration of the rAAV results in an improved performance or improved phenotype in the subject compared to performance or phenotype prior to administration of the rAAV, wherein the improved performance is one or more of i) an increase in muscle contractibility, or ii) an improved performance in the run to exhaustion test, and wherein the improved phenotype is one or more of i) an increase in muscle fiber diameter, ii) an increase in number of oxidative muscle fibers, iii) a switch from fast-twitch glycolytic (FTG) fibers to a slow-twitch oxidative (STO) fibers, or iv) an increase in the percentage of oxidative muscle fibers.

The disclosure provides for methods of treating limb girdle muscular dystrophy 2A in a subject comprising administering to the subject a recombinant adeno-associated virus (rAAV), wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein administration of rAAV results in an increase in Capn3 mRNA expression of at least 10% compared with the level of Capn3 mRNA expression prior to administration of the rAAV and wherein an increase of at least 10% is indicative of an increase in performance or phenotype in the subject. The increase Capn3 mRNA expression of at least 10% compared with the level of Capn3 mRNA expression prior to administration of the rAAV is a threshold that is indicative of improvement in performance or phenotype in the subject. The threshold may be at least about 10%, or at least about 11%, or at least about 12%, or at least about 13%, or at least about 14%, or at least about 15%, or at least about 16%, or at least about 17%, or at least about 18%, or at least about 19%, or at least about 20%, or at least about 25%, or at least about 30%, or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%.

For example, in any of the methods, the improved performance is an increase in muscle contractibility compared to a control, such as at least about 20% increase in maximum twitch response, or at least about 21% increase in maximum twitch response, or at least about 22% increase in maximum twitch response, or at least about 23% increase in maximum twitch response, or at least about 24% increase in maximum twitch response, or at least about 25% increase in maximum twitch response, or at least about 26% increase in maximum twitch response, or at least about 27% increase in maximum twitch response, or at least about 28% increase in maximum twitch response, or at least about 29% increase in maximum twitch response, or at least about 30% increase in maximum twitch response, or at least about 40% increase in maximum twitch response, or at least about 50% increase in maximum twitch response, or at least about 60% increase in maximum twitch response, or at least about 70% increase in maximum twitch response, or at least about 80% increase in maximum twitch response or at least about 90% increase in maximum twitch response

For example, in any of the methods, the improved performance is an increase in muscle contractibility compared to a control, such as at least about 10% increase in maximum tetanic response, or at least about 11% increase in maximum tetanic response, or at least about 12% increase in maximum tetanic response, or at least about 13% increase in maximum tetanic response, or at least about 14% increase in maximum tetanic response, or at least about 15% increase in maximum tetanic response, or at least about 16% increase in maximum tetanic response, or at least about 17% increase in maximum tetanic response, or at least about 18% increase in maximum tetanic response, or at least about 19% increase in maximum tetanic response, or at least about 20% increase in maximum tetanic response, or at least about 30% increase in maximum tetanic response, or at least about 40% increase in maximum tetanic response, or at least about 50% increase in maximum tetanic response, or at least about 60% increase in maximum tetanic response, or at least about 70% increase in maximum tetanic response, or at least about 80% increase in maximum tetanic response or at least about 90% increase in maximum tetanic response.

For example, in any of the methods, the improved performance is an improvement in the run to exhaustion test, such as at least about 70% improvement in the treadmill run to exhaustion test, at least about 71% improvement in the treadmill run to exhaustion test, at least about 72% improvement in the treadmill run to exhaustion test, at least about 73% improvement in the treadmill run to exhaustion test, at least about 74% improvement in the treadmill run to exhaustion test, at least about 75% improvement in the treadmill run to exhaustion test, at least about 76% improvement in the treadmill run to exhaustion test, at least about 77% improvement in the treadmill run to exhaustion test, at least about 78% improvement in the treadmill run to exhaustion test, at least about 79% improvement in the treadmill run to exhaustion test, at least about 80% improvement in the treadmill run to exhaustion test, at least about 85% improvement in the treadmill run to exhaustion test, at least about 90% improvement in the treadmill run to exhaustion test or at least about 95% improvement in the treadmill run to exhaustion test.

For example, in any of the methods, the improved performance is an improvement in the distance to run to exhaustion test, such as at least about 100% improvement in the distance to run to exhaustion test, at least about 110% improvement in the distance to run to exhaustion test, at least about 120% improvement in the distance to run to exhaustion test, at least about 130% improvement in the distance to run to exhaustion test, at least about 140% improvement in the distance to run to exhaustion test, at least about 150% improvement in the distance to run to exhaustion test, at least about 160% improvement in the distance to run to exhaustion test, at least about 170% improvement in the distance to run to exhaustion test, at least about 180% improvement in the distance to run to exhaustion test, at least about 190% improvement in the distance to run to exhaustion test, or at least about 200% improvement in the distance to run to exhaustion test.

In any of the methods, the muscle fiber comprises one or more of slow twitch oxidative (STO) muscle fiber, fast twitch oxidative (FTO) muscle fiber, and fast twitch glycolytic (FTG) fiber.

In addition, in any of the methods the rAAV are administered by intramuscular injection or intravenous injection.

The disclosure also provides rAAV or a composition comprising a recombinant adeno-associated virus (rAAV) for increasing expression of peroxisome proliferator activated receptor γ coactivator 1 alpha (PGC1α) in a subject suffering from limb girdle muscular dystrophy 2A, wherein the rAAV comprises a polynucleotide which comprises a first AAV inverted terminal repeat (ITR), a promoter, a nucleotide sequence encoding a protein with calpain 3 (CAPN3) activity and a second AAV ITR. For example, administration of the rAAV results in an increase in peroxisome proliferator activated receptor γ coactivator 1 alpha (PGC1α) level compared to a control level of PGC1α. The control level of PGC1α is the level of PGC1α in the subject prior to administration of the rAAV, or a known standard level of PGC1. The level of PGC1α may be detected by measuring expression of PGC1α nucleic acid by PCR, Northern blot or Southern blot or any other method to detect PGC1α nucleic acids. In other embodiments, the level of PGC1α protein may be measured by immunohistochemistry, Western blot, ELISA or any methods of detecting PGC1α protein.

In another embodiment, the disclosure provides for recombinant adeno-associated virus (rAAV) or a composition comprising a rAAV for treating limb girdle muscular dystrophy 2A in a subject, wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein administration of the rAAV results in an improved performance or improved phenotype in the subject compared to performance or phenotype prior to administration of the rAAV, wherein the improved performance is one or more of i) an increase in muscle contractibility, or ii) an improved performance in the run to exhaustion test, and wherein the improved phenotype is one or more of i) an increase in muscle fiber diameter, ii) an increase in number of oxidative muscle fibers, iii) a switch from fast-twitch glycolytic (FTG) fibers to a slow-twitch oxidative (STO) fibers, or iv) an increase in the percentage of oxidative muscle fibers.

The disclosure provides for recombinant adeno-associated virus (rAAV) or compositions comprising a rAAV for treating limb girdle muscular dystrophy 2A in a subject, wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein administration of the rAAV results in an increase in Capn3 mRNA expression of at least 10% compared with the level of Capn3 mRNA expression prior to administration of the rAAV and wherein an increase at least 10% is indicative of an increase in performance or phenotype in the subject. The increase Capn3 mRNA expression of at least 10% compared with the level of Capn3 mRNA expression prior to administration of the rAAV is a threshold that is indicative of improvement in performance or phenotype in the subject. The threshold may be at least about 10%, or at least about 11%, or at least about 12%, or at least about 13%, or at least about 14%, or at least about 15%, or at least about 16%, or at least about 17%, or at least about 18%, or at least about 19%, or at least about 20%, or at least about 25%, or at least about 30%, or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%.

For example, in any of the rAAV or compositions, the improved performance is an increase in muscle contractibility compared to a control, such as at least about 20% increase in maximum twitch response, or at least about 21% increase in maximum twitch response, or at least about 22% increase in maximum twitch response, or at least about 23% increase in maximum twitch response, or at least about 24% increase in maximum twitch response, or at least about 25% increase in maximum twitch response, or at least about 26% increase in maximum twitch response, or at least about 27% increase in maximum twitch response, or at least about 28% increase in maximum twitch response, or at least about 29% increase in maximum twitch response, or at least about 30% increase in maximum twitch response, or at least about 40% increase in maximum twitch response, or at least about 50% increase in maximum twitch response, or at least about 60% increase in maximum twitch response, or at least about 70% increase in maximum twitch response, or at least about 80% increase in maximum twitch response or at least about 90% increase in maximum twitch response.

For example, in any of the rAAV or compositions, the improved performance is an increase in muscle contractibility compared to a control, such as at least about 10% increase in maximum tetanic response, or at least about 11% increase in maximum tetanic response, or at least about 12% increase in maximum tetanic response, or at least about 13% increase in maximum tetanic response, or at least about 14% increase in maximum tetanic response, or at least about 15% increase in maximum tetanic response, or at least about 16% increase in maximum tetanic response, or at least about 17% increase in maximum tetanic response, or at least about 18% increase in maximum tetanic response, or at least about 19% increase in maximum tetanic response, or at least about 20% increase in maximum tetanic response, or at least about 30% increase in maximum tetanic response, or at least about 40% increase in maximum tetanic response, or at least about 50% increase in maximum tetanic response, or at least about 60% increase in maximum tetanic response, or at least about 70% increase in maximum tetanic response, or at least about 80% increase in maximum tetanic response or at least about 90% increase in maximum tetanic response.

For example, in any of the rAAV or compositions, the improved performance is an improvement in the run to exhaustion test, such as at least about 70% improvement in the treadmill run to exhaustion test, at least about 71% improvement in the treadmill run to exhaustion test, at least about 72% improvement in the treadmill run to exhaustion test, at least about 73% improvement in the treadmill run to exhaustion test, at least about 74% improvement in the treadmill run to exhaustion test, at least about 75% improvement in the treadmill run to exhaustion test, at least about 76% improvement in the treadmill run to exhaustion test, at least about 77% improvement in the treadmill run to exhaustion test, at least about 78% improvement in the treadmill run to exhaustion test, at least about 79% improvement in the treadmill run to exhaustion test, at least about 80% improvement in the treadmill run to exhaustion test, at least about 85% improvement in the treadmill run to exhaustion test, at least about 90% improvement in the treadmill run to exhaustion test or at least about 95% improvement in the treadmill run to exhaustion test.

For example, in any of the rAAV or compositions, the improved performance is an improvement in the distance to run to exhaustion test, such as at least about 100% improvement in the distance to run to exhaustion test, at least about 110% improvement in the distance to run to exhaustion test, at least about 120% improvement in the distance to run to exhaustion test, at least about 130% improvement in the distance to run to exhaustion test, at least about 140% improvement in the distance to run to exhaustion test, at least about 150% improvement in the distance to run to exhaustion test, at least about 160% improvement in the distance to run to exhaustion test, at least about 170% improvement in the distance to run to exhaustion test, at least about 180% improvement in the distance to run to exhaustion test, at least about 190% improvement in the distance to run to exhaustion test, or at least about 200% improvement in the distance to run to exhaustion test.

In any of the rAAV or compositions, the muscle fiber comprises one or more of slow twitch oxidative (STO) muscle fiber, fast twitch oxidative (FTO) muscle fiber, and fast twitch glycolytic (FTG) fiber.

In addition, any of the rAAV or compositions are formulated for administration by intramuscular injection or intravenous injection.

The disclosure also provides use of a recombinant adeno-associated virus (rAAV) for preparation of a medicament for increasing expression of peroxisome proliferator activated receptor γ coactivator 1 alpha (PGC1α) in a subject suffering from limb girdle muscular dystrophy 2A, wherein the rAAV comprises a polynucleotide which comprises a first AAV inverted terminal repeat (ITR), a promoter, a nucleotide sequence encoding a protein with calpain 3 (CAPN3) activity and a second AAV ITR. For example, administration of the rAAV results in an increase in peroxisome proliferator activated receptor γ coactivator 1 alpha (PGC1α) level compared to a control level of PGC1α. The control level of PGC1α is the level of PGC1α in the subject prior to administration of the rAAV, or a known standard level of PGC1α. The level of PGC1α may be detected by measuring expression of PGC1α nucleic acid by PCR, Northern blot or Southern blot or any other method to detect PGC1α nucleic acids. In other embodiments, the level of PGC1α protein may be measured by immunohistochemistry, Western blot, ELISA or any methods of detecting PGC1α protein.

In another embodiment, the disclosure provides for use of a recombinant adeno-associated virus (rAAV) for preparation of a medicament for treating limb girdle muscular dystrophy 2A in a subject, wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein administration of the rAAV results in an improved performance or improved phenotype in the subject compared to performance or phenotype prior to administration of the rAAV, wherein the improved performance is one or more of i) an increase in muscle contractibility, or ii) an improved performance in the run to exhaustion test, and wherein the improved phenotype is one or more of i) an increase in muscle fiber diameter, ii) an increase in number of oxidative muscle fibers, iii) a switch from fast-twitch glycolytic (FTG) fibers to a slow-twitch oxidative (STO) fibers, or iv) an increase in the percentage of oxidative muscle fibers.

The disclosure provides for use of a rAAV for preparation of a medicament comprising a rAAV for treating limb girdle muscular dystrophy 2A in a subject, wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein administration of the rAAV results in an increase in Capn3 mRNA expression of at least 10% compared with the level of Capn3 mRNA expression prior to administration of the rAAV and wherein an increase of at least 10% is indicative of an increase in performance or phenotype in the subject. The increase Capn3 mRNA expression of at least 10% compared with the level of Capn3 mRNA expression prior to administration of the rAAV is a threshold that is indicative of improvement in performance or phenotype in the subject. The threshold may be at least about 10%, or at least about 11%, or at least about 12%, or at least about 13%, or at least about 14%, or at least about 15%, or at least about 16%, or at least about 17%, or at least about 18%, or at least about 19%, or at least about 20%, or at least about 25%, or at least about 30%, or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%.

For example, in any disclosed use of a rAAV for preparation of a medicament, the improved performance is an increase in muscle contractibility compared to a control, such at least about 20% increase in maximum twitch response, or at least about 21% increase in maximum twitch response, or at least about 22% increase in maximum twitch response, or at least about 23% increase in maximum twitch response, or at least about 24% increase in maximum twitch response, or at least about 25% increase in maximum twitch response, or at least about 26% increase in maximum twitch response, or at least about 27% increase in maximum twitch response, or at least about 28% increase in maximum twitch response, or at least about 29% increase in maximum twitch response, or at least about 30% increase in maximum twitch response, or at least about 40% increase in maximum twitch response, or at least about 50% increase in maximum twitch response, or at least about 60% increase in maximum twitch response, or at least about 70% increase in maximum twitch response, or at least about 80% increase in maximum twitch response or or at least about 90% increase in maximum twitch response.

For example, in any disclosed use of a rAAV for preparation of a medicament, the improved performance is an increase in muscle contractibility compared to a control, such at least about 10% increase in maximum tetanic response, or at least about 11% increase in maximum tetanic response, or at least about 12% increase in maximum tetanic response, or at least about 13% increase in maximum tetanic response, or at least about 14% increase in maximum tetanic response, or at least about 15% increase in maximum tetanic response, or at least about 16% increase in maximum tetanic response, or at least about 17% increase in maximum tetanic response, or at least about 18% increase in maximum tetanic response, or at least about 19% increase in maximum tetanic response, or at least about 20% increase in maximum tetanic response, or at least about 30% increase in maximum tetanic response, or at least about 40% increase in maximum tetanic response, or at least about 50% increase in maximum tetanic response, or at least about 60% increase in maximum tetanic response, or at least about 70% increase in maximum tetanic response, or at least about 80% increase in maximum tetanic response or or at least about 90% increase in maximum tetanic response.

For example, in any disclosed use of a rAAV for preparation of a medicament, the improved performance is an improvement in the run to exhaustion test, such as at least about 70% improvement in the treadmill run to exhaustion test, at least about 71% improvement in the treadmill run to exhaustion test, at least about 72% improvement in the treadmill run to exhaustion test, at least about 73% improvement in the treadmill run to exhaustion test, at least about 74% improvement in the treadmill run to exhaustion test, at least about 75% improvement in the treadmill run to exhaustion test, at least about 76% improvement in the treadmill run to exhaustion test, at least about 77% improvement in the treadmill run to exhaustion test, at least about 78% improvement in the treadmill run to exhaustion test, at least about 79% improvement in the treadmill run to exhaustion test, at least about 80% improvement in the treadmill run to exhaustion test, at least about 85% improvement in the treadmill run to exhaustion test, at least about 90% improvement in the treadmill run to exhaustion test or at least about 95% improvement in the treadmill run to exhaustion test.

For example, in any disclosed use of a rAAV for preparation of a medicament, the improved performance is an improvement in the distance to run to exhaustion test, such as at least about 100% improvement in the distance to run to exhaustion test, at least about 110% improvement in the distance to run to exhaustion test, at least about 120% improvement in the distance to run to exhaustion test, at least about 130% improvement in the distance to run to exhaustion test, at least about 140% improvement in the distance to run to exhaustion test, at least about 150% improvement in the distance to run to exhaustion test, at least about 160% improvement in the distance to run to exhaustion test, at least about 170% improvement in the distance to run to exhaustion test, at least about 180% improvement in the distance to run to exhaustion test, at least about 190% improvement in the distance to run to exhaustion test, or at least about 200% improvement in the distance to run to exhaustion test.

In any of the disclosed uses, the muscle fiber comprises one or more of slow twitch oxidative (STO) muscle fiber, fast twitch oxidative (FTO) muscle fiber, and fast twitch glycolytic (FTG) fiber.

In addition, any of the disclosed uses the rAAV or medicaments comprising a rAAV are formulated for administration by intramuscular injection or intravenous injection.

Any of the methods, compositions or medicaments disclosed herein may administer and/or comprise a recombinant adeno-associated viruses (rAAVs) encoding a protein with calpain 3 (CAPN3) activity. In addition, any of the disclosed rAAV, compositions comprising a rAAV or medicament comprise a recombinant adeno-associated viruses (rAAVs) encoding a protein with calpain 3 (CAPN3) activity. For example, the recombinant adeno-associated viruses comprise a polynucleotide that comprises a nucleotide sequence encoding the protein with CAPN3 activity. The nucleotide sequence encoding the protein with CAPN3 activity, for example, is at least 90% identical to SEQ ID NO: 2 or comprises the sequence of SEQ ID NO: 2.

For example, the provided rAAV comprise a polynucleotide which comprises a first AAV inverted terminal repeat (ITR), a promoter, a nucleotide sequence encoding a protein with calpain 3 (CAPN3) activity and a second AAV ITR. The nucleotide sequence encoding the protein with CAPN3 activity, for example, is at least 90% identical to SEQ ID NO: 2, or at least 91% identical to SEQ ID NO: 2, at least 92% identical to SEQ ID NO: 2, at least 93% identical to SEQ ID NO: 2, at least 94% identical to SEQ ID NO: 2, at least 95% identical to SEQ ID NO: 2, at least 96% identical to SEQ ID NO: 2, at least 97% identical to SEQ ID NO: 2, at least 98% identical to SEQ ID NO: 2, or at least 99% identical to SEQ ID NO: 2. The rAAV comprises a nucleotide sequence encoding a protein with CAPN3 activity comprises the sequence of SEQ ID NO: 2.

In addition, the provided rAAV comprises a nucleotide sequence encoding a protein with CAPN3 activity that comprises an amino acid sequence that is at least 90% identical to SEQ ID NO:5, at least 91% identical to SEQ ID NO:5, at least 92% identical to SEQ ID NO:5, at least 93% identical to SEQ ID NO:5, at least 94% identical to SEQ ID NO:5, at least 95% identical to SEQ ID NO:5, at least 96% identical to SEQ ID NO:5, at least 97% identical to SEQ ID NO:5, at least 98% identical to SEQ ID NO:5, or at least 99% identical to SEQ ID NO: 5. The rAAV comprises a nucleotide sequence encoding a protein with CAPN3 activity comprising the amino acid sequence of SEQ ID NO: 5.

The provided rAAV comprise a polynucleotide sequence which is at least 90% identical to SEQ ID NO: 1, at least 91% identical to SEQ ID NO: 1, at least 92% identical to SEQ ID NO: 1, at least 93% identical to SEQ ID NO: 1, at least 94% identical to SEQ ID NO: 1, at least 95% identical to SEQ ID NO: 1, at least 96% identical to SEQ ID NO: 1, at least 97% identical to SEQ ID NO: 1, at least 98% identical to SEQ ID NO: 1, or at least 99% identical to SEQ ID NO: 1. The rAAV comprises a polynucleotide sequence of SEQ ID NO: 1.

The provided rAAV comprise a polynucleotide sequence which is at least 90% identical to nucleotides 1 to 3977 of SEQ ID NO: 13, at least 91% identical nucleotides 1 to 3977 of SEQ ID NO: 13, at least 92% identical nucleotides 1 to 3977 of SEQ ID NO: 13, at least 93% identical to nucleotides 1 to 3977 of SEQ ID NO: 13, at least 94% identical to nucleotides 1 to 3977 of SEQ ID NO: 13, at least 95% identical to nucleotides 1 to 3977 of SEQ ID NO: 13, at least 96% identical to nucleotides 1 to 3977 of SEQ ID NO: 13, at least 97% identical to nucleotides 1 to 3977 of SEQ ID NO: 13, at least 98% identical to nucleotides 1 to 3977 of SEQ ID NO: 13, or at least 99% identical to nucleotides 1 to 3977 of SEQ ID NO: 13. The rAAV comprises a polynucleotide sequence of nucleotides 1 to 3977 of SEQ ID NO: 13.

The provided rAAV comprise a polynucleotide sequence which is at least 90% identical to nucleotides 1 to 3977 of SEQ ID NO: 20, at least 91% identical nucleotides 1 to 3977 of SEQ ID NO: 20, at least 92% identical nucleotides 1 to 3977 of SEQ ID NO: 20, at least 93% identical to nucleotides 1 to 3977 of SEQ ID NO: 20, at least 94% identical to nucleotides 1 to 3977 of SEQ ID NO: 20, at least 95% identical to nucleotides 1 to 3977 of SEQ ID NO: 20, at least 96% identical to nucleotides 1 to 3977 of SEQ ID NO: 20, at least 97% identical to nucleotides 1 to 3977 of SEQ ID NO: 20, at least 98% identical to nucleotides 1 to 3977 of SEQ ID NO: 20, or at least 99% identical to nucleotides 1 to 3977 of SEQ ID NO: 20. The rAAV comprises a polynucleotide sequence of nucleotides 1 to 3977 of SEQ ID NO: 20.

The nucleotide sequence, in one embodiment, is under the transcription control of a muscle-specific promoter. For example, the muscle-specific promoter comprises one or more of a human skeletal actin gene element, a cardiac actin gene element, a desmin promoter, a skeletal alpha-actin (ASKA) promoter, a troponin I (TNNI2) promoter, a myocyte-specific enhancer binding factor mef binding element, a muscle creatine kinase (MCK) promoter, a truncated MCK (tMCK) promoter, a myosin heavy chain (MHC) promoter, a hybrid a-myosin heavy chain enhancer-/MCK enhancer-promoter (MHCK7) promoter, a C5-12 promoter, a murine creatine kinase enhancer element, a skeletal fast-twitch troponin c gene element, a slow-twitch cardiac troponin c gene element, a slow-twitch troponin i gene element, hypoxia-inducible nuclear factor (HIF)-response element (HRE), a steroid-inducible element, and a glucocorticoid response element (gre). In one embodiment, the muscle-specific promoter is a tMCK promoter, which comprises a sequence of SEQ ID NO: 3. In another embodiment, the muscle-specific promoter is MHCK7 promoter, which comprises a sequence of SEQ ID NO: 4.

For example, the rAAV comprises a polynucleotide which comprises, in one embodiment, a first AAV inverted terminal repeat (ITR), a tMCK promoter, the nucleotide sequence encoding the protein with calpain 3 activity, and a second AAV inverted terminal repeat (ITR). The AAV ITR (e.g., the first and/or second AAV ITRs) is, for example, an AAV2 inverted terminal repeat. The capsid proteins of the rAAV comprise, for example, an AAV rh.74 capsid protein or an AAV9 capsid protein.

The provided rAAV comprises one or more of AAV-1, AAV-2, AAV-3, AAV-4, AAV-5, AAV-6, AAV-7, AAV-8, AAV-9, AAV-10, AAV-11, AAV-12, AAV-13, AAV rh.74 and AAV rh.10 capsid proteins.

In another embodiment, compositions comprising any of the disclosed rAAV are provided. For example, the compositions are formulated for intramuscular injection or intravenous injection.

In any of the methods, compositions or uses of a therapeutically effective amount of any of the disclosed rAAV or a composition provided, after treatment with the rAAV, medicament or composition, the heart muscle of the subject shows no, minimum or low calpain 3 protein expressed from the disclosed rAAV, medicament or composition.

In any of the methods, rAAV, compositions or uses of the disclosure, the subject is an older adult subject. In some embodiments, the subject is an adult (18 years or older). In particular, the subject is a young adult (18-39 years of age), middle aged adult (40-64 year of age) or an older adult or elderly subject (over 65 years of age) or a geriatric subject (over 70 years of age).

The disclosure also provides for a polynucleotide comprising the nucleotide sequence of that is at least 90% 95% or 99% identical to the nucleotide sequence of SEQ ID NO: 13 or SEQ ID NO: 20. For example, the disclosure provides for a polynucleotide comprising the nucleotide sequence of SEQ ID NO: 13 or SEQ ID NO: 20.

The disclosure also provides for a polynucleotide comprising a nucleotide sequence that is at least 90%, 95% or 99% identical to the nucleotide sequence comprising nucleotides 1 to 3977 of SEQ ID NO: 13 or nucleotides 1 to 3977 of SEQ ID NO: 20. For example, the disclosure provides for a polynucleotide sequence comprising nucleotides 1 to 3977 of SEQ ID NO: 13 or nucleotides 1 to 3977 of SEQ ID NO: 20.

The disclosure also provides for recombinant AAV (rAAV) comprising a nucleotide sequence that is at least 90%, 95% or 99% identical to the nucleotide sequence comprising nucleotides 1 to 3977 of SEQ ID NO: 13 or nucleotides 1 to 3977 of SEQ ID NO: 20. For example, the rAAV comprises a polynucleotide sequence comprising nucleotides 1 to 3977 of SEQ ID NO: 13 or nucleotides 1 to 3977 of SEQ ID NO: 20. Any of the disclosed rAAV comprises one or more of AAV-1, AAV-2, AAV-3, AAV-4, AAV-5, AAV-6, AAV-7, AAV-8, AAV-9, AAV-10, AAV-11, AAV-12, AAV-13, AAV rh.74 and AAV rh.10 capsid proteins. For example, the rAAV comprises an rh.74 capsid protein or an AAV9 capsid protein.

The disclosure provides for methods of generating a recombinant AAV rAAV.tMCK.CAPN3, comprising transferring a plasmid to a cell, wherein the plasmid comprises a nucleotide sequence that is at least 90%, 95%, or 99% identical to SEQ ID NO: 13 or SEQ ID NO: 20, such as a nucleotide sequence of SEQ ID NO: 13 or SEQ ID NO: 20. Any of the methods may further comprise the step of transferring a packaging plasmid and/or a helper virus to the cell. In any of the methods, the cell comprises a stably integrated AAV cap gene and/or a a stably integrated AAV rep gene.

The disclosure provides for cells comprising a plasmid that comprises a nucleotide sequence that is at least 90%, 95%, or 99% identical to SEQ ID NO: 13, such as a nucleotide of SEQ ID NO: 13. For example, the cell is a bacterial cell, an insect cell, a mosquito cell, or a mammalian cell. Exemplary cells are HEK 293 cells, HeLa cells and an Sf9 *(Spodoptera frugiperda)* insect cells.

The disclosure provides for cells comprising a plasmid that comprises a nucleotide sequence that is at least 90%, 95%, or 99% identical to SEQ ID NO: 20, such as a nucleotide of SEQ ID NO: 20. For example, the cell is a bacterial cell, an insect cell, a mosquito cell, or a mammalian cell. Exemplary cells are HEK 293 cells, HeLa cells and an Sf9 *(Spodoptera frugiperda)* insect cells.

### Brief Description of the Drawings

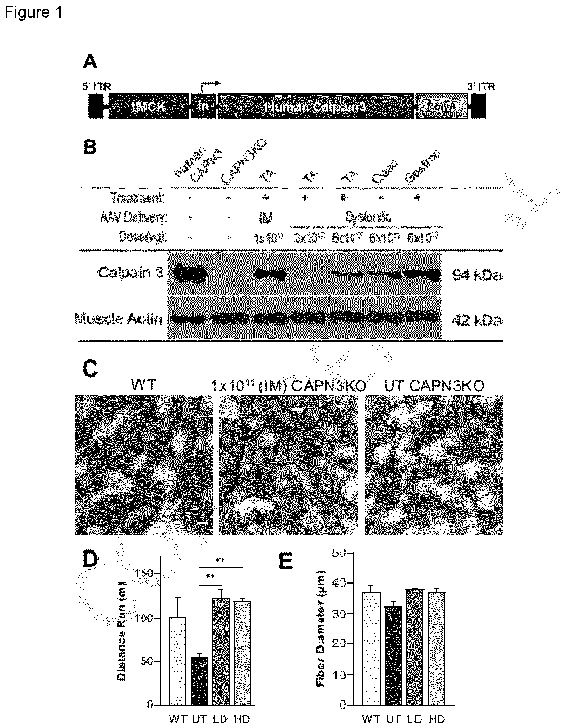

Figures 1A-1E demonstrate the biopotency of AAVrh74.tMCK.CAPN3 vector and short-term efficacy at 4 weeks post-gene delivery. (A) Schematic diagram of AAVrh74.tMCK.CAPN3 vector showing 5' and 3' AAV2 inverted terminal repeats (ITR), tMCK promoter, chimeric intron (In), human calpain 3 as transgene, and SV40 polyadenylation signal (Poly A). (B) Representative western blot images of calpain 3 protein in muscle samples following intramuscular (IM) and systemic gene delivery in CAPN3KO mice with normal human muscle lysate (Gel load of 60% total protein as compared to mouse lysates from tibialis anterior/TA, quadriceps and gastrocnemius), and untreated CAPN3KO for comparison. 94kDa full-length human calpain3 was detected in TA at 4 weeks post IM injection of the vector at 1×10¹¹ vg. Following systemic delivery, note the prominent increase of full-length calpain 3 protein from the 6×10¹² vg treatment (HD, high dose) as compared to the 3×10¹² vg (LD, low dose) cohort. (C) Representative images of succinic dehydrogenase, SDH-stained tissue sections from TA muscle in wild type (WT) and CAPN3KO mice following IM delivery of AAV.hCAPN3 or ringer lactate (UT, untreated). A fiber size increase towards normalization was evident with CAPN3 gene therapy. (D) Following systemic AAV.hCAPN3 gene therapy, both LD and HD treated CAPN3 KO mice performed better on treadmill test compared to UT counterparts. (E) Mean fiber diameter of TA muscle is shown following systemic delivery in LD and HD cohorts. Data from UT CAPN3KO and WT mice was included in D and E. Each bar represents the mean ± SEM of 3 to 4 mice. **p<0.01 One-way ANOVA, Tukey's multiple comparisons test. Scale bar = 30 µm in the WT figure 1applies to all.

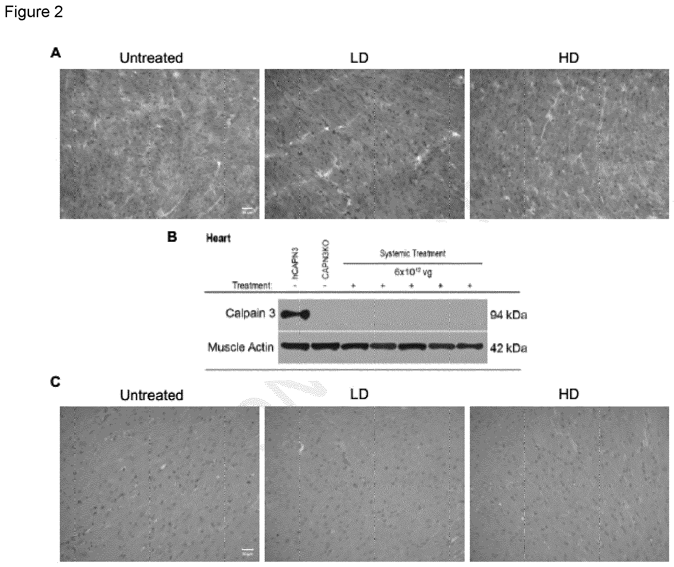

Figures 2A-2C provide histological and western blot analyses data showing no evidence of cardiotoxicity following systemic injection of AAVrh7.4.tMCK.hCAPN3 vector. (A) H&E stained fresh frozen sections of the left ventricles from representative heart samples of CAPN3KO mice at 4 post-systemic injection of the AAVrh7.4.tMCK.hCAPN3 vector at low dose (LD, 3×10¹² vg) and high dose (HD, 6×10¹² vg) with matching untreated control. Muscle fiber necrosis, regeneration or inflammation was not seen. (B) Western blot analysis of the cardiac tissue from HD cohort showed no detectable calpain 3 protein. Full length 94kDa hCAPN3 band is present in human skeletal muscle as positive control, which is absent in control CAPN3KO muscle. (C) H&E stained sections from paraffin embedded cardiac tissue at 20-weeks post-systemic injection of the AAVrh7.4.tMCK.hCAPN3 vector at LD and HD revealed no histopathology indicating no longterm toxicity.

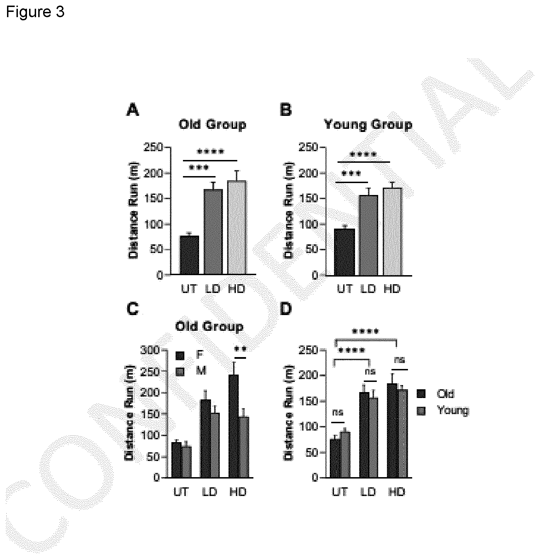

Figures 3A-3D provides functional efficacy of AAV.CAPN3 gene therapy by treadmill performance. In treadmill test, both LD (3×10¹² vg) and HD (6×10¹² vg) treatment cohorts showed significant improvements in (A) old and (B) young age groups compared to untreated (UT) CAPN3KO mice. (C) Females of the HD cohort from old group performed significantly better than the males of the same group. (D) There was no significant difference in treadmill outcomes resulted from early-onset treatment. Each bar represents the mean ± SEM of 12 to14 mice. **p<0.01, ***p<0.001, ****p<0.0001, one-way ANOVA, Tukey's multiple comparisons test for (A) and (B), and two-way ANOVA, Sidak's multiple comparisons test for (C) and (D).

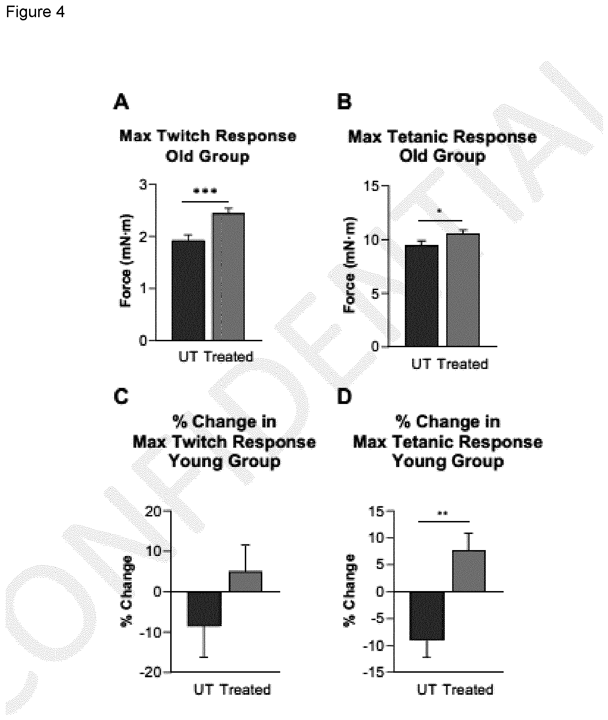

Figures 4A-4D demonstrates improvements in muscle physiology with AAV.CAPN3 gene therapy. In vivo muscle contractility assay showing a higher force output in both (A) maximum twitch and (B) tetanic responses in LD and HD treatment cohorts as compared to UT CAPN3KO mice animals from old age group. Data reflects all treatment groups combined (treated cohorts, n=.23; UT-cohort, n=28). Similarly, treated mice showed increased (C) twitch and (D) tetanic response in the young age group (treated cohorts, n=.22-23; UT-cohort, n=13). Each bar represents the mean ± SEM. *p≤0.05, **p≤0.01, ***p≤0.001, unpaired t-test.

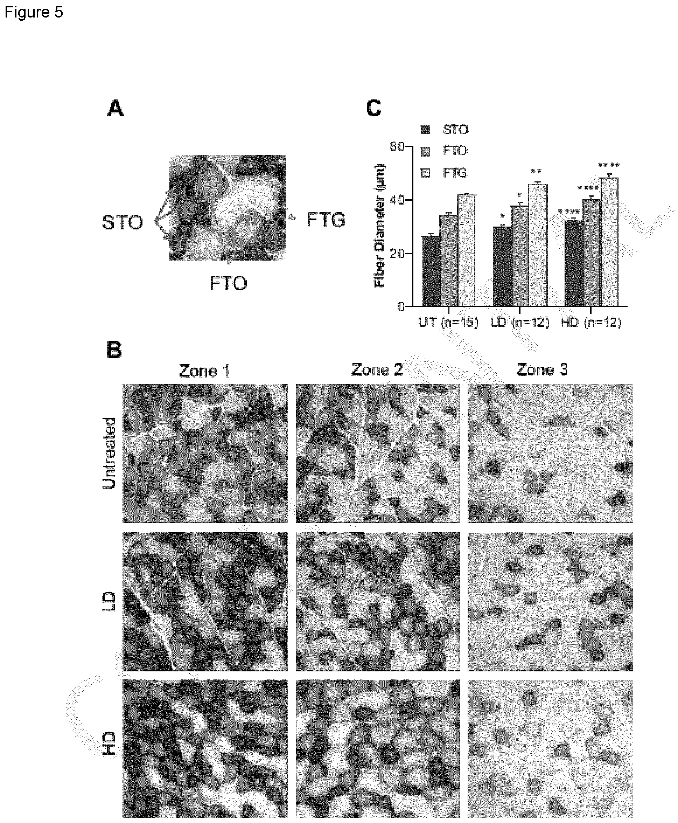

Figures 5A -5C demonstrates muscle fiber size increase in CAPN3KO mice with AAV.CAPN3 gene therapy. (A) Succinic dehydrogenase staining showing muscle fiber types based on mitochondria content: fatigue-resistant Slow Twitch Oxidative (STO, dark) or type1 fibers, the Fast Twitch Oxidative (FTO, intermediate) fibers with intermediate staining intensity and Fast Twitch Glycolytic (FTG, light) fibers with lightest staining intensity. (B) Representative images from tibialis anterior muscle showing three different zones (deep, intermediate and superficial; designated as Zone 1, 2, and 3) from the untreated (UT), low dose (LD, 3×10¹² vg) and high dose (HD, 6×10¹² vg) treatment cohorts. (C) Fiber size measurements, done on images derived from 3 zones of the muscle showed significant increases for all fiber types (STO, FTO and FTG) in both LD and HD cohorts compared to UT CAPN3KO group. Each bar represents the mean ± SEM of 12 to 15 mice in each group. *p<0.05, **p<0.01, ****p<0.0001 (compared to UT), two-way ANOVA, Tukey's multiple comparisons test. Scale bar = 30 µm in the untreated-Zone 1.

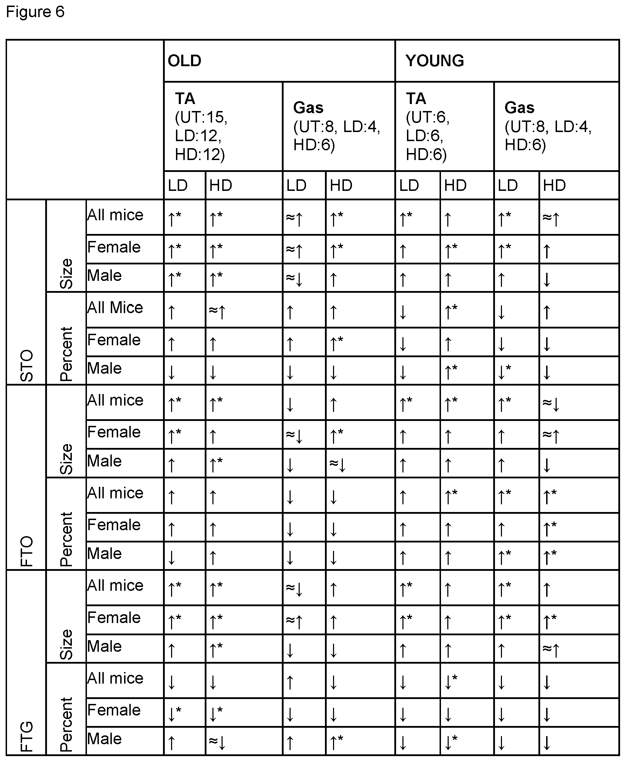

Figure 6 demonstrates muscle fiber type remodeling in CAPN3KO mice with AAV.CAPN3 gene therapy. Compared to untreated (UT) cohort, direction of changes observed in the mean fiber size and percent distribution of each fiber type in tibialis anterior (TA), triceps (Tri), gastrocnemius (Gas) and quadriceps (Quad) muscles from the treated CAPN3KO mice of both age groups are depicted with arrows. Asterisk indicates that the change observed is significantly different from untreated mice.

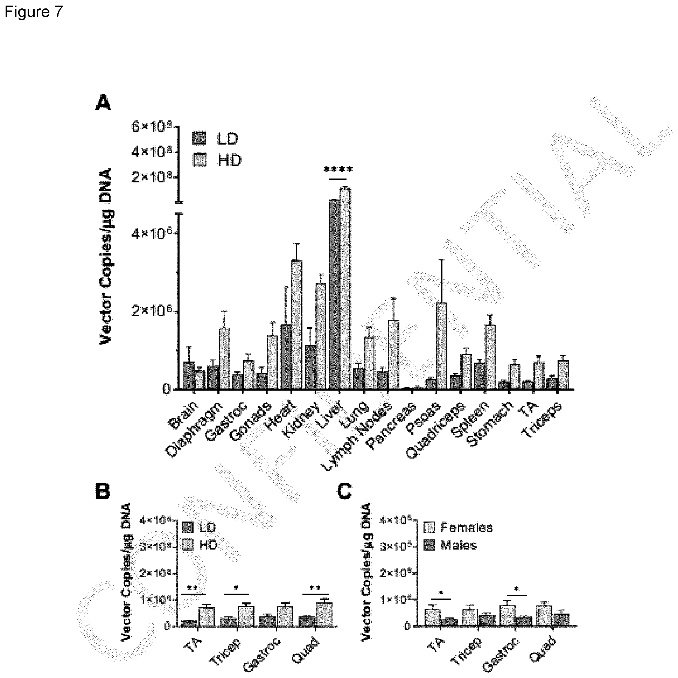

Figures 7A-C provides AAVrh74.tMCK.hCAPN3 vector biodistribution 20 weeks post-injection. (A-C) Vector copy number which corresponds to viral genomes/diploid genome (vg/dg or vector copies per µg of genomic DNA), was quantified in various tissues including four different muscle groups from lower and upper limbs from CAPN3KO mice of LD and HD cohorts of old age group (A) Vector genome distribution was variable in organs and muscles in a dose dependent manner. The highest vector genome copy number was present in the liver from both treatment cohorts. (B) The HD treatment resulted in a 59.6% increase in skeletal muscle vg levels, p< 0.0001, compared with LD. (C) Gender difference in the AAV biodistribution in CAPN3KO is shown. Females had 48.7% increase in combined skeletal muscle vg levels across all treatments, p= 0.0001, as compared with male mice, unpaired t-test. Each bar represents the mean ± SEM of 9 to 12 samples. *p<0.05, **p<0.01, ****p<0.0001, two-way ANOVA, Tukey's multiple comparisons test.

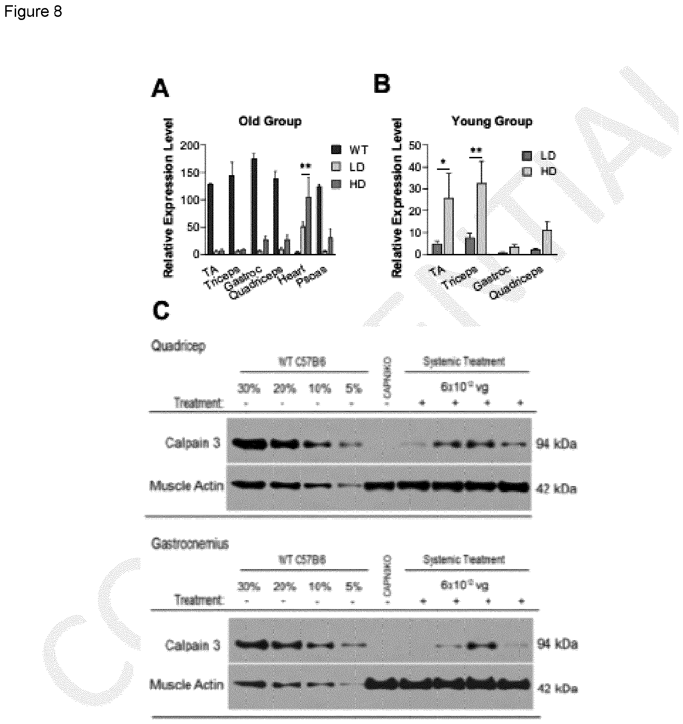

Figures 8A-8C provides muscle tissue expression of hCAPN3 at mRNA and protein levels in CAPN3KO mice 20 weeks post-gene delivery. Relative expression levels of hCAPN3 in several skeletal and cardiac muscle tissue samples from the old (A) and young age groups of CAPN3KO mice (B) are shown. A dose-dependent increase in CAPN3 mRNA transcripts was observed in the high dose (HD, 6×10¹² vg; n=12) treatment cohort compared to the low dose (LD, 3x1012 vg; n=12) in both age groups. Data are represented as mean ± SEM. *p<0.05, **p<0.01, two-way ANOVA, Sidak's multiple comparisons test. (C) Representative western blot images from 4 mice show variable amounts of 94kDa CAPN3 protein in quadriceps and gastrocnemius muscles from the HD cohort of the old age group. A calibration curve ranging from 15 µg to 2.5 µg (30-5%) total protein from the corresponding untreated WT C57BL/6 reference muscles were used for semiquantitative analysis of the AAV-mediated CAPN3 levels in the corresponding treated CAPN3-KO mice muscles. CAPN3 levels were detected using the CALP-12A2 antibody which recognizes epitopes in the muscle extracts from human and mouse. Assay sensitivity was optimized to reliably detect the CAPN3 protein in amounts as low as 2.5% of the WT control level.

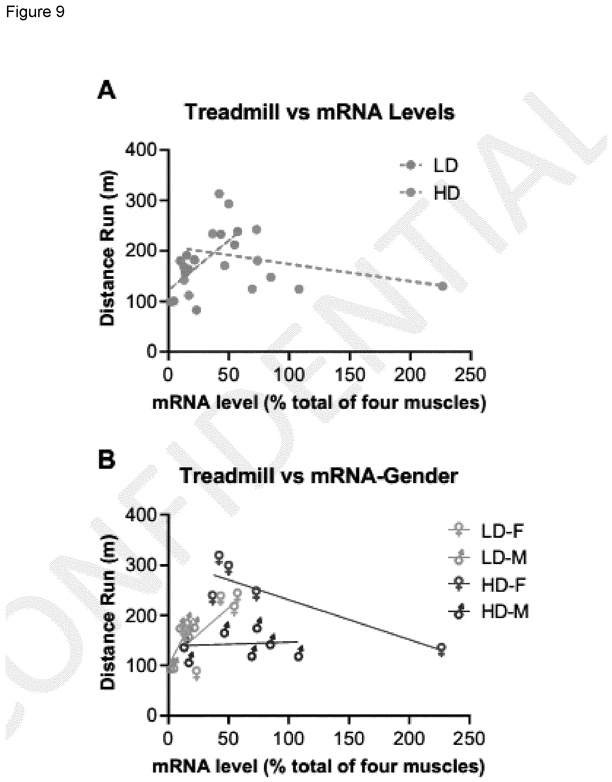

Figures 9A-9B demonstrate correlation of the CAPN3 expression levels with treadmill performance. (A) Scattergram showing correlation between the total mRNA levels from 4 representative muscles (tibialis anterior, gastrocnemius, quadriceps and triceps) correlating with run to exhaustion test in the low dose (LD, 3x1012 vg, n=12) cohort (R2=0.5192, p=0.0082, linear regression analysis) while in the high dose cohort (HD, 6×10¹² vg, n=12), no correlation was seen between mRNA levels and the run to exhaustion test performance. (B) The mRNA level window for the best treadmill performance favored females from both HD and LD cohorts.

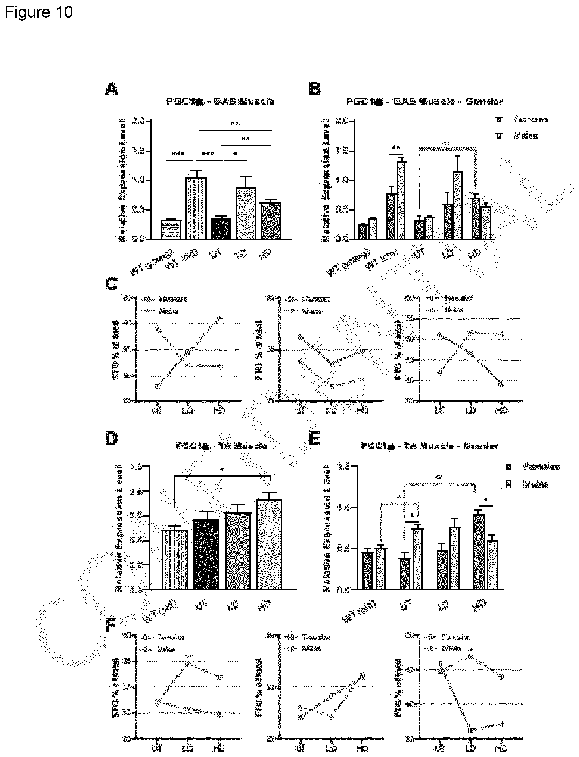

Figures 10A-10F provides relative PGC1α expression levels in muscle and its correlation with fiber type remodeling. Relative expression levels of PGC1α in gastrocnemius (Gas, A and B) and tibialis anterior (TA, D and E) muscles from the old age group of CAPN3KO (UT, n=8; LD, n=12; HD, n=12) and age-matched wild type (WT, n=8) mice at 20 weeks post hCAPN3 gene delivery are shown as both sexes combined (A and D) and separated (B and E). Line graphs showing the percent changes in the fiber type (STO, FTO and FTG) distribution in the same muscles, gastrocnemius (C) and tibialis anterior (F) from LD and HD treatment cohorts. Data are represented as mean ± SEM. *p<0.05, **p<0.01, ***p<0.001, unpaired t-test. A switch to STO (as % increase) in both HD and LD cohorts was observed only in females (C, F) even though LD males showed higher PGC1α expression than females (B, E).

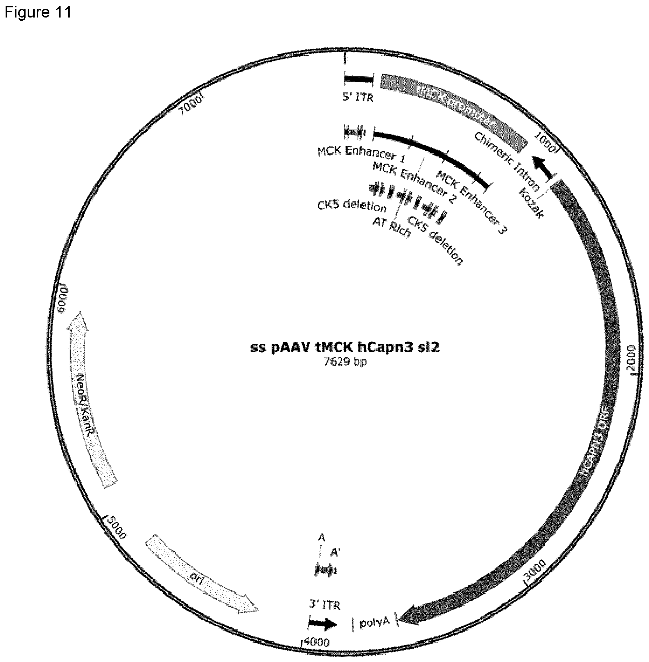

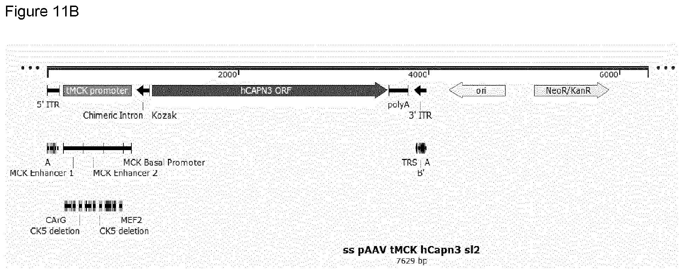

Figures 11A-11B provides a schematic view of the pAAV.tMCK.hCAPN3 vector. Fig. 11A provides a circular map and Fig. 11B provides a linear map.

### Detailed Description

Recombinant AAVs (rAAVs) provided herein comprise a polynucleotide that comprises a first AAV inverted terminal repeat (ITR), a promoter, a nucleotide sequence encoding a protein with calpain 3 (CAPN3) activity and a second AAV ITR. In one embodiment, the nucleotide encodes CAPN3. Embodiments include, but are not limited to, an rAAV comprising a nucleotide sequence encoding CAPN3 or a protein with CAPN3 activity, wherein the nucleotide sequence is at least 65%, at least 70%, at least 75%, at least 80%, 81%, 82%, 83%, 84%, 85%, 86%, 87%, 88%, or 89% identical to the nucleotide sequence of SEQ ID NO: 2. Additional embodiments include, but are also not limited to, rAAV comprising a nucleotide sequence at least 90%, 91%, 92%, 93%, 94%, 95%, 96%, 97%, 98%, 99% identical to the nucleotide sequence set forth in SEQ ID NO: 2 and encodes a polypeptide with a CAPN3 proteolytic activity. The CAPN3 proteolytic activity is understood in the art as the activity of proteolyzing potential substrates such as fodrin and HSP60, and/or to the activity of autolytically self-cleaving. Thus, as used herein, the term "a protein with calpain 3 (CAPN3) activity" refers to a protein with CAPN3 proteolytic activity, which includes but is not limited to the activity of proteolyzing substrates such as fodrin and HSP60, and/or to the activity of autolytically self-cleaving. The protein with CAPN3 activity can have the full or partial activity of a full length calpain 3 protein. In one embodiment, the protein with CAPN3 activity has at least 60%, 70%, 80%, 90%, 95%, or 99% of activity of a full length CAPN3 protein. In another embodiment, the protein with CAPN3 activity comprises an amino acid sequence with at least 90%, 91%, 92%, 93%, 94%, 95%, 96%, 97%, 98%, or 99% identical to SEQ ID NO: 5.

In some embodiments, the nucleotide sequence encoding the protein with CAPN3 activity comprises a sequence of SEQ ID NO: 2. In another embodiment, the protein with CAPN3 activity comprises an amino acid sequence with at least 90%, 91%, 92%, 93%, 94%, 95%, 96%, 97%, 98%, or 99% identical to SEQ ID NO: 5. In another embodiment, the protein with CAPN3 activity comprises the amino acid sequence of SEQ ID NO: 5. In another embodiment, the polynucleotide of the rAAV comprises a sequence which is at least 90%, 91%, 92%, 93%, 94%, 95%, 96%, 97%, 98%, or 99% identical to SEQ ID NO: 1. In another embodiment, the polynucleotide comprises a sequence at least 95% identical to SEQ ID NO: 1. In one embodiment, the polynucleotide comprises the sequence of SEQ ID NO: 1.

In another aspect, described herein is a recombinant AAV comprising a nucleotide sequence that encodes a protein with CAPN3 activity and/or that comprises a nucleotide sequence that hybridizes under stringent conditions to the nucleic acid sequence of SEQ ID NO: 2, or the complement thereof. The term "stringent" is used to refer to conditions that are commonly understood in the art as stringent. Hybridization stringency is principally determined by temperature, ionic strength, and the concentration of denaturing agents such as formamide. Examples of stringent conditions for hybridization and washing are 0.015 M sodium chloride, 0.0015 M sodium citrate at 65-68°C or 0.015 M sodium chloride, 0.0015M sodium citrate, and 50% formamide at 42°C. See Sambrook et al., Molecular Cloning: A Laboratory Manual, 2nd Ed., Cold Spring Harbor Laboratory, (Cold Spring Harbor, N.Y. 1989).

In recombinant genomes described herein, the CAPN3 polynucleotide is operatively linked to transcriptional control elements (including, but not limited to, promoters, enhancers and/or introns), specifically transcriptional control elements functional in target cells of interest. For example, various embodiment provide methods of transducing muscle cells using muscle-specific transcriptional control elements, including, but not limited to, those derived from the actin and myosin gene families, such as from the myoD gene family [See Weintraub et al., Science, 251: 761-766 (1991)], the myocyte-specific enhancer binding factor MEF-2 [Cserjesi and Olson, Mol Cell Biol, 11: 4854-4862 (1991)], control elements derived from the human skeletal actin gene [Muscat et al., Mol Cell Biol, 7: 4089-4099 (1987)], muscle creatine kinase sequence elements [See Johnson et al., Mol Cell Biol, 9:3393-3399 (1989)] and the murine creatine kinase enhancer (mCK) element, control elements derived from the skeletal fast-twitch troponin C gene, the slow-twitch cardiac troponin C gene and the slow-twitch troponin I gene: hypozia-inducible nuclear factors [Semenza et al., Proc Natl Acad Sci USA, 88: 5680-5684 (1991)], steroid-inducible elements and promoters including the glucocorticoid response element (GRE) [See Mader and White, Proc. Natl. Acad. Sci. USA, 90: 5603-5607 (1993)], the tMCK promoter [see Wang et al., Gene Therapy, 15: 1489-1499 (2008)], the CK6 promoter [see Wang *et al., supra]* and other control elements. In one embodiment, the nucleotide sequence encoding a protein with calpain 3 (CAPN3) activity is operably linked to a muscle-specific promoter. In one embodiment, the muscle-specific promoter comprises one or more of a human skeletal actin gene element, a cardiac actin gene element, a desmin promoter, a skeletal alpha-actin (ASKA) promoter, a troponin I (TNNI2) promoter, a myocyte-specific enhancer binding factor mef binding element, a muscle creatine kinase (MCK) promoter, a truncated MCK (tMCK) promoter, a myosin heavy chain (MHC) promoter, a hybrid a-myosin heavy chain enhancer-/MCK enhancer-promoter (MHCK7) promoter, a C5-12 promoter, a murine creatine kinase enhancer element, a skeletal fast-twitch troponin c gene element, a slow-twitch cardiac troponin c gene element, a slow-twitch troponin i gene element, hypoxia- inducible nuclear factor (HIF)-response element (HRE), a steroid-inducible element, a glucocorticoid response element (gre). In another embodiment, the muscle-specific promoter is an MCK promoter, a tMCK promoter, or an MHCK7 promoter. In some embodiments, the muscle-specific promoter is tMCK that comprises a nucleotide sequence of SEQ ID NO: 3. In some embodiments, the muscle-specific promoter is MHCK7 that comprises a nucleotide sequence of SEQ ID NO: 4.

Previous studies showed that expression of CAPN3 driven by desmin promoter resulted in cardiotoxicity. In follow up studies, selective skeletal muscle expression of the gene eliminated the cardiac defects. The AAV genomes disclosed herein comprise a muscle specific promoter, tMCK to restrict CAPN3 expression to the skeletal muscle and showed no cardiac toxicity following systemic delivery of the virus at 6E12 vg (twice the proposed initial high dose) 4 weeks after gene injection.

Recombinant AAV provided herein are thus replication-deficient, infectious, encapsidated viral particles which comprise a recombinant genome. Examples include, but are not limited to, a rAAV including a genome comprising the sequence set out in SEQ ID NO: 1 encoding CAPN3, a rAAV including a genome consisting essentially of the sequence set out in SEQ ID NO: 1 encoding CAPN3, and a rAAV (named "AAVrh.74.tMCK.CAPN3") including a genome consisting of the sequence set out in SEQ ID NO: 1 encoding CAPN3. The genomes of the rAAV lack AAV rep and cap DNA, that is, there is no AAV rep or cap DNA between the ITRs of the rAAV genome.

The sequence of the AAVrh.74.tMCK.CAPN3 sequence is set out in SEQ ID NO: 1, in which an AAV2 ITR spans nucleotides 1-128, the tMCK promoter spans nucleotides 165-884, a chimeric intron spans nucleotides 937-1069, a Kozak Sequence spans nucleotides 1101-1106, the CAPN3 polynucleotide spans nucleotides 1107-3572, a poly A signal spans nucleotides 3581-3780, and a second AAV2 ITR spans nucleotides 3850-3977.

Methods of transducing a target cell such as a muscle cell with rAAV, *in vivo* or *in vitro,* are contemplated herein. The *in vivo* methods comprise the step of administering an effective dose, or effective multiple doses, of a composition comprising a rAAV provided herein to subject (e.g., an animal including but not limited to a human patient) in need thereof. If the dose is administered prior to development of a disorder/disease, the administration is prophylactic. If the dose is administered after the development of a disorder/disease, the administration is therapeutic. An effective dose is a dose that alleviates (eliminates or reduces) at least one symptom associated with the disorder/disease state being treated, that slows or prevents progression to a disorder/disease state, that slows or prevents progression of a disorder/disease state, that diminishes the extent of disease, that results in remission (partial or total) of disease, and/or that prolongs survival. In comparison to the subject before treatment, methods herein result in one or more of: an increased muscle fiber diameter, a decreased number of small lobulated muscle fibers, a decreased number of fibers with internal nuclei, a decreased endomysial connective tissue content, correction of muscle atrophy, and an increased muscle force generation. In one embodiment, the muscle fiber comprises one or more of slow twitch oxidative (STO) muscle fiber, fast twitch oxidative (FTO) muscle fiber, and fast twitch glycolytic (FTG) fiber. In one embodiment, the treatment results in one or more of (a) at least a 5%, 10%, 15%, 20%, 25%, 30%, or 35%, or 40% decrease of total muscle fiber number per mm² by 4 weeks after administration; (b) at least a 5%, 10%, 15%, 20%, or 25% increase of muscle fiber diameter by 4 weeks after administration; (c) at least a 5%, 10%, 15%, 20%, 25%, 30%, 35%, or 42% decrease of STO muscle fiber number per mm² by 4 weeks after administration; (d) at least a 5%, 10%, 15%, 20%, or 25% increase of STO muscle fiber diameter by 4 weeks after administration; (e) at least a 5%, 10%, 15%, or 20% decrease of FTO muscle fiber number per mm² by 4 weeks after administration; (f) at least a 5%, 10%, 15%, or 20% increase of FTO muscle fiber diameter by 4 weeks after administration; (g) at least a 5%, 10%, 15%, 20%, 25%, 30%, or 35% decrease of FTG muscle fiber number per mm² by 4 weeks after administration; and (h) at least a 5%, 10%, 15%, 20%, or 25% increase of FTG muscle fiber diameter by 4 weeks after administration. The method of this disclosure, in one embodiment, leads to no, minimum or low calpain 3 protein expressed from the rAAV in the heart muscle of the subject administered with the rAAV.

Assays to examine these results are understood in the art and/or are described in the examples herein. Use of the methods described herein to prevent or treat disorders/diseases (e.g., muscular dystrophies) caused by defects in CAPN3 activity or defects in expression of CAPN3 is contemplated. LGMD2A is an example of a disease contemplated for prevention or treatment according to the methods.

Combination therapies are also contemplated. Combination as used herein includes both simultaneous treatment or sequential treatments. Combinations of methods described herein with standard medical treatments (e.g., corticosteroids) are specifically contemplated, as are combinations with novel therapies.

Administration of an effective dose of the compositions may be by routes standard in the art including, but not limited to, intramuscular, parenteral, intravenous, intrathecal, oral, buccal, nasal, pulmonary, intracranial, intraosseous, intraocular, rectal, or vaginal. Route(s) of administration and serotype(s) of AAV components of the rAAV (in particular, the AAV ITRs and capsid protein) may be chosen and/or matched by those skilled in the art taking into account the infection and/or disease state being treated and the target cells/tissue(s) that are to express the CAPN3. In one embodiment, the rAAV is administered by intramuscular injection, intravenous injection, intraperitoneal injection, subcutaneous injection, epicutaneous administration, intravaginal injection, intradermal administration, or nasal administration. In another embodiment, the rAAV is administered by intramuscular injection or intravenous injection.

The disclosure provides for local administration and systemic administration of an effective dose of rAAV and compositions of the disclosure. For example, systemic administration is administration into the circulatory system so that the entire body is affected. Systemic administration includes enteral administration such as absorption through the gastrointestinal tract and parenteral administration through injection, infusion or implantation.

In particular, actual administration of rAAV described herein may be accomplished by using any physical method that will transport the rAAV recombinant vector into the target tissue of an animal. Administration includes, but is not limited to, injection into muscle, the bloodstream, and/or directly into the liver. Simply resuspending a rAAV in phosphate buffered saline has been demonstrated to be sufficient to provide a vehicle useful for muscle tissue expression, and there are no known restrictions on the carriers or other components that can be co-administered with the rAAV. Capsid proteins of a rAAV may be modified so that the rAAV is targeted to a particular target tissue of interest such as muscle. See, for example, WO 02/053703, the disclosure of which is incorporated by reference herein. Pharmaceutical compositions can be prepared as injectable formulations or as topical formulations to be delivered to the muscles by transdermal transport. Numerous formulations for both intramuscular injection and transdermal transport have been previously developed and can be used in the practice of the methods. The rAAV can be used with any pharmaceutically acceptable carrier for ease of administration and handling.

For purposes of intramuscular injection, solutions in an adjuvant such as sesame or peanut oil or in aqueous propylene glycol can be employed, as well as sterile aqueous solutions. Such aqueous solutions can be buffered, if desired, and the liquid diluent first rendered isotonic with saline or glucose. Solutions of rAAV as a free acid (DNA contains acidic phosphate groups) or a pharmacologically acceptable salt can be prepared in water suitably mixed with a surfactant such as hydroxpropylcellulose. A dispersion of rAAV can also be prepared in glycerol, liquid polyethylene glycols and mixtures thereof and in oils. Under ordinary conditions of storage and use, these preparations contain a preservative to prevent the growth of microorganisms. In this connection, the sterile aqueous media employed are all readily obtainable by standard techniques well-known to those skilled in the art.

The pharmaceutical forms suitable for systemic (e.g., intravenous) injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating actions of microorganisms such as bacteria and fungi. The carrier can be a solvent or dispersion medium containing, for example, water, ethanol, polyol (for example, glycerol, propylene glycol, liquid polyethylene glycol and the like), suitable mixtures thereof, and vegetable oils. The proper fluidity can be maintained, for example, by the use of a coating such as lecithin, by the maintenance of the required particle size in the case of a dispersion and by the use of surfactants. The prevention of the action of microorganisms can be brought about by various antibacterial and antifungal agents, for example, parabens, chlorobutanol, phenol, sorbic acid, thimerosal and the like. In many cases it will be preferable to include isotonic agents, for example, sugars or sodium chloride. Prolonged absorption of the injectable compositions can be brought about by use of agents delaying absorption, for example, aluminum monostearate and gelatin.

Sterile injectable solutions are prepared by incorporating rAAV in the required amount in the appropriate solvent with various other ingredients enumerated above, as required, followed by filter sterilization. Generally, dispersions are prepared by incorporating the sterilized active ingredient into a sterile vehicle which contains the basic dispersion medium and the required other ingredients from those enumerated above. In the case of sterile powders for the preparation of sterile injectable solutions, the methods of preparation, in some embodiments, comprises vacuum drying and/or the freeze drying technique, each of which can yield a powder of the active ingredient plus any additional desired ingredient from the previously sterile-filtered solution thereof.

In one embodiment of the disclosure, the rAAV is formulated in a buffer containing 20 mM Tris (pH 8.0), 1mM magnesium chloride (MgCl₂), 200 mM sodium chloride (NaCI), and 0.001% poloxamer 188.

Transduction with rAAV may also be carried out *in vitro.* In one embodiment, desired target muscle cells are removed from the subject, transduced with rAAV and reintroduced into the subject. Alternatively, syngeneic or xenogeneic muscle cells can be used where those cells will not generate an inappropriate immune response in the subject.

Suitable methods for the transduction and reintroduction of transduced cells into a subject are known in the art. In one embodiment, cells can be transduced *in vitro* by combining rAAV with muscle cells, e.g., in appropriate media, and screening for those cells harboring the DNA of interest using conventional techniques such as Southern blots and/or PCR, or by using selectable markers. Transduced cells can then be formulated into pharmaceutical compositions, and the composition introduced into the subject by various techniques, such as by intramuscular, intravenous, subcutaneous and intraperitoneal injection, or by injection into smooth and cardiac muscle, using e.g., a catheter.

Transduction of cells with rAAV by methods described herein results in sustained expression of CAPN3 or a protein with CAPN3 activity. Methods are thus provided for administering rAAV which expresses CAPN3 or a protein with CAPN3 activity to a subject, preferably a human being. The subject of this disclosure includes but is not limited to human, a dog, a cat, a horse, a cow, a pig, a sheep, a goat, a chicken, a rodent (e.g., rats and mice), and a primate. These methods include transducing tissues (including, but not limited to, tissues such as muscle, organs such as liver and brain, and glands such as salivary glands) with one or more rAAV described herein.

Muscle tissue is an attractive target for *in vivo* DNA delivery, because it is not a vital organ and is easy to access. The methods herein provide sustained expression of CAPN3 from transduced muscle cells.

By "muscle cell," "muscle fiber," or "muscle tissue" is meant a cell or group of cells derived from muscle of any kind (for example, skeletal muscle and smooth muscle (e.g., from the digestive tract, urinary bladder, blood vessels or cardiac tissue). Such muscle cells may be differentiated or undifferentiated, such as myoblasts, myocytes, myotubes, cardiomyocytes and cardiomyoblasts.

For example, the term "transduction" is used to refer to the administration/delivery of CAPN3 to a recipient cell either *in vivo* or *in vitro,* via a rAAV described resulting in expression of CAPN3 by the recipient cell.

Thus, methods are provided of administering an effective dose (or doses, administered essentially simultaneously or doses given at intervals) of rAAV that encode CAPN3 to a subject in need thereof.

As noted above, the methods described herein result in the subject, in comparison to the subject before treatment, one or more of: increased muscle fiber diameter, decreased number of small lobulated slow twitch oxidative (STO) muscle fibers, decreased number of fibers with internal nuclei, decreased endomysial connective tissue content, correction of muscle atrophy, and increased muscle force generation.

The practice of the present invention will employ, unless otherwise indicated, conventional methods of virology, microbiology, molecular biology and recombinant DNA techniques within the skill of the art. Such techniques are explained fully in the literature. See, e.g., Sambrook et al. Molecular Cloning: A Laboratory Manual (Current Edition); DNA Cloning: A Practical Approach, Vol. I & II (D. Glover, ed.); Oligonucleotide Synthesis (N. Gait, ed., Current Edition); Nucleic Acid Hybridization (B. Hames & S. Higgins, eds., Current Edition); Transcription and Translation (B. Hames & S. Higgins, eds., Current Edition); CRC Handbook of Parvoviruses, vol. I & II (P. Tijssen, ed.); Fundamental Virology, 2nd Edition, vol. I & II (B. N. Fields and D. M. Knipe, eds.); Freshney Culture of Animal Cells, A Manual of Basic Technique (Wiley-Liss, Third Edition); and Ausubel et al. (1991) Current Protocols in Molecular Biology (Wiley Interscience, N.Y.).

### Definitions

As used herein, the singular forms "a," "an," and "the" include plural referents unless the context clearly dictates otherwise. Thus, for example, reference to "a cell" includes a plurality of such cells and reference to "the culture" includes reference to one or more cultures and equivalents thereof known to those skilled in the art, and so forth. Reference to "a recombinant AAV" includes a mixture of two or more rAAV virions, and the like. All technical and scientific terms used herein have the same meaning as commonly understood to one of ordinary skill in the art to which this invention belongs unless clearly indicated otherwise.

The use of the term "or" in the claims is used to mean "and/or" unless explicitly indicated to refer to alternatives only, or the alternatives are mutually exclusive, although the disclosure supports a definition that refers to only alternatives and "and/or."

Throughout this application, the term "about" is used to indicate that a value includes the statistical experimental error (standard deviation of error) for the device or method being employed to determine the value.

The term "vector" is meant to be any genetic element, such as a plasmid, phage, transposon, cosmid, chromosome, virus, virion, etc., which is capable of replication when associated with the proper control elements and which can transfer gene sequences between cells. In one embodiment, the vector is a viral vector.

As used herein, the term "AAV" is a standard abbreviation for adeno-associated virus. Adeno-associated virus is a single-stranded DNA parvovirus that grows only in cells in which certain functions are provided by a co-infecting helper virus. There are currently thirteen serotypes of AAV that have been characterized. General information and reviews of AAV can be found in, for example, Carter, 1989, Handbook of Parvoviruses, Vol. 1, pp. 169-228, and Berns, 1990, Virology, pp. 1743-1764, Raven Press, (New York). However, it is fully expected that these same principles will be applicable to additional AAV serotypes since it is well known that the various serotypes are quite closely related, both structurally and functionally, even at the genetic level. (See, for example, Blacklowe, 1988, pp. 165-174 of Parvoviruses and Human Disease, J. R. Pattison, ed.; and Rose, Comprehensive Virology 3:1-61 (1974)). For example, all AAV serotypes apparently exhibit very similar replication properties mediated by homologous rep genes; and all bear three related capsid proteins such as those expressed in AAV2. The degree of relatedness is further suggested by heteroduplex analysis which reveals extensive cross-hybridization between serotypes along the length of the genome; and the presence of analogous self-annealing segments at the termini that correspond to "inverted terminal repeat sequences" (ITRs). The similar infectivity patterns also suggest that the replication functions in each serotype are under similar regulatory control.

An "AAV vector" as used herein refers to a vector comprising one or more polynucleotides of interest (or transgenes) that are flanked by AAV terminal repeat sequences (ITRs). Such AAV vectors can be replicated and packaged into infectious viral particles when present in a host cell that has been transfected with a vector encoding and expressing rep and cap gene products. In one embodiment, the AAV vector is a vector derived from an adeno-associated virus serotype, including without limitation, AAV-1, AAV-2, AAV-3, AAV-4, AAV-5, AAV-6, AAV-7, AAV-8, AAV-9, AAV-10, AAV-11, AAV-12, AAV-13, AAV rh10, and AAV rh74. AAV vectors can have one or more of the AAV wild-type genes deleted in whole or part, preferably the rep and/or cap genes, but retain functional flanking ITR sequences. Functional ITR sequences are necessary for the rescue, replication and packaging of the AAV virion. Thus, an AAV vector is defined herein to include at least those sequences required in cis for replication and packaging (e.g., functional ITRs) of the virus. The ITRs need not be the wild-type nucleotide sequences, and may be altered, e.g., by the insertion, deletion or substitution of nucleotides, as long as the sequences provide for functional rescue, replication and packaging.

The term "AAV helper functions" refer to AAV-derived coding sequences that can be expressed to provide AAV gene products that, in turn, function in trans for productive AAV replication. Thus, AAV helper functions comprise the major AAV open reading frames (ORFs), reps and cap. The Rep expression products have been shown to possess many functions, including, among others: recognition, binding and nicking of the AAV origin of DNA replication; DNA helicase activity; and modulation of transcription from AAV (or other heterologous) promoters. The Cap expression products supply necessary packaging functions. AAV helper functions are used herein to complement AAV functions in trans that are missing from AAV vectors.

By "recombinant virus" is meant a virus that has been genetically altered, e.g., by the addition or insertion of a heterologous nucleic acid sequence into the viral particle.

An "AAV virion" or "AAV viral particle" or "AAV vector particle" refers to a viral particle composed of at least one AAV capsid protein and an encapsidated polynucleotide AAV vector. The AAV virion, in one embodiment, comprises a heterologous polynucleotide (i.e. a polynucleotide other than a wild-type AAV genome such as a transgene to be delivered to a mammalian cell). Production of AAV viral particles, in some embodiment, includes production of AAV vector, as such a vector is contained within an AAV vector particle.

AAV genome such as a transgene to be delivered to a mammalian cell), it is typically referred to as an "AAV vector particle" or simply an "AAV vector". Thus, production of AAV vector particle necessarily includes production of AAV vector, as such a vector is contained within an AAV vector particle.

For example, a wild-type (wt) AAV virus particle comprising a linear, single-stranded AAV nucleic acid genome associated with an AAV capsid protein coat. The AAV virion can be either a single-stranded (ss) AAV or self-complementary (SC) AAV. In one embodiment, a single-stranded AAV nucleic acid molecules of either complementary sense, e.g., "sense" or "antisense" strands, can be packaged into a AAV virion and both strands are equally infectious.

The term "recombinant AAV," or "rAAV" is defined herein as an infectious, replication-defective virus composed of an AAV protein shell, encapsidating a heterologous nucleotide sequence of interest which is flanked on both sides by AAV ITRs. A rAAV, in one embodiment, is produced in a suitable host cell which has an AAV vector, AAV helper functions and accessory functions introduced therein. In this manner, the host cell is capable of encoding AAV polypeptides that are required for packaging the AAV vector (containing a recombinant nucleotide sequence of interest) into infectious recombinant virion particles for subsequent gene delivery.

The term "transfection" refers to the uptake of foreign DNA by a cell, and a cell has been "transfected" when exogenous DNA has been introduced inside the cell membrane. A number of transfection techniques are generally known in the art. See, e.g., Graham et al. (1973) Virology, 52:456, Sambrook et al. (1989) Molecular Cloning, a laboratory manual, Cold Spring Harbor Laboratories, New York, Davis et al. (1986) Basic Methods in Molecular Biology, Elsevier, and Chu et al. (1981) Gene 13:197. Such techniques can be used to introduce one or more exogenous DNA moieties, such as a nucleotide integration vector and other nucleic acid molecules, into suitable host cells.

The term "transduction" denotes the delivery of a DNA molecule to a recipient cell either in vivo or in vitro, via a replication-defective viral vector, such as via a recombinant AAV virion.

The term "host cell" denotes, for example, microorganisms, yeast cells, insect. cells, and mammalian cells, that can be, or have been, used as recipients of an AAV helper construct, an AAV vector plasmid, an accessory function vector, or other transfer DNA. The term includes the progeny of the original cell which has been transfected. Thus, a "host cell" as used herein generally refers to a cell which has been transfected with an exogenous DNA sequence. It is understood that the progeny of a single parental cell may not necessarily be completely identical in morphology or in genomic or total DNA complement as the original parent, due to natural, accidental, or deliberate mutation.

The term "heterologous" as it relates to nucleic acid sequences such as coding sequences and control sequences, denotes sequences that are not normally joined together, and/or are not normally associated with a particular cell. Thus, a "heterologous" region of a nucleic acid construct or a vector is a segment of nucleic acid within or attached to another nucleic acid molecule that is not found in association with the other molecule in nature. For example, a heterologous region of a nucleic acid construct could include a coding sequence flanked by sequences not found in association with the coding sequence in nature. Another example of a heterologous coding sequence is a construct where the coding sequence itself is not found in nature (e.g., synthetic sequences having codons different from the native gene). Similarly, a cell transformed with a construct which is not normally present in the cell would be considered heterologous for purposes of this invention. Allelic variation or naturally occurring mutational events do not give rise to heterologous DNA, as used herein.

A "coding sequence" or a sequence which "encodes" a particular protein, is a nucleic acid sequence which is transcribed (in the case of DNA) and translated (in the case of mRNA) into a polypeptide in vitro or in vivo when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, cDNA from prokaryotic or eukaryotic mRNA, genomic DNA sequences from prokaryotic or eukaryotic DNA, and even synthetic DNA sequences. A transcription termination sequence will usually be located 3' to the coding sequence.

A "nucleic acid" sequence refers to a DNA or RNA sequence. The nucleic acids include base analogues of DNA and RNA including, but not limited to 4-acetylcytosine, 8-hydroxy-N6-methyladenosine, aziridinylcytosine, pseudoisocytosine, 5-(carboxyhydroxylmethyl)uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, -uracil-5-oxyacetic acid methylester, uracil-5-oxyacetic acid, pseudouracil, queosine, 2-thiocytosine, and 2,6-diaminopurine.

The term DNA "control sequences" refers collectively to promoter sequences, polyadenylation signals, transcription termination sequences, upstream regulatory domains, origins of replication, internal ribosome entry sites ("IRES"), enhancers, and the like, which collectively provide for the replication, transcription and translation of a coding sequence in a recipient cell. Not all of these control sequences need always be present so long as the selected coding sequence is capable of being replicated, transcribed and translated in an appropriate host cell.

The term "promoter" is used herein in its ordinary sense to refer to a nucleotide region comprising a DNA regulatory sequence, wherein the regulatory sequence is derived from a gene which is capable of binding RNA polymerase and initiating transcription of a downstream (3'-direction) coding sequence. Transcription promoters can include "inducible promoters" (where expression of a polynucleotide sequence operably linked to the promoter is induced by an analyte, cofactor, regulatory protein, etc.), "repressible promoters" (where expression of a polynucleotide sequence operably linked to the promoter is induced by an analyte, cofactor, regulatory protein, etc.), and "constitutive promoters." In one embodiment, the promoter is a muscle-specific promoter, which includes but is not limited to, a human skeletal actin gene element, a cardiac actin gene element, a desmin promoter, a skeletal alpha-actin (ASKA) promoter, a troponin I (TNNI2) promoter, a myocyte-specific enhancer binding factor mef binding element, a muscle creatine kinase (MCK) promoter, a truncated MCK (tMCK) promoter, a myosin heavy chain (MHC) promoter, a hybrid a-myosin heavy chain enhancer-/MCK enhancer-promoter (MHCK7) promoter, a C5-12 promoter, a murine creatine kinase enhancer element, a skeletal fast-twitch troponin c gene element, a slow-twitch cardiac troponin c gene element, a slow-twitch troponin i gene element, hypoxia- inducible nuclear factor (HIF)-response element (HRE), a steroid-inducible element, and a glucocorticoid response element (gre). In another embodiment, the promoter is an MCK promoter, a tMCK promoter, or an MHCK7 promoter.

The term "operably linked" refers to an arrangement of elements wherein the components so described are configured so as to perform their usual function. Thus, control sequences operably linked to a coding sequence are capable of effecting the expression of the coding sequence. The control sequences need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence.

A promoter "directs the transcription" of a coding sequence in a cell when RNA polymerase will bind the promoter sequence and transcribe the coding sequence into mRNA, which is then translated into the polypeptide encoded by the coding sequence.

"Expression cassette" or "expression construct" refers to an assembly which is capable of directing the expression of the sequence(s) or gene(s) of interest. The expression cassette includes control elements, as described above, such as a promoter which is operably linked to (so as to direct transcription of) the sequence(s) or gene(s) of interest, and often includes a polyadenylation sequence as well. Within certain embodiments of the invention, the expression cassette described herein may be contained within a plasmid construct. In addition to the components of the expression cassette, the plasmid construct may also include, one or more selectable markers, a signal which allows the plasmid construct to exist as single-stranded DNA, at least one multiple cloning site, and a "mammalian" origin of replication (e.g., a SV40 or adenovirus origin of replication).

By "isolated" when referring to a nucleotide sequence, is meant that the indicated molecule is present in the substantial absence of other biological macromolecules such as other nucleotide sequences, chromatin material, etc. Thus, an "isolated nucleic acid molecule which encodes a particular polypeptide" refers to a nucleic acid molecule which is substantially free of other nucleic acid molecules that do not encode the subject polypeptide; however, the molecule may include some additional bases or moieties which do not deleteriously affect the basic characteristics of the composition.

For the purpose of describing the relative position of nucleotide sequences in a particular nucleic acid molecule throughout the instant application, such as when a particular nucleotide sequence is described as being situated "upstream," "downstream," "3," or "5" relative to another sequence, it is to be understood that it is the position of the sequences in the "sense" or "coding" strand of a DNA molecule that is being referred to as is conventional in the art.

The terms "sequence identity", "percent sequence identity", or "percent identical" in the context of nucleic acid or amino acid sequences refers to the residues in the two sequences which are the same when aligned for maximum correspondence. The length of sequence identity comparison may be over the full-length of the genome, the full-length of a gene coding sequence, or a fragment of at least about 500 to 5000 nucleotides, is desired. However, identity among smaller fragments, e.g. of at least about nine nucleotides, usually at least about 20 to 24 nucleotides, at least about 28 to 32 nucleotides, at least about 36 or more nucleotides, may also be desired. The percentage identity of the sequences can be determined by techniques known in the art. For example, homology can be determined by a direct comparison of the sequence information between two polypeptide molecules by aligning the sequence information and using readily available computer programs such as ALIGN, ClustalW2 and BLAST. In one embodiment, when BLAST is used as the alignment tool, the following default parameters: genetic code=standard; filter=none; strand=both; cutoff=60; expect=10; Matrix=BLOSUM62; Descriptions=50 sequences; sort by=HIGH SCORE; Databases=non-redundant, GenBank+EMBL+DDBJ+PDB+GenBank CDS translations+Swiss protein+Spupdate+PIR.

The term "subject" refers to any member of the animal kingdom, which includes, without limitation, humans and nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like. In some embodiments, the subject is a human ranging in age from birth to 2 years, from 1 to 10 years, or ranging from 4 to 15 years, or ranging from 10 to 19 years, or from 20 to 40 years of age, or from 15 to 29 years of age or from 25-55 years, or ranging from 40 to 60 years, or over 50 years or over 60 years or over 65 years or over 70 years. In some embodiments, the subject is an adult (18 years or older). In particular, the subject is a young adult (18-39 years of age), middle aged adult (40-64 year of age) or an older adult or elderly subject (over 65 years of age) or a geriatric subject (over 70 years of age). In some embodiments, the subject is 1 to 10 years of age, or 2 to 12 years of age, 4 to 15 years of age, or 10 to 19 years of age. The subject, in one embodiment, is an adolescent subject, such as a subject ranging in age from 12 to 21 years. In addition, the subject, in one embodiment, is a young adult subject such as a subject ranging in age from 15 to 29 years of age or 18-39 years of age. In some embodiment, the subject is a middle-aged adult or an elderly subject, such that the middle-aged adult may range in age from 25-55 years of age, the older adult subject may range in age over 50 years of age, and the elderly subject may range in age over 65 years of age.

### AAV

Adeno-associated virus (AAV) is a replication-deficient parvovirus, the single-stranded DNA genome of which is about 4.7 kb in length including 145 nucleotide inverted terminal repeat (ITRs). There are multiple serotypes of AAV. The nucleotide sequences of the genomes of the AAV serotypes are known. For example, the nucleotide sequence of the AAV serotype 2 (AAV2) genome is presented in Srivastava et al., J Virol, 45: 555-564 (1983) as corrected by Ruffing et al., J Gen Virol, 75: 3385-3392 (1994). As other examples, the complete genome of AAV-1 is provided in GenBank Accession No. NC_002077; the complete genome of AAV-3 is provided in GenBank Accession No. NC_1829; the complete genome of AAV-4 is provided in GenBank Accession No. NC_001829; the AAV-5 genome is provided in GenBank Accession No. AF085716; the complete genome of AAV-6 is provided in GenBank Accession No. NC_00 1862; at least portions of AAV-7 and AAV-8 genomes are provided in GenBank Accession Nos. AX753246 and AX753249, respectively (see also U.S. Patent Nos. 7,282,199 and 7,790,449 relating to AAV-8); the AAV-9 genome is provided in Gao et al., J. Virol., 78: 6381-6388 (2004); the AAV-10 genome is provided in Mol. Ther., 13(1): 67-76 (2006); and the AAV-11 genome is provided in Virology, 330(2): 375-383 (2004). Cloning of the AAVrh.74 serotype is described in Rodino-Klapac., et al. Journal of translational medicine 5, 45 (2007). Cis-acting sequences directing viral DNA replication (rep), encapsidation/packaging and host cell chromosome integration are contained within the ITRs. Three AAV promoters (named p5, p19, and p40 for their relative map locations) drive the expression of the two AAV internal open reading frames encoding rep and cap genes. The two rep promoters (p5 and p19), coupled with the differential splicing of the single AAV intron (e.g., at AAV2 nucleotides 2107 and 2227), result in the production of four rep proteins (rep 78, rep 68, rep 52, and rep 40) from the rep gene. Rep proteins possess multiple enzymatic properties that are ultimately responsible for replicating the viral genome. The cap gene is expressed from the p40 promoter and it encodes the three capsid proteins VP1, VP2, and VP3. Alternative splicing and non-consensus translational start sites are responsible for the production of the three related capsid proteins. A single consensus polyadenylation site is located at map position 95 of the AAV genome. The life cycle and genetics of AAV are reviewed in Muzyczka, Current Topics in Microbiology and Immunology, 158: 97-129 (1992).

AAV possesses unique features that make it attractive as a vector for delivering foreign DNA to cells, for example, in gene therapy. AAV infection of cells in culture is noncytopathic, and natural infection of humans and other animals is silent and asymptomatic. Moreover, AAV infects many mammalian cells allowing the possibility of targeting many different tissues in vivo. Moreover, AAV transduces slowly dividing and non-dividing cells, and can persist essentially for the lifetime of those cells as a transcriptionally active nuclear episome (extrachromosomal element). The AAV proviral genome is infectious as cloned DNA in plasmids which makes construction of recombinant genomes feasible. Furthermore, because the signals directing AAV replication, genome encapsidation and integration are contained within the ITRs of the AAV genome, some or all of the internal approximately 4.3 kb of the genome (encoding replication and structural capsid proteins, rep-cap) may be replaced with foreign DNA such as a gene cassette containing a promoter, a DNA of interest and a polyadenylation signal. The rep and cap proteins may be provided in trans. Another significant feature of AAV is that it is an extremely stable and hearty virus. It easily withstands the conditions used to inactivate adenovirus (56°C to 65°C for several hours), making cold preservation of AAV less critical. AAV may even be lyophilized. Finally, AAV-infected cells are not resistant to superinfection.

Multiple studies have demonstrated long-term (> 1.5 years) recombinant AAV-mediated protein expression in muscle. See, Clark et al., Hum Gene Ther, 8: 659-669 (1997); Kessler et al., Proc Nat. Acad Sc. USA, 93: 14082-14087 (1996); and Xiao et al., J Virol, 70: 8098-8108 (1996). See also, Chao et al., Mol Ther, 2:619-623 (2000) and Chao et al., Mol Ther, 4:217-222 (2001). Moreover, because muscle is highly vascularized, recombinant AAV transduction has resulted in the appearance of transgene products in the systemic circulation following intramuscular injection as described in Herzog et al., Proc Natl Acad Sci USA, 94: 5804-5809 (1997) and Murphy et al., Proc Natl Acad Sci USA, 94: 13921-13926 (1997). Moreover, Lewis et al., J Virol, 76: 8769-8775 (2002) demonstrated that skeletal myofibers possess the necessary cellular factors for correct antibody glycosylation, folding, and secretion, indicating that muscle is capable of stable expression of secreted protein therapeutics.

Recombinant AAV genomes of the disclosure comprise nucleic acid molecule of the disclosure and one or more AAV ITRs flanking a nucleic acid molecule. AAV DNA in the rAAV genomes may be from any AAV serotype for which a recombinant virus can be derived including, but not limited to, AAV serotypes AAVrh.74, AAV-1, AAV-2, AAV-3, AAV-4, AAV-5, AAV-6, AAV-7, AAV-8, AAV-9, AAV-10, AAV-11, AAV-12, AAV-13 and AAVrh.74. Production of pseudotyped rAAV is disclosed in, for example, WO 01/83692. Other types of rAAV variants, for example rAAV with capsid mutations, are also contemplated. See, for example, Marsic et al., Molecular Therapy, 22(11): 1900-1909 (2014). As noted in the Background section above, the nucleotide sequences of the genomes of various AAV serotypes are known in the art. To promote muscle-specific expression, AAVrh.74 can be used.

DNA plasmids of the disclosure comprise rAAV genomes of the disclosure. The DNA plasmids are transferred to cells permissible for infection with a helper virus of AAV (e.g., adenovirus, E1-deleted adenovirus or herpesvirus) for assembly of the rAAV genome into infectious viral particles. Techniques to produce rAAV particles, in which an AAV genome to be packaged, rep and cap genes, and helper virus functions are provided to a cell are standard in the art. Production of rAAV requires that the following components are present within a single cell (denoted herein as a packaging cell): a rAAV genome, AAV rep and cap genes separate from (i.e., not in) the rAAV genome, and helper virus functions. The AAV rep and cap genes may be from any AAV serotype for which recombinant virus can be derived and may be from a different AAV serotype than the rAAV genome ITRs, including, but not limited to, AAV serotypes AAV-1, AAV-2, AAV-3, AAV-4, AAV-5, AAV-6, AAV-7, AAVrh.74, AAV-8, AAV-9, AAV-10, AAV-11, AAV-12 and AAV-13. Production of pseudotyped rAAV is disclosed in, for example, WO 01/83692 which is incorporated by reference herein in its entirety.

A method of generating a packaging cell is to create a cell line that stably expresses all the necessary components for AAV particle production. For example, a plasmid (or multiple plasmids) comprising a rAAV genome lacking AAV rep and cap genes, AAV rep and cap genes separate from the rAAV genome, and a selectable marker, such as a neomycin resistance gene, are integrated into the genome of a cell. AAV genomes have been introduced into bacterial plasmids by procedures such as GC tailing (Samulski et al., 1982, Proc. Natl. Acad. S6. USA, 79:2077-2081), addition of synthetic linkers containing restriction endonuclease cleavage sites (Laughlin et al., 1983, Gene, 23:65-73) or by direct, blunt-end ligation (Senapathy & Carter, 1984, J. Biol. Chem., 259:4661-4666). The packaging cell line is then infected with a helper virus such as adenovirus. The advantages of this method are that the cells are selectable and are suitable for large-scale production of rAAV. Other examples of suitable methods employ adenovirus or baculovirus rather than plasmids to introduce rAAV genomes and/or rep and cap genes into packaging cells.

General principles of rAAV production are reviewed in, for example, Carter, 1992, Current Opinions in Biotechnology, 1533-539; and Muzyczka, 1992, Curr. Topics in Microbial. and Immunol., 158:97-129). Various approaches are described in Ratschin et al., Mol. Cell. Biol. 4:2072 (1984); Hermonat et al., Proc. Natl. Acad. Sci. USA, 81:6466 (1984); Tratschin et al., Mo1. Cell. Biol. 5:3251 (1985); McLaughlin et al., J. Virol., 62:1963 (1988); and Lebkowski et al., Mol. Cell. Biol., 7:349 (1988). Samulski et al., J. Virol., 63:3822-3828 (1989); U.S. Patent No. 5,173,414; WO 95/13365 and corresponding U.S. Patent No. 5,658.776 ; WO 95/13392; WO 96/17947; PCT/US98/18600; WO 97/09441 (PCT/US96/14423); WO 97/08298 (PCT/US96/13872); WO 97/21825 (PCT/US96/20777); WO 97/06243 (PCT/FR96/01064); WO 99/11764; Perrin et al. Vaccine 13:1244-1250 (1995); Paul et al. Human Gene Therapy 4:609-615 (1993); Clark et al. Gene Therapy 3:1124-1132 (1996); U.S. Patent. No. 5,786,211; U.S. Patent No. 5,871,982; and U.S. Patent. No. 6,258,595. The foregoing documents are hereby incorporated by reference in their entirety herein, with particular emphasis on those sections of the documents relating to rAAV production.

The disclosure thus provides packaging cells that produce infectious rAAV. In one embodiment packaging cells may be stably transformed cancer cells such as HeLa cells, 293 cells and PerC.6 cells (a cognate 293 line). In another embodiment, packaging cells are cells that are not transformed cancer cells, such as low passage 293 cells (human fetal kidney cells transformed with E1 of adenovirus), MRC-5 cells (human fetal fibroblasts), WI-38 cells (human fetal fibroblasts), Vero cells (monkey kidney cells) and FRhL-2 cells (rhesus fetal lung cells).

Recombinant AAV (i.e., infectious encapsidated rAAV particles) of the disclosure comprise a rAAV genome. In exemplary embodiments, the genomes of both rAAV lack AAV rep and cap DNA, that is, there is no AAV rep or cap DNA between the ITRs of the genomes. Examples of rAAV that may be constructed to comprise the nucleic acid molecules of the disclosure are set out in International Patent Application No. PCT/US2012/047999 (WO 2013/016352) incorporated by reference herein in its entirety.

The pNLREP2-Caprh74 is an AAV helper plasmid that encodes the 4 wild-type AAV2 rep proteins and the 3 wild-type AAV VP capsid proteins from serotype rh74. A schematic map of the pNLREP2-Caprh74 plasmid is shown in Figure 3.

The pHELP adenovirus helper plasmid is 11,635 bp and was obtained from Applied Viromics. The plasmid contains the regions of adenovirus genome that are important for AAV replication, namely E2A, E4ORF6, and VA RNA (the adenovirus E1 functions are provided by the 293 cells). The adenovirus sequences present in this plasmid only represents -40% of the adenovirus genome, and does not contain the cis elements critical for replication such as the adenovirus terminal repeats. Therefore, no infectious adenovirus is expected to be generated from such a production system. A schematic map of the pHELP plasmid is shown in Figure 4.

The rAAV may be purified by methods standard in the art such as by column chromatography or cesium chloride gradients. Methods for purifying rAAV vectors from helper virus are known in the art and include methods disclosed in, for example, Clark et al., Hum. Gene Ther., 10(6): 1031-1039 (1999); Schenpp and Clark, Methods Mol. Med., 69 427-443 (2002); U.S. Patent No. 6,566,118 and WO 98/09657.

In another embodiment, the disclosure contemplates compositions comprising rAAV of the present disclosure. Compositions of the disclosure comprise rAAV and a pharmaceutically acceptable carrier. The compositions may also comprise other ingredients such as diluents and adjuvants. Acceptable carriers, diluents and adjuvants are nontoxic to recipients and are preferably inert at the dosages and concentrations employed and include buffers and surfactants such as pluronics.

Titers of rAAV to be administered in methods of the disclosure will vary depending, for example, on the particular rAAV, the mode of administration, the treatment goal, the individual, and the cell type(s) being targeted, and may be determined by methods standard in the art. Titers of rAAV may range from about 1×10⁶, about 1×10⁷, about 1×10⁸, about 1×10⁹, about 1x1010, about 1x1011, about 1x1012, about 1×1013 to about 1x1014 or more DNase resistant particles (DRP) per ml. Dosages may also be expressed in units of viral genomes (vg). Exemplary disclosed doses include 1E11 vg, 3E12 vg and 6E12 vg. One exemplary method of determining encapsilated vector genome titer uses quantitative PCR such as the methods described in (Pozsgai et al., Mol. Ther. 25(4): 855-869, 2017).

The dose of rAAV to be administered in methods disclosed herein will vary depending, for example, on the particular rAAV, the mode of administration, the treatment goal, the individual, and the cell type(s) being targeted, and may be determined by methods standard in the art. Titers of each rAAV administered may range from about 1×10⁶, about 1×10⁷, about 1x108, about 1×109, about 1x1010, about 1x1011, about 1x1012, about 1x1013, about 1x1014, about 2×10¹⁴, or to about 1×10¹⁵ or more DNase resistant particles (DRP) per ml. Dosages may also be expressed in units of viral genomes (vg) (i.e., 1×10⁷ vg, 1×10⁸ vg, 1×10⁹ vg, 1×10¹⁰ vg, 1×10¹¹ vg, 1×10¹² vg, 1×10¹³ vg, 1×10¹⁴ vg, 2×10¹⁴ vg, 1×10¹⁵vg respectively). Dosages may also be expressed in units of viral genomes (vg) per kilogram (kg) of bodyweight (i.e., 1×10¹⁰ vg/kg, 1×10¹¹ vg/kg, 1×10¹²vg/kg, 1×10¹³ vg/kg, 1×10¹⁴ vg/kg, 1.25×10¹⁴ vg/kg, 1.5×10¹⁴ vg/kg, 1.75×10¹⁴ vg/kg, 2.0×10¹⁴ vg/kg, 2.25×10¹⁴ vg/kg, 2.5×10¹⁴ vg/kg, 2.75×10¹⁴ vg/kg, 3.0×10¹⁴ vg/kg, 3.25×10¹⁴ vg/kg, 3.5×10¹⁴ vg/kg, 3.75×10¹⁴ vg/kg, 4.0×10¹⁴vg/kg, 1×10¹⁵ vg/kg respectively). Methods for titering AAV are described in Clark et al., Hum. Gene Ther., 10: 1031-1039 (1999).

Transduction with rAAV may also be carried out in vitro. In one embodiment, desired target muscle cells are removed from the subject, transduced with rAAV and reintroduced into the subject. Alternatively, syngeneic or xenogeneic muscle cells can be used where those cells will not generate an inappropriate immune response in the subject.

### Treatment of muscular dystrophy, including Duchenne Muscular Dystrophy ("DMD") or Becker's Muscular Dystrophy ("BMD") with a with an rAAV comprising microdystrophin

The invention encompasses a method of treating muscular dystrophy in a human subject in need thereof comprising the step of administering a recombinant adeno-virus associated (rAAV) comprising a heterologous nucleotide sequence encoding microdystrophin and further comprising administering a immunosuppressing regimen. In various embodiments of the invention, the methods comprise administering a recombinant adeno-virus associated (rAAV) comprising a heterologous nucleotide sequence encoding microdystrophin and further comprise administering an anti-inflammatory steroid, including for example prednisone.

In all embodiments of the invention directed to treating a muscular dystrophy with an rAAV comprising microdystrophin and further comprising administering an immuno-suppressing regimen or an anti-inflammatory steroid, the rAAV or microdystrophin encoding nucleotide sequences that can be utilized in the methods of the invention includes those described in WO-2020/123645, WO-2019/209777, WO-2019/195362, WO-2016/115543, WO-2019118806, WO-2017/221145, the contents of each of which is hereby incorporated herein by reference, and include SGT-001, zildistrogene varoparvovec, and PF-06939926.

### Treatment of Limb-Girdle Muscular Dystrophies

Treatment of a Limb-Girdle Muscular Dystrophy ("LGMD") is an aspect of the invention. It is recognized that the methods of the invention described herein can be utilized in the treatment of Limb-Girdle muscular dystrophies by employing rAAV vectors that are useful for treating such dystrophies. Such rAAV vectors include AAVrh.74.tMCK.CAPN3 (SEQ ID NO: 1) also described in PCT/US2019/039893 (WO 2020/06458), the contents of each of which is hereby incorporated herein by reference.

All publications, patents and patent applications cited herein, whether supra or infra, are hereby incorporated by reference in their entirety.

The following EXAMPLES are provided by way of illustration and not limitation. Described numerical ranges are inclusive of each integer value within each range and inclusive of the lowest and highest stated integer.

### Examples

Aspects and embodiments are illustrated by the following examples. Example 1 describes production of AAVrh.74.tMCK.CAPN3. Example 2 describes studies investigating rAAVrh74.tMCK.hCAPN3 vector potency. Example 3 describes long term treatment efficacy of rAAVrh74.tMCK.hCAPN3 determined by run to exhaustion test. Example 4 describes long term treatment efficacy of rAAVrh74.tMCK.hCAPN3 determined by in vivo muscle contractibility assay. Example 5 describes long term treatment efficacy of rAAVrh74.tMCK.hCAPN3 determined by histopathology analysis. Example 6 long term treatment efficacy of rAAVrh74.tMCK.hCAPN3 determined by vector copy number analysis. Example 7 describes long term treatment efficacy of rAAVrh74.tMCK.hCAPN3 determined by vector copy number analysis. Example 7 describes analysis of CAPN3 expression and functional correlation. Example 8 describes GC1α expression levels and muscle remodeling. Example 9 describes toxicology studies after administration of rAAVrh74.tMCK.CAPN3.

### Example 1

### Production of rAAVrh.74.tMCK.CAPN3

An AAV vector (named rAAVrh74.tMCK.CAPN3) carrying the human *CAPN3* cDNA coding sequence consisting of 24 exons under the control of tMCK promoter.under a truncated muscle specific MCK promoter (tMCK promoter) was produced. Figure 1A shows a schematic diagram of the cassette used for gene transfer including a tMCK promoter, an intron to enhance gene expression, the full human *CAPN3* cDNA, and the SV40 polyadenylation signal. A circular map of the plasmid pAAV.tMCK.hCAPN3 vector is shown in Figure 11A and the linear map of the plasmid is shown in Figure 11B.

The nucleotide sequence of pAAV.tMCK.hCAPN3 vector is provided as SEQ ID NO: 13 or SEQ ID NO: 20. Table A shows the molecular features of plasmid pAAV.tMCK.hCAPN3.

| Table A. Molecular Features of plasmid pAAV.tMCK.hCAPN3 | | | | |
|---|---|---|---|---|
| TYPE | START | END | NAME | DESCRIPTION |
| REGION | 1 | 28 | 5' ITR | Wild-type AAV2 inverted terminal repeat |
| REGION | 165 | 884 | tMCK | |
| REGION | 937 | 1069 | Chimeric intron | 5' donor site from human β-globin gene and the branchpoint and 3' splice acceptor site from IgG heavy chain variable region |
| REGION | 1101 | 1106 | Kozak sequence | |
| GENE | 1107 | 3572 | hCAPN3 cDNA | Wild type human CAPN3 cDNA |
| REGION | 3581 | 3780 | PolyA | Synthetic PolyA |
| REGION | 3850 | 3977 | 3' ITR | Wild-type AAV2 inverted terminal repeat |
| REGION | 4261 | 4804 | Ori | Plasmid origin of replication |
| GENE | 5106 | 5900 | NeoR/KanR | |

A single-stranded (ss) AAV expression cassette with tMCK (triple-muscle creatine kinase enhancer) promoter and a Kanamycin resistance sequence was designed. The human *CAPN3* open reading frame, from a gene fragment (Eurofins Genomics, USA), into downstream of the tMCK promoter followed by a chimeric intron and KOZAK sequence. The final cassette was sequenced before it was sent to AAV production. AAV-hCAPN3 vectors with rh74 serotype were produced, purified, and tittered. For example, the titer can be determined by using a supercoiled plasmid as a quantification control, Aliquoted vectors were kept at -80 °C until use.

Production was accomplished using a standard three-plasmid DNA/CaPO₄ precipitation method using HEK293 cells. 293 cells were maintained in DMEM supplemented with 10% fetal bovine serum (FBS) and penicillin and streptomycin. Aliquoted viruses were kept at -80 °C until use.

### Example 2

### rAAVrh74.tMCK.hCAPN3 Vector Potency

In vivo biopotency testing of rAAVrh74.tMCK.hCAPN3 was carried out first using IM route, following injection of AAVrh74.tMCK.hCAPN3 (1×10¹¹ vg) into tibialis anterior muscle in CAPN3KO mice (n=3) at 4 weeks post-gene delivery. RT-qPCR and western blot analyses showed human CAPN3 transcripts and 94 kDa full-length CAPN3 protein (Figure 1B) indicating the production of CAPN3 was achieved. Moreover, histological analysis showed that CAPN3 replacement increased the muscle fiber diameter of the tibialis anterior compared to the control (ringer lactate injected) muscle (Figure 1C).

As the next step, biopotency of the vector was assessed following systemic delivery via tail vein of CAPN3KO mice at low and high doses (LD, at 3x1012 vg, n=4, and HD, at 6×10¹² vg, n=3) and generated biodistribution and short-term treatment efficacy data at 4 weeks post-injection in both cohorts. hCAPN3 expression in the CAPN3KO muscle following systemic delivery was variable amongst different muscles and especially in the LD cohort which was significantly low compared to the IM delivery at 1×10¹¹ vg (< 1% of IM delivery for tibialis anterior muscle). Accordingly, the full-length 94kDa protein was below the limit of detection by western blot (Figure 1B). However, robust gene expression and prominent amounts of full-length calpain 3 protein were exhibited following the 6×10¹² vg systemic dosage in the HD cohort, suggesting a dose-dependent response following AAVrh74.tMCK.hCAPN3 delivery (Figure 1B). Although the number of CAPN3KO mice used in the biopotency experiments was small, additional data from these mice were obtained to assess if short term functional or histological efficacy can be detected. Improvements in these parameters were observed in both cohorts of CAPN3KO mice that received either 3×10¹² or 6×10¹² vg of vector systemically at 4 weeks post-gene injection compared to ringers lactate injected (n=3) counterparts (Figure 1D and E). In the run-to-exhaustion test, both treatment cohorts improved significantly compared to the untreated cohort and there was no dose-related difference in performance. Accompanying this, muscle fiber diameter showed an increase toward normalization in both cohorts, again with no dose-related difference (Table S1).

**Table 1S. Fiber size analysis of the tibialis anterior muscle CAPN3KO mice at 4 weeks post-gene delivery and age-matched WT mice.**

| | Untreated (n = 3) | | Low Dose (n = 4) | |
|---|---|---|---|---|
| | **Number** | **Diameter** | **Number** | **Diameter** |
| **STO** | 214.5 ± 32.0 | 27.15 ± 0.7 | 116.0 ± 11.9 | 30.65 ± 0.6 |
| **FTO** | 91.0 ± 6.7 | 35.4 ± 1.3 | 111.8 ± 7.4 | 40.78 ± 0.8* |
| **FTG** | 113.8 ± 7.6 | 39.67 ± 1.6 | 100.8 ± 71 | 44.11 ± 0.8 |
| **All Fiber** | 328.5 ± 8.4 | 32.47 ± 1.5 | 328.5 ± 8.4 | 38.18 ± 0.2 |
| | | | | |

| | High Dose (n= 3) | | WT | (n=3) |
|---|---|---|---|---|
| | **Number** | **Diameter** | **Number** | **Diameter** |
| **STO** | 147.0 ± 20.1 | 31.70 ± 1.6 | 177.3 ± 46.1 | 30.02 ± 1.2 |
| **FTO** | 115.0 ± 30.6 | 38.82 ± 0.9 | 92.7 ± 15.1 | 40.66 ± 2.7* |
| **FTG** | 75.3 ± 4.1 | 45.83 ± 1.1* | 91.7 ± 13.3 | 46.07 ± 2.3** |
| **All Fiber** | 337.3 ± 29.6 | 37.27 ± 11 | 361.7 ± 43.1 | 37.00 ± 2.2 |

| | | | | |
|---|---|---|---|---|
| Data represented as mean ± SEM. *p<0.05, **p<0.01 (compared to UT), Two-way ANOVA, Tukey's multiple comparisons test. | | | | |

In addition, no histopathological evidence of cardiac toxicity was observed following systemic injection of AAVrh7.4.tMCK.hCAPN3 vector at 4 weeks in either cohort. Microscopic examination of sections from two levels (superficial and deep sections from ventricles) through the apex of the heart revealed no inflammation, necrosis, regeneration, or fibrotic changes in the cardiac muscle indicating no short term cardiotoxic effects from systemic delivery of the vector at these two doses (Figure 2A). No detectable CAPN3 protein bands in the heart tissue was observed by western blot in either cohort (Figure 2B).

### Example 3

### Long Term Treatment Efficacy of rAAVrh74.tMCK.hCAPN3 Determined by Run to Exhaustion Test

A study to investigate the long term efficacy of systemic AAVrh74.tMCK.hCAPN3 gene therapy at the same low and high doses in young and old age groups of CAPN3KO mice using functional, in vivo muscle physiology and histological outcomes was carried out. In these studies, AAVrh74.tMCK.CAPN3 was systemically delivered to two different age groups of CAPN3KO mice, 6-10 week-old and 21-24 week-old. Each group included two treatment cohorts receiving LD (3×10¹² vg) and HD (6×10¹² vg) of the vector and age-matched untreated (UT) control CAPN3KO mice. Treatment efficacy was tested at 20 weeks post-gene delivery using functional (treadmill - run to exhaustion test), physiological *(in vivo* muscle contractility assay) and histopathological outcomes. Table 2S summarizes the experimental cohorts in each age group.

**Table S2. Experimental cohorts of CAPN3KO mice included in the study.**

| **Groups** | **Age at treatment** | **Treatment cohorts** | **Treatment Dose** | **Total # of mice** | **Female** | **Male** | **Treatment Duration** | **Age at End Point** |
|---|---|---|---|---|---|---|---|---|
| | | Low dose | 3×10¹² vg | 12 | 6 | 6 | | |
| Young | 6-10 wks | High Dose | 6x 10¹² vg | 12 | 6 | 6 | 20 wks | 26-30 wks |
| | | Untreated | Ringer's Lactate | 13 | 7 | 6 | | |
| | | Low dose | 3×10¹² vg | 12 | 6 | 6 | | |
| Old | 20-24 wks | High Dose | 6×10¹² vg | 12 | 5 | 7 | 20 wks | 40-44 wks |
| | | Untreated | Ringer's Lactate | 15 | 8 | 7 | | |

Run to exhaustion treadmill test was used to assess the functional efficacy of CAPN3 gene therapy. At both doses in young and older age groups, gene therapy improved treadmill performance at 20 weeks post gene delivery, leading to striking increases in run distance and time until exhaustion compared to the age-matched UT-CAPN3KO cohort. In the older age group, CAPN3 gene therapy led to a 119% increase in distance run to exhaustion in the LD (167.2 ± 14.1 m, n=12), and a 142% increase in distance run to exhaustion in the in HD (184.5 ± 19.2 m, n=12) compared to UT cohort (76.2 ± 6.2 m, n=14; p≤0.0001 for UT vs. both treatment cohorts, Figure 3A). Similarly, in the young-age group, treadmill data indicated a 72% performance increase in the LD (156.7 ± 14.0 m, n=12) and an 88% increase in distance run to exhaustion in the HD (171.4 ± 9.6 m, n=12) compared to UT cohort (93.9 ± 6.0 m, n=12; p≤0.001 for UT vs. both treatment cohorts, Figure 3B). The UT cohort of young age group performed better than the UT counterparts from the old age group without reaching statistical significance. The treadmill performance did not differ significantly among the treated cohorts suggesting that AAVrh74.tMCK.CAPN3 vector at LD was sufficient to give rise to functional improvements, comparable to the HD cohorts. On the other hand, comparing male (n=7) and female (n=5) performance at HD, the females performed significantly better (p=0.006, Figure 3C) whereas in the young group, gender difference did not influence treadmill performance (Figure 3D). Collectively, these results indicate that age at the time of vector-administration and the vector dose did not affect the treadmill performance in this model.

### Example 4

### Long Term Treatment Efficacy of rAAVrh74.tMCK.hCAPN3 Determined by In Vivo Muscle Contractibility Assay

*In vivo* muscle contractility assay was used to assess muscle physiology in response to CAPN3 gene therapy. This assay measures the aggregate torque produced by stimulating the tibial nerve to achieve gastrocnemius muscle torque around the ankle joint.

In the old group of CAPN3KO mice, varying degrees of heel cord contractures were observed in the untreated males as part of the aged CAPN3KO phenotype, recapitulating phenotypical differences between males and females in humans (Fanin et al., Clin. Neuropathology 33:179-801, 2014). The contractures were also observed in males of the LD treatment cohort at endpoint (4/6 mice); however, males in the HD cohort were rescued with gene therapy. Taking this variability into consideration, the HD and LD- combined treatment cohort (n=23) were compared to the UT cohort (n=28) for the analysis of in vivo muscle contractility data. Treated mice displayed higher force for both maximum twitch and tetanic responses. The maximum twitch response of the treated group (2.43 ± 0.1 mN.m) showed a 27% increase (p= 0.00101) and maximum tetanic response (10.58 ± 0.3 mN.m) improved by 12% (p= 0.047) compared to untreated group (twitch, 1.92 ± 0.1 mN.m; tetanic, 9.44 ± 0.4 mN.m, Figure 4A and B).

In the young group of CAPN3KO mice which displayed a milder phenotype to begin with (see treadmill performance of UT cohorts from young vs. old age groups in Fig 3A and B), the time-course change of muscle contractility was assessed by comparing baseline measurements to endpoint at 20 weeks post-treatment. The percent change of twitch and tetanic responses were calculated for each mouse. With treatment, the average percent increase in maximum twitch response was 5% in the combined treatment group (n=23) while an average of 9 % decrease occurred in the untreated group (n=13, p=0.20, Figure 4C). We observed an 8% increase in the average percent of maximum tetanic response in the treated mice (n=22) while the untreated group (n=13) had a 9% decrease (p=0.002, Figure 4D).

### Example 5

### Long Term Treatment Efficacy of rAAVrh74.tMCK.hCAPN3 Determined by Histopathology Analysis

The effects of CAPN3 gene therapy in CAPN3KO mice upon muscle fiber size and fiber type composition were quantified at 20 weeks post gene injection in distal and proximal lower limb muscles (gastrocnemius, tibialis anterior, and quadriceps) as well as in triceps muscle from upper limbs using succinic dehydrogenase (SDH) stain which delineates fibers with high mitochondria content, i.e. fatigue-resistant slow-twitch oxidative (STO) or type 1 fibers, the fast-twitch oxidative (FTO) fibers with intermediate staining intensity and fast-twitch glycolytic (FTG) fibers with lightest staining intensity (Figure 5A). Fiber size measurements were done on the representative images from 3 zones of the muscle (deep, intermediate, and superficial, designated as Zone 1, 2, and 3) as illustrated in the tibialis anterior muscle from the old age group (Figure 5B and 5C,Table S3). Compared to WT, muscle bulk and muscle fiber size of all fiber types were smaller in UT-Capn3 null muscles, corroborating previous reports (Kramerova et al. Hum. Mol. Genet. 13: 1373-1388, 2004; Yalvac et al. Skeletal Muscles 7:27, 2017) (see Tables S4-S13). CAPN3 gene therapy in the KO mice resulted in a significant fiber size increase in all fiber types in both LD and HD cohorts compared to UT cohort (Table S3). Overall, increases in fiber size were observed in other muscles from both old and young age groups of CAPN3KO mice (Tables S4-13).

**Table S3. Fiber size analysis of the tibialis anterior muscle from old CAPN3KO mice at 20 weeks post-gene delivery and age-matched WT mice.**

| | Untreated (n=15) | | Low Dose (n=12) | | High Dose (n=12) | | WT (n=8) | |
|---|---|---|---|---|---|---|---|---|
| | **Number** | **Diameter (µm)** | **Number** | **Diameter (µm)** | **Number** | **Diameter (µm)** | **Number** | **Diameter (µm)** |
| **STO** | 87.2 ± 8.3 | 26.71 ± 0.6 | 92.0 ± 6.6 | 29.96 ± 0.8** | 85.3 ± 11.5 | 32.24 ± 1.0** | 91.6 ± 10.1 | 30.49 ± 1.0** |
| **FTO** | 86.1 ± 4.5 | 34.48 ± 0.6 | 86.0 ± 4.9 | 37.88 ± 1.0** | 91.3 ± 6.2 | 40.31 ± 1.1** | 66.3 ± 3.5 | 36.33 ± 1.1^{$$} |
| **FTG** | 142.0 ± 61 | 42.08 ± 0.6 | 126.0 ± 7.7 | 46.21 ± 0.9** | 119.9 ± 7.0^{#} | 48.52 ± 1.1** | 162.5 ± 5.7 | 41.54 ± 2.0^{##$$} |
| **All Fibers** | 315.3 ± 13.8 | 35.85 ± 0.5 | 304.0 ± 8.9 | 38.96 ± 0.7** | 296.5 ± 17.9 | 41.39 ± 1.0** | 320.4 ± 13.6 | 37.23 ± 0.9^{$$} |

| | | | | | | | | |
|---|---|---|---|---|---|---|---|---|
| Data represented as mean ± SEM. *p<0.05, **p<0.01 (compared to UT), ^{#}p<0.05, ^{##}p<0.01 (compared to LD), ^{$}P<0.05, ^{$$}p<0.01 (compared to HD), unpaired t-test. | | | | | | | | |

**Table S4. Fiber size analysis of the gastrocnemius muscle from old CAPN3KO mice at 20 weeks post-gene delivery and age-matched WT mice.**

| | Untreated (n=8) | | Low Dose (n=5) | | High Dose (n=6) | | WT (n=8) | |
|---|---|---|---|---|---|---|---|---|
| | **Number** | **Diameter (µm)** | **Number** | **Diameter (µm)** | **Number** | **Diameter (µm)** | **Number** | **Diameter (µm)** |
| **STO** | 102.9 ± 8.3 | 26.41 ± 0.4 | 118.0 ± 8.0 | 26.91 ± 0.3 | 123.3 ± 6.9 | 29.50 ± 0.4^{**##} | 101.9 ± 6.8 | 29.96 ± 0.7^{**##} |
| **FTO** | 65.8 ± 5.6 | 35.02 ± 0.9 | 62.0 ± 4.3 | 33.82 ± 0.6 | 61.3 ± 4.1 | 36.90 ± 0.2^{#} | 48.5 ± 2.6 | 34.07 ± 0.7^{$} |
| **FTG** | 148.1 ± 5.2 | 43.25 ± 0.8 | 168.2 ± 5.7 | 42.09 ± 0.7 | 137.7 ± 3.4^{#} | 44.66 ± 0.7 | 170.5 ± 6.3^{*$$$} | 41.19 ± 1.0^{$} |
| **All Fibers** | 316.8 ± 12.1 | 36.18 ± 0.74 | 348.2 ± 8.7 | 35.37 ± 0.4 | 322.3 ± 6.9 | 37.35 ± 0.4^{#} | 320.9 ± 9.7 | 36.51 ± 0.8 |

| | | | | | | | | |
|---|---|---|---|---|---|---|---|---|
| Data represented as mean ± SEM. *p<0.05, **p<0.01 (compared to UT), ^{#}p<0.05, ^{##}p<0.01 (compared to LD), ^{$}p<0.05, ^{$$}p<0.01 (compared to HD), unpaired t-test. | | | | | | | | |

**Table S5. Fiber size analysis of the quadriceps muscle from old CAPN3KO mice at 20 weeks post-gene delivery and age-matched WT mice**

| | Untreated (n=7) | | High Dose (n=7) | | WT (n=7) | |
|---|---|---|---|---|---|---|
| | **Number** | **Diameter (µm)** | **Number** | **Diameter (µm)** | **Number** | **Diameter (µm)** |
| **STO** | 52.0 ± 5.2 | 26.34 ± 1.2 | 56.0 ± 13.4 | 30.27 ± 1.5 | 57.6 ± 9.6 | 31.66 ± 1.0** |
| **FTO** | 44.4 ± 4.7 | 34.63 ±1.7 | 50.9 ± 5.4 | 35.67 ± 1.5 | 39.0 ± 3.7 | 37.59 ± 0.8 |
| **FTG** | 169.7 ± 11.0 | 44.99 ± 1.5 | 157.4 ±9.9 | 46.77 ±1.8 | 162.0 ± 9.0 | 46.56 ± 1.4 |
| **All Fibers** | 232.9 ± 34.4 | 39.59 ± 1.5 | 264.3 ± 24.2 | 41.50 ± 1.9 | 258.6 ± 16.4 | 42.02 ± 1.3 |

| | | | | | | |
|---|---|---|---|---|---|---|
| Data represented as mean ± SEM. **p<0.01 (compared to UT), unpaired t-test. | | | | | | |

**Table S6. Fiber size analysis of the tibialis anterior muscle of CAPN3KO mice from young age group at 20 weeks post-gene delivery**

| | Untreated (n=6) | | Low Dose (n=6) | | High Dose | (n=6) |
|---|---|---|---|---|---|---|
| | **Number** | **Diameter (µm)** | **Number** | **Diameter (µm)** | **Number** | **Diameter (µm)** |
| **STO** | 67.0 ± 8.3 | 24.29 ± 0.5 | 48.2 ± 5.0 | 27.47 ± 0.4** | 94.0 ± 5.9^{##} | 27.13 ± 0.9 |
| **FTO** | 75.2 ± 4.6 | 29.06 ± 0.6 | 74.8 ± 6.0 | 33.69 ± 0.4** | 88.7 ± 6.9 | 33.35 ± 1.0* |
| **FTG** | 215.8 ± 62 | 38.40 ± 0.7 | 157.5 ± 6.0** | 44.27 ± 0.5** | 169.2 ± 7.9* | 41.97 ±1.3 |
| **All Fibers** | 358.0 ± 14.0 | 33.95 ± 0.8 | 289.3 ± 8.8* | 38.88 ± 0.5** | 351.8 ±17.2 | 35.86 ± 1.1 |

| | | | | | | |
|---|---|---|---|---|---|---|
| Data represented as mean ± SEM. *p<0.05, **p<0.01 (compared to UT), ^{#}p<0.05, ^{##}p<0.01 (compared to LD), unpaired t-test. | | | | | | |

**Table S7. Fiber size analysis of the gastrocnemius muscle of CAPN3KO mice from young age at 20 weeks post-gene delivery and age-matched WT mice.**

| | Untreated (n=8) | | Low Dose (n=9) | | High Dose (n=8) | | WT (n=4) | |
|---|---|---|---|---|---|---|---|---|
| | **Number** | **Diameter (µm)** | **Number** | **Diameter (µm)** | **Number** | **Diameter (µm)** | **Number** | **Diameter (µm)** |
| **STO** | 106.1 ± 6.8 | 25.17 ± 0.8 | 83.00 ± 5.7* | 30.10 ± 0.9*** | 96.9 ± 8.2 | 25.20 ± 0.3^{###} | 131.3 ± 4.7*^{####$} | 29.89 ± 0.2**^{$$$$} |
| **FTO** | 53.5 ± 1.4 | 32.46 ± 0.8 | 75.2 ± 7.8* | 35.99 ± 1.2* | 84.5 ± 9.9* | 32.00 ± 0.6 ^{##} | 38.8 ± 6.68*^{#$} | 36.16 ± 0.8*^{$$} |
| **FTG** | 196.3 ± 10.4 | 39.42 ± 1.2 | 153.9 ± 10.0* | 44.70 ± 1.1** | 161.4 ± 10.9* | 42.50 ±1.3 | 171.0 ± 5.4 | 42.40 ± 0.6 |
| **All Fibers** | 355.9 ± 15.0 | 34.12 ± 0.9 | 312.1 ± 12.4** | 38.75 ± 1.0** | 341.4 ± 12.5 | 35.29 ± 0.6^{#} | 341.0 ± 6.6 | 36.84 ± 0.3 |

| | | | | | | | | |
|---|---|---|---|---|---|---|---|---|
| Data represented as mean ± SEM. *p<0.05, **p<0.01 (compared to UT), ^{#}p<0.05, ^{##}p<0.01 (compared to LD), ^{$}p<0.05, ^{$$}p<0.01 (compared to HD), unpaired t-test. | | | | | | | | |

**Table S8. Fiber type distributions (%) in the tibialis anterior muscle of CAPN3KO mice from old group and age-matched WT mice.**

| | UT | LD | HD | WT |
|---|---|---|---|---|
| **STO** | 27.1 ± 1.4 | 30.2 ± 2.1 | 27.7 ± 2.4 | 28.1 ± 2.2 |
| **FTO** | 27.6 ± 1.3 | 28.2 ± 1.1 | 31.1 ± 1.5 | 20.7 ± 0.9 ^{**###$$$} |
| **FTG** | 45.4 ± 1.4 | 41.6 ± 2.5 | 41.2 ± 2.2 | 51.1 ± 2.0^{*#$$} |

| | | | | |
|---|---|---|---|---|
| Data represented as mean ± SEM. *p<0.05, **p<0.01 (compared to UT), ^{#}p<0.05, ^{##}p<0.01 (compared to LD), ^{$}p<0.05, ^{$$}p<0.01 (compared to HD), unpaired t-test. | | | | |

**Table S9. Fiber type distributions (%) in the triceps muscle of CAPN3KO mice from old group and age-matched WT mice.**

| | UT | LD | HD | WT |
|---|---|---|---|---|
| **STO** | 26.2 ± 2.5 | 26.8. ± 1.8 | 24.5 ± 2.0 | 22.6 ± 1.2 |
| **FTO** | 24.5 ± 1.5 | 25.4 ± 1.3 | 24.2 ± 1.4 | 18.3 ± 0.4 **^{##$$} |
| **FTG** | 49.3 ± 2.3 | 47.8 ± 2.7 | 51.3 ± 2.9 | 59.1 ± 1.0**^{##} |

| | | | | |
|---|---|---|---|---|
| Data represented as mean ± SEM. *p<0.05, **p<0.01 (compared to UT), ^{#}p<0.05, ^{##}p<0.01 (compared to LD), ^{$}P<0.05, ^{$$}p<0.01 (compared to HD), unpaired t-test. | | | | |

**Table S10. Fiber type distributions (%) in the gastrocnemius muscle of CAPN3KO mice from old group and age-matched WT mice**

| | UT | LD | HD | WT |
|---|---|---|---|---|
| **STO** | 32.0 ± 2.1 | 33.5. ± 1.8 | 38.0 ± 1.6 | 31.6 ± 1.7 |
| **FTO** | 20.3 ± 1.1 | 17.8 ± 1.1 | 19.0 ± 1.1 | 15.1 ± 0.6^{∗∗$} |
| **FTG** | 47.7 ± 2.5 | 48.7 ± 2.0 | 43.1 ± 1.7 | 53.2 ± 1.6^{$$} |

| | | | | |
|---|---|---|---|---|
| Data represented as mean ± SEM. *p<0.05, **p<0.01 (compared to UT), ^{#}p<0.05, ^{##}p<0.01 (compared to LD), ^{$}p<0.05, ^{$$}p<0.01 (compared to HD), unpaired t-test. | | | | |

**Table S11. Fiber type distributions (%) in the quadriceps muscle of CAPN3KO mice from old group and age-matched WT mice**

| | UT | HD | WT |
|---|---|---|---|
| **STO** | 19.6 ± 1.5 | 19.5 ± 3.1 | 21.4 ± 2.5 |
| **FTO** | 16.5 ± 1.2 | 19.4 ± 1.6 | 15.4 ± 1.6 |
| **FTG** | 64.0 ± 1.9 | 61.1 ± 2.8 | 63.2 ± 2.3 |

| | | | |
|---|---|---|---|
| Data represented as mean ± SEM. Differences were not significant. Unpaired t-test. | | | |

**Table S12. Fiber type distributions (%) in the tibialis anterior muscle of CAPN3KO mice from young group and age-matched WT mice**

| | UT | LD | HD |
|---|---|---|---|
| **STO** | 18.0 ± 2.1 | 16.4 ± 1.5 | 26.8 ± 1.2^{∗##} |
| **FTO** | 20.8 ± 0.6 | 25.6 ± 1.4 | 24.8 ± 0.8^{∗} |
| **FTG** | 61.2 ± 2.1 | 55.5 ± 3.1 | 48.4 ± 1.0** |

| | | | |
|---|---|---|---|
| Data represented as mean ± SEM. *p<0.05, **p<0.01 (compared to UT), ^{#}p<0.05, ^{##}p<0.01 (compared to LD), unpaired t-test. | | | |

**Table S13. Fiber type distributions (%) in the gastrocnemius muscle of CAPN3KO mice from young group and age-matched WT mice**

| | UT | LD | HD | WT |
|---|---|---|---|---|
| **STO** | 29.8 ± 1.3 | 26.5 ± 1.1 | 28.2 ± 1.8 | 38.5 ± 1.088^{∗∗####$} |
| **FTO** | 15.2 ± 0.5 | 24.1 ± 2.4 | 24.5 ± 2.2^{∗∗} | 11.4 ± 2.0^{$$##} |
| **FTG** | 55.1 ± 1.3 | 49.4 ± 2.8 | 47.7 ± 3.3 | 50.1 ± 1.0^{∗} |

| | | | | |
|---|---|---|---|---|
| Data represented as mean ± SEM. *p<0.05, **p<0.01 (compared to UT), ^{#}p<0.05, ^{##}p<0.01 (compared to LD), ^{$}p<0.05, ^{$$}p<0.01 (compared to HD), unpaired t-test. | | | | |

Further analysis of data for females and males suggested that the improvements in the treadmill test correlated with muscle remodeling, a switch to fatigue-resistant oxidative fibers in females along with an increase in fiber size, and a significant increase in fiber diameter which was most prominent in STO fibers in males. Changes in the fiber size and fiber type distribution of STO, FTO, and FTG (as a percent of total) observed in the treated cohorts in comparison to untreated counterparts were represented in Figure 6. Fiber type diameter (mean ± P µm; derived from the mean of measurements individually made in each mice) and fiber type distributions (derived from percent distribution in each mice) with age-matched WT data are detailed in Tables S3-14. Collectively, histological analyses of the skeletal muscle in CAPN3KO mice at 20 weeks post gene delivery reflected effects of CAPN3 replacement by remodeling of muscle, an overall switch to oxidative fibers in both young and old age groups along with fiber size increase.

### Example 6

### Long Term Treatment Efficacy of rAAVrh74.tMCK.hCAPN3 Determined by Vector Copy Number Analysis

As part of efficacy analysis of the *CAPN3* gene transfer, the biodistribution of the vector among tissues was assessed by analyzing the vector genome copies in the muscles (tibialis anterior, gastrocnemius, quadriceps, triceps) and the internal organs collected from LD and HD cohorts of the old age group of CAPN3KO mice, at 20 weeks post-treatment.

Biodistribution of AAVrh74.tMCK.CAPN3 in the CAPN3KO mice had broad tropism and efficiently targeted skeletal muscles. The highest vector genome copy number was present in the liver as expected following systemic vector delivery (Figure 7A). Vector genome distribution was variable in organs and muscles in a dose-dependent manner. HD delivery led to a 59.6% increase in skeletal muscle vector genome levels (p< 0.0001), compared with LD (Figure 7B). Interestingly, further analysis of data revealed that females had an increase of 48.7% in skeletal muscle vector genome levels across all treatments (p= 0.0001), as compared with male mice (Figure 7C) suggesting that gender may influence the transduction efficiency in *Capn3* null background.

### Example 7

### Analysis of CAPN3 Expression and Functional Correlation

Tissue expression of *hCAPN3* was carried out at mRNA and protein levels in skeletal and cardiac muscle samples collected from UT, LD, or HD-treated CAPN3KO cohorts at 20 weeks post AAVrh74.tMCK.CAPN3 vector delivery as well as in muscles from age-matched WT mice. Using qPCR, a sustained CAPN3 mRNA expression was observed in four representative muscles from hind limbs and forelimbs (tibialis anterior, gastrocnemius, quadriceps, and triceps) and heart in the CAPN3KO model. *CAPN3* mRNA levels were variable among muscles/animals in a dose-dependent manner. Skeletal muscles of both young and old age groups received HD vector showed higher CAPN3 mRNA levels (difference in young age group: 14.4%, p=0.001; in old age group: 11.0%, p<0.001) compared to LD cohorts in both age groups (Figure 8A and B). There was no significant difference in skeletal muscle CAPN3 mRNA expression related to the animal's treatment age (mean difference < 2%, p= 0.72, unpaired t-test).

Western blot analyses showed measurable amounts of full-length 94kDa CAPN3 protein in quadriceps and gastrocnemius muscles of the HD treated mice as shown from the older age group (Figure 8C). These two muscles had the highest CAPN3 expression levels. Protein levels were also variable among muscles/animal and did not directly correlate with mRNA levels. In the LD cohort, full-length 94 kDa protein was below the limit of detection in all except a gastrocnemius sample with the highest mRNA expression suggesting a threshold of mRNA level for detecting the 94 kDa CAPN3 protein band in muscles with *Capn3* null background.

The next analysis determined whether CAPN3 mRNA levels correlated with functional improvements in treadmill performance. For this analysis, a total mRNA percent value for each animal was calculated, derived from the summation of relative CAPN3 mRNA levels of the gastrocnemius, tibialis anterior, quadriceps, and triceps muscles. Since all four muscles are exerted during the treadmill run it was reasoned that the total mRNA percent value may serve as a functional performance indicator. For the old age group, the total mRNA levels from all four muscles correlated with the run to exhaustion test in the LD cohort (R2=0.5192, p=0.0082) while in the HD cohort, no correlation was seen between mRNA levels and the run to exhaustion test performance (Figure 9A). The mice with high performance in the LD cohort overlapped with the best performers of the HD, suggesting that mRNA levels exceeding a threshold might not further improve the performance. These results favor an mRNA threshold effect required to improve performance. The occurrence of a window of mRNA levels for the best treadmill performance indicated an effect of sex difference, favoring the females (Figure 9B). The correlation between treadmill performance and mRNA levels observed in the old group was not evident in the young, suggesting gender and age-related molecular changes might affect the severity of disease manifestation and treatment efficacy.

### Example 8

### PGC1α Expression Levels and Muscle Remodeling

Thus far, the data presented herein demonstrated that CAPN3 gene therapy resulted in a fiber-type switch from FTG to STO-fatigue resistant fiber-type, associated with improvements in the run to exhaustion-endurance performance favoring females. These observations prompted further studies to explore if there were sex-dependent alterations in expression levels of peroxisome proliferator-activated receptor γ coactivator 1 alpha (PGC1α) which is an important transcriptional coactivator of mitochondrial biogenesis and respiration (Handschin et al., Endocrine reviews 27: 728-735, 2002). Previous studies have shown that PGC1α drives the formation of fatigue resistant STO muscle fibers (Lin et al., Nature 418: 797-801, 2002). Moreover, PGC-1α regulates the expression and activity of the orphan nuclear receptor estrogen-related receptor alpha (ERRα) (Schreiber et al., J. Biol. Chem. 278: 9013-9018, 2003). The present have previously shown that PGC1α levels decreased in CAPN3KO mice during the regeneration process compared to WT muscle (Yalvac et al., Skeletal muscle 7: 27, 2017). In the present study, the expression levels of PGC1α in response to CAPN3 gene therapy in muscles from HD and LD treatment cohorts of old and young groups of CAPN3KO and age-matched WT mice were analyzed.

**Table S14. Primers used in the study.**

| Target | Primer | Sequence (5' to 3') | SEQ ID NO: |
|---|---|---|---|
| *AAVrh74* | Forward | CGGAGAGCAACTGCATAAG | 14 |
| | Reverse | GGCTGATGATGGCTGAATAG | 15 |
| *CAPN3* | Forward | CTACGAGGTTCCCAAAGAGATG | 16 |
| | Reverse | CCCGCATGTTGATGTAGGT | 17 |
| *PGC-1*α | Forward | AATCAGACCTGACACAACGC | 18 |
| | Reverse | GCATTCCTCAATTTCACCAA | 19 |

When gastrocnemius muscles from two different ages of WT controls were compared, an increase in relative expression levels of PGC1α was observed at 10 months of age, males showing significantly higher PGC1α expression than females compared to samples from 4-6 months old WT mice showing no gender difference (Figure 10A and B]). In contrast, PGC1α expression was significantly low in the UT-CAPN3KO mice of both sexes at 10 months of age, suggesting that *Capn3* null muscle failed to show this increase, likely an age-related compensatory change occurred in WT at this age (Figure 10A and B). Gene therapy, in both LD- and HD cohorts increased PGC1α transcripts significantly compared to UT-cohort (Figure 10A). Increases in PGC1α transcripts were seen in both genders towards normalization, (Figure 10B); however, fiber type switch to STO occurred only in females of both treatment cohorts (Figure 6, Figure 10C).

Next, studies were carried out to determine if changes in PGC1α expression varies between posterior and anterior compartment muscles in response to AAV.CAPN3 gene therapy. In the anterior tibialis, both sexes combined, no statistically significant changes in PGC1α expression between WT, LD, and UT-cohorts except in the HD was there an increase of PGC1α transcripts compared to WT (Figure 10D) were observed. Contrary to the gastrocnemius muscle of WT, PGC1α relative expression in the tibialis anterior from 10 months old WT was about 50% lower and did not show sex difference (Figure 10D and E). UT-CAPN3KO males displayed significantly higher PGC1α transcripts than WT males (Figure 10E). Interestingly, while there was no sex difference for PGC1α expression in the UT-gastrocnemius, significantly increased levels were found in the UT-anterior tibialis muscle from males compared to females. Males in LD treatment cohort showed a similar pattern with the UT cohort, having higher PGC1α expression than females. A reversal of this pattern in the HD cohort in favor of females having a 2.36-fold increase in PGC1α transcripts compared to untreated CAPN3 KO females was observed. Like gastrocnemius (extensor muscle), in tibialis anterior (flexor muscle), a switch to STO (as % increase) in both HD and LD cohorts was observed only in females (Figure 10F) even though LD males showed higher PGC1α expression than females (Figure 10E). PGC1α transcripts in these muscles from young CAPN3KO mice did not differ from each other except LD males showed significantly higher PGC1α expression than females in the same group and males of UT cohort (data not shown). Overall, these results suggest that females are responding to PGC1α increase in muscle more efficiently by converting FTG to STO fiber type. Or the other possibility is that male response to PGC1α increase in muscle is not in the same manner as female does.

### Example 9

### Toxicology Studies

GLP-like experiments were carried out to assess the safety of the test article including vector biodistribution, capsid immunogenicity, hematology, serum chemistries, and pathological review of multiple organ tissues. Biodistribution analysis showed the vector located globally, however, protein detection was limited to the target skeletal muscles; specifically, full-length 94 kDa CAPN3 protein was below the limit of detection in the tested western blot analyses of the liver, kidney, or heart tissues (from all HD and LD old group) and despite high vector genome load and mRNA levels observed in these tissues (Figure 2C). Importantly, no adverse clinical signs of toxicity were observed throughout the 20 weeks study duration at either treatment dose. No gross abnormalities were noted and no differences in animal or organ weights were observed in treatment groups, as compared to the untreated CAPN3KO controls. Microscopic evaluation of the heart, lung, liver, kidney, spleen, gonads, diaphragm, stomach, pancreas, brain, inguinal lymph nodes, and skeletal muscles removed for histopathology observations by a third party, Vet Path Services, Inc. reported no treatment-related microscopic differences among the untreated and treated groups at the 20-week study endpoint (not shown). Furthermore, hematology and serum chemistries showed no abnormal changes, suggesting the potential for rAAVrh74.tMCK.CAPN3 gene therapy is an overall safe and effective treatment for LGMD2A.

### Sequences

### Homo sapiens calpain 3 (CAPN3), transcript variant 1, mRNA

### NCBI Reference Sequence: NM_000070.2 (SEQ ID NO: 2)

>NM_000070.2:307-2772 Homo sapiens calpain 3 (CAPN3), transcript variant 1, mRNA 
ATGCCGACCGTCATTAGCGCATCTGTGGCTCCAAGGACAGCGGCTGAGCCCCGGTCCCCAGGGCCAGTTC
CTCACCCGGCCCAGAGCAAGGCCACTGAGGCTGGGGGTGGAAACCCAAGTGGCATCTATTCAGCCATCAT
CAGCCGCAATTTTCCTATTATCGGAGTGAAAGAGAAGACATTCGAGCAACTTCACAAGAAATGTCTAGAA
AAGAAAGTTCTTTATGTGGACCCTGAGTTCCCACCGGATGAGACCTCTCTCTTTTATAGCCAGAAGTTCC
CCATCCAGTTCGTCTGGAAGAGACCTCCGGAAATTTGCGAGAATCCCCGATTTATCATTGATGGAGCCAA
CAGAACTGACATCTGTCAAGGAGAGCTAGGGGACTGCTGGTTTCTCGCAGCCATTGCCTGCCTGACCCTG
AACCAGCACCTTCTTTTCCGAGTCATACCCCATGATCAAAGTTTCATCGAAAACTACGCAGGGATCTTCC
ACTTCCAGTTCTGGCGCTATGGAGAGTGGGTGGACGTGGTTATAGATGACTGCCTGCCAACGTACAACAA
TCAACTGGTTTTCACCAAGTCCAACCACCGCAATGAGTTCTGGAGTGCTCTGCTGGAGAAGGCTTATGCT
AAGCTCCATGGTTCCTACGAAGCTCTGAAAGGTGGGAACACCACAGAGGCCATGGAGGACTTCACAGGAG
GGGTGGCAGAGTTTTTTGAGATCAGGGATGCTCCTAGTGACATGTACAAGATCATGAAGAAAGCCATCGA
GAGAGGCTCCCTCATGGGCTGCTCCATTGATGATGGCACGAACATGACCTATGGAACCTCTCCTTCTGGT
 
CTGAACATGGGGGAGTTGATTGCACGGATGGTAAGGAATATGGATAACTCACTGCTCCAGGACTCAGACC
TCGACCCCAGAGGCTCAGATGAAAGACCGACCCGGACAATCATTCCGGTTCAGTATGAGACAAGAATGGC
CTGCGGGCTGGTCAGAGGTCACGCCTACTCTGTCACGGGGCTGGATGAGGTCCCGTTCAAAGGTGAGAAA
GTGAAGCTGGTGCGGCTGCGGAATCCGTGGGGCCAGGTGGAGTGGAACGGTTCTTGGAGTGATAGATGGA
AGGACTGGAGCTTTGTGGACAAAGATGAGAAGGCCCGTCTGCAGCACCAGGTCACTGAGGATGGAGAGTT
CTGGATGTCCTATGAGGATTTCATCTACCATTTCACAAAGTTGGAGATCTGCAACCTCACGGCCGATGCT
CTGCAGTCTGACAAGCTTCAGACCTGGACAGTGTCTGTGAACGAGGGCCGCTGGGTACGGGGTTGCTCTG
CCGGAGGCTGCCGCAACTTCCCAGATACTTTCTGGACCAACCCTCAGTACCGTCTGAAGCTCCTGGAGGA
GGACGATGACCCTGATGACTCGGAGGTGATTTGCAGCTTCCTGGTGGCCCTGATGCAGAAGAACCGGCGG
AAGGACCGGAAGCTAGGGGCCAGTCTCTTCACCATTGGCTTCGCCATCTACGAGGTTCCCAAAGAGATGC
ACGGGAACAAGCAGCACCTGCAGAAGGACTTCTTCCTGTACAACGCCTCCAAGGCCAGGAGCAAAACCTA
CATCAACATGCGGGAGGTGTCCCAGCGCTTCCGCCTGCCTCCCAGCGAGTACGTCATCGTGCCCTCCACC
TACGAGCCCCACCAGGAGGGGGAATTCATCCTCCGGGTCTTCTCTGAAAAGAGGAACCTCTCTGAGGAAG
TTGAAAATACCATCTCCGTGGATCGGCCAGTGAAAAAGAAAAAAACCAAGCCCATCATCTTCGTTTCGGA
CAGAGCAAACAGCAACAAGGAGCTGGGTGTGGACCAGGAGTCAGAGGAGGGCAAAGGCAAAACAAGCCCT
GATAAGCAAAAGCAGTCCCCACAGCCACAGCCTGGCAGCTCTGATCAGGAAAGTGAGGAACAGCAACAAT
TCCGGAACATTTTCAAGCAGATAGCAGGAGATGACATGGAGATCTGTGCAGATGAGCTCAAGAAGGTCCT
TAACACAGTCGTGAACAAACACAAGGACCTGAAGACACACGGGTTCACACTGGAGTCCTGCCGTAGCATG
ATTGCGCTCATGGATACAGATGGCTCTGGAAAGCTCAACCTGCAGGAGTTCCACCACCTCTGGAACAAGA
TTAAGGCCTGGCAGAAAATTTTCAAACACTATGACACAGACCAGTCCGGCACCATCAACAGCTACGAGAT
GCGAAATGCAGTCAACGACGCAGGATTCCACCTCAACAACCAGCTCTATGACATCATTACCATGCGGTAC
GCAGACAAACACATGAACATCGACTTTGACAGTTTCATCTGCTGCTTCGTTAGGCTGGAGGGCATGTTCA
GAGCTTTTCATGCATTTGACAAGGATGGAGATGGTATCATCAAGCTCAACGTTCTGGAGTGGCTGCAGCT
CACCATGTATGCCTGA
**5'ITR (SEQ ID NO: 6)** 
GCGCGCTCGCTCGCTCACTGAGGCCGCCCGGGCAAAGCCCGGGCGTCGGCGACCTTTGGTCG
CCCGGCCTCAGTGAGCGAGCGAGCGCGCAGAGAGGGAGTGGCCAACTCCATCACTAGGGGTTC
CT (nt. 1..128 or SEQ ID NO: 13 or SEQ ID NO: 20)
**tMCK Promoter (SEQ ID NO: 3)** 
CCACTACGGGTCTAGGCTGCCCATGTAAGGAGGCAAGGCCTGGGGACACCCGAGATGCCTGGT
TATAATTAACCCCAACACCTGCTGCCCCCCCCCCCCCAACACCTGCTGCCTGAGCCTGAGCGG
TTACCCCACCCCGGTGCCTGGGTCTTAGGCTCTGTACACCATGGAGGAGAAGCTCGCTCTAAA
AATAACCCTGTCCCTGGTGGATCCACTACGGGTCTAGGCTGCCCATGTAAGGAGGCAAGGCCT
GGGGACACCCGAGATGCCTGGTTATAATTAACCCCAACACCTGCTGCCCCCCCCCCCCCAACA
CCTGCTGCCTGAGCCTGAGCGGTTACCCCACCCCGGTGCCTGGGTCTTAGGCTCTGTACACCA
TGGAGGAGAAGCTCGCTCTAAAAATAACCCTGTCCCTGGTGGATCCACTACGGGTCTAGGCTG
CCCATGTAAGGAGGCAAGGCCTGGGGACACCCGAGATGCCTGGTTATAATTAACCCCAACACC
TGCTGCCCCCCCCCCCCCAACACCTGCTGCCTGAGCCTGAGCGGTTACCCCACCCCGGTGCCT
GGGTCTTAGGCTCTGTACACCATGGAGGAGAAGCTCGCTCTAAAAATAACCCTGTCCCTGGTG
GATCCTCCCTGGGGACAGCCCCTCCTGGCTAGTCACACCCTGTAGGCTCCTCTATATAACCCA
GGGGCACAGGGGCTGCCCCCGGGTCAC (nt 165-884 of SEQ ID NO: 13 or SEQ
ID NO: 20)
**Chimeric Intron (SEQ ID NO: 7)** 
Ctgtggagagaaaggcaaagtggatgtcagtaagaccaataggtgcctatcagaaacgcaaga
gtcttctctgtctcgacaagcccagtttctattggtctccttaaacctgtcttgtaaccttga
tacttac (nt. 937..1069 of SEQ ID NO: 13 or SEQ ID NO: 20)
**Kozak Sequence (SEQ ID NO: 8)**
   GCCACC (nt. 1101..1106 of SEQ ID NO: 13 or SEQ ID NO: 20)
**WT hCAPN3 (SEQ ID NO: 2)** 
ATGCCGACCGTCATTAGCGCATCTGTGGCTCCAAGGACAGCGGCTGAGCCCCGGTCCCCAGGG
CCAGTTCCTCACCCGGCCCAGAGCAAGGCCACTGAGGCTGGGGGTGGAAACCCAAGTGGCATC
TATTCAGCCATCATCAGCCGCAATTTTCCTATTATCGGAGTGAAAGAGAAGACATTCGAGCAA
CTTCACAAGAAATGTCTAGAAAAGAAAGTTCTTTATGTGGACCCTGAGTTCCCACCGGATGAG
ACCTCTCTCTTTTATAGCCAGAAGTTCCCCATCCAGTTCGTCTGGAAGAGACCTCCGGAAATT
TGCGAGAATCCCCGATTTATCATTGATGGAGCCAACAGAACTGACATCTGTCAAGGAGAGCTA
GGGGACTGCTGGTTTCTCGCAGCCATTGCCTGCCTGACCCTGAACCAGCACCTTCTTTTCCGA
GTCATACCCCATGATCAAAGTTTCATCGAAAACTACGCAGGGATCTTCCACTTCCAGTTCTGG
CGCTATGGAGAGTGGGTGGACGTGGTTATAGATGACTGCCTGCCAACGTACAACAATCAACTG
GTTTTCACCAAGTCCAACCACCGCAATGAGTTCTGGAGTGCTCTGCTGGAGAAGGCTTATGCT
AAGCTCCATGGTTCCTACGAAGCTCTGAAAGGTGGGAACACCACAGAGGCCATGGAGGACTTC
ACAGGAGGGGTGGCAGAGTTTTTTGAGATCAGGGATGCTCCTAGTGACATGTACAAGATCATG
AAGAAAGCCATCGAGAGAGGCTCCCTCATGGGCTGCTCCATTGATGATGGCACGAACATGACC
TATGGAACCTCTCCTTCTGGTCTGAACATGGGGGAGTTGATTGCACGGATGGTAAGGAATATG
GATAACTCACTGCTCCAGGACTCAGACCTCGACCCCAGAGGCTCAGATGAAAGACCGACCCGG
ACAATCATTCCGGTTCAGTATGAGACAAGAATGGCCTGCGGGCTGGTCAGAGGTCACGCCTAC
TCTGTCACGGGGCTGGATGAGGTCCCGTTCAAAGGTGAGAAAGTGAAGCTGGTGCGGCTGCGG
AATCCGTGGGGCCAGGTGGAGTGGAACGGTTCTTGGAGTGATAGATGGAAGGACTGGAGCTTT
GTGGACAAAGATGAGAAGGCCCGTCTGCAGCACCAGGTCACTGAGGATGGAGAGTTCTGGATG
TCCTATGAGGATTTCATCTACCATTTCACAAAGTTGGAGATCTGCAACCTCACGGCCGATGCT
CTGCAGTCTGACAAGCTTCAGACCTGGACAGTGTCTGTGAACGAGGGCCGCTGGTACGGGGT
TGCTCTGCCGGAGGCTGCCGCAACTTCCCAGATACTTTCTGGACCAACCCTCAGTACCGTCTG
AAGCTCCTGGAGGAGGACGATGACCCTGATGACTCGGAGGTGATTTGCAGCTTCCTGGTGGCC
CTGATGCAGAAGAACCGGCGGAAGGACCGGAAGCTAGGGGCCAGTCTCTTCACCATTGGCTTC
GCCATCTACGAGGTTCCCAAAGAGATGCACGGGAACAAGCAGCACCTGCAGAAGGACTTCTTC
CTGTACAACGCCTCCAAGGCCAGGAGCAAAACCTACATCAACATGCGGGAGGTGTCCCAGCGC
TTCCGCCTGCCTCCCAGCGAGTACGTCATCGTGCCCTCCACCTACGAGCCCCACCAGGAGGGG
GAATTCATCCTCCGGGTCTTCTCTGAAAAGAGGAACCTCTCTGAGGAAGTTGAAAATACCATC
TCCGTGGATCGGCCAGTGAAAAAGAAAAAAACCAAGCCCATCATCTTCGTTTCGGACAGAGCA
AACAGCAACAAGGAGCTGGGTGTGGACCAGGAGTCAGAGGAGGGCAAAGGCAAAACAAGCCCT
GATAAGCAAAAGCAGTCCCCACAGCCACAGCCTGGCAGCTCTGATCAGGAAAGTGAGGAACAG
CAACAATTCCGGAACATTTTCAAGCAGATAGCAGGAGATGACATGGAGATCTGTGCAGATGAG
CTCAAGAAGGTCCTTAACACAGTCGTGAACAAACACAAGGACCTGAAGACACACGGGTTCACA
CTGGAGTCCTGCCGTAGCATGATTGCGCTCATGGATACAGATGGCTCTGGAAAGCTCAACCTG
CAGGAGTTCCACCACCTCTGGAACAAGATTAAGGCCTGGCAGAAAATTTTCAAACACTATGAC
ACAGACCAGTCCGGCACCATCAACAGCTACGAGATGCGAAATGCAGTCAACGACGCAGGATTC
CACCTCAACAACCAGCTCTATGACATCATTACCATGCGGTACGCAGACAAACACATGAACATC
GACTTTGACAGTTTCATCTGCTGCTTCGTTAGGCTGGAGGGCATGTTCAGAGCTTTTCATGCA
TTTGACAAGGATGGAGATGGTATCATCAAGCTCAACGTTCTGGAGTGGCTGCAGCTCACCATG
TATGCCTGA
(nt. 1107..3572 OF SEQ ID NO: 13 or SEQ ID NO: 20)
**Poly A sequence (SEQ ID NO: 9)** 
GGGGATCCAGACATGATAAGATACATTGATGAGTTTGGACAAACCACAACTAGAATGCAGTGA
AAAAAATGCTTTATTTGTAAAATTTGTGATGCTATTGCTTTATTTGTAACCATTATAAGCTGC
 
AATAAACAAGTTAACAACAACAATTGCATTCATTTTATGTTTCAGGTTCAGGGGGAGGTGTGG
GAGGTTTTTTC (nt. 3581-3780 OF SEQ ID NO: 13 or SEQ ID NO: 20)
**3'ITR sequence (SEQ ID NO: 10)** 
AGGAACCCCTAGTGATGGAGTTGGCCACTCCCTCTCTGCGCGCTCGCTCGCTCACTGAGGCCG
GGCGACCAAAGGTCGCCCGACGCCCGGGCTTTGCCCGGGCGGCCTCAGTGAGCGAGCGAGCGC
GC (nt. 3850-3977 of SEQ ID NO: 13 or SEQ ID NO: 20)
**ORI sequence (SEQ ID NO: 11)** 
TTTCCATAGGCTCCGCCCCCCTGACGAGCATCACAAAAATCGACGCTCAAGTCAGAGGTGGCG
AAACCCGACAGGACTATAAAGATACCAGGCGTTTCCCCCTGGAAGCTCCCTCGTGCGCTCTCC
TGTTCCGACCCTGCCGCTTACCGGATACCTGTCCGCCTTTCTCCCTTCGGGAAGCGTGGCGCT
TTCTCATAGCTCACGCTGTAGGTATCTCAGTTCGGTGTAGGTCGTTCGCTCCAAGCTGGGCTG
TGTGCACGAACCCCCCGTTCAGCCCGACCGCTGCGCCTTATCCGGTAACTATCGTCTTGAGTC
CAACCCGGTAAGACACGACTTATCGCCACTGGCAGCAGCCACTGGTAACAGGATTAGCAGAGC
GAGGTATGTAGGCGGTGCTACAGAGTTCTTGAAGTGGTGGCCTAACTACGGCTACACTAGAAG
GACAGTATTTGGTATCTGCGCTCTGCTGAAGCCAGTTACCTTCGGAAAAAGAGTTGGTAGCTC
TTGATCCGGCAAACAAACCACCGCTGGTAGCGGTGGTTTTTTTGTTTGCAAGCAGCAGATTAC
GCGCAGAAAAAAAGGATCTCAA (nt. 4216..4804 of 13 or SEQ ID NO: 20)
**NeoR/KanR Sequence (SEQ ID NO: 12)** 
ATGATTGAACAAGATGGATTGCACGCAGGTTCTCCGGCCGCTTGGGTGGAGAGGCTATTCGGC
TATGACTGGGCACAACAGACAATCGGCTGCTCTGATGCCGCCGTGTTCCGGCTGTCAGCGCAG
GGGCGCCCGGTTCTTTTTGTCAAGACCGACCTGTCCGGTGCCCTGAATGAACTGCAGGACGAG
GCAGCGCGGCTATCGTGGCTGGCCACGACGGGCGTTCCTTGCGCAGCTGTGCTCGACGTTGTC
ACTGAAGCGGGAAGGGACTGGCTGCTATTGGGCGAAGTGCCGGGGCAGGATCTCCTGTCATCC
CACCTTGCTCCTGCCGAGAAAGTATCCATCATGGCTGATGCAATGCGGCGGCTGCATACGCTT
GATCCGGCTACCTGCCCATTCGACCACCAAGCGAAACATCGCATCGAGCGAGCACGTACTCGG
ATGGAAGCCGGTCTTGTCGATCAGGATGATCTGGACGAAGAGCATCAGGGGCTCGCGCCAGCC
GAACTGTTCGCCAGGCTCAAGGCGCGCATGCCCGACGGCGAGGATCTCGTCGTGACCCATGGC
GATGCCTGCTTGCCGAATATCATGGTGGAAAATGGCCGCTTTTCTGGATTCATCGACTGTGGC
CGGCTGGGTGTGGCGGACCGCTATCAGGACATAGCGTTGGCTACCCGTGATATTGCTGAAGAG
CTTGGCGGCGAATGGGCTGACCGCTTCCTCGTGCTTTACGGTATCGCCGCTCCCGATTCGCAG
CGCATCGCCTTCTATCGCCTTCTTGACGAGTTCTTCTGA (nt. 5106-5900 of SEQ
ID NO: 13 or SEQ ID NO: 20)
**pAAV.tMCK.CAPN3 plasmid full sequence (SEQ ID NO: 13)**

gcgcgctcgctcgctcactgaggccgcccgggcaaagcccgggcgtcgggcgacctttgg
tcgcccggcctcagtgagcgagcgagcgcgcagagagggagtggccaactccatcactag
gggttccttaatacgactcactatagggccgcaccggtaagcttccactacgggtctagg
ctgcccatgtaaggaggcaaggcctggggacacccgagatgcctggttataattaacccc
aacacctgctgcccccccccccccaacacctgctgcctgagcctgagcggttaccccacc
ccggtgcctgggtcttaggctctgtacaccatggaggagaagctcgctctaaaaataacc
ctgtccctggtggatccactacgggtctaggctgcccatgtaaggaggcaaggcctgggg
acacccgagatgcctggttataattaaccccaacacctgctgcccccccccccccaacac
ctgctgcctgagcctgagcggttaccccaccccggtgcctgggtcttaggctctgtacac
catggaggagaagctcgctctaaaaataaccctgtccctggtggatccactacgggtcta
ggctgcccatgtaaggaggcaaggcctggggacacccgagatgcctggttataattaacc
ccaacacctgctgcccccccccccccaacacctgctgcctgagcctgagcggttacccca
ccccggtgcctgggtcttaggctctgtacaccatggaggagaagctcgctctaaaaataa

ccctgtccctggtggatcctccctggggacagcccctcctggctagtcacaccctgtagg
ctcctctatataacccaggggcacaggggctgcccccgggtcacggtggatatccctgca
ggtaccacgcgctgtaattgaactgggagtggacacctgtggagagaaaggcaaagtgga
tgtcagtaagaccaataggtgcctatcagaaacgcaagagtcttctctgtctcgacaagc
ccagtttctattggtctccttaaacctgtcttgtaaccttgatacttacggagagcaact
gcataagggctagcctcgaggccaccatgccgaccgtcattagcgcatctgtggctccaa
ggacagcggctgagccccggtccccagggccagttcctcacccggcccagagcaaggcca
ctgaggctgggggtggaaacccaagtggcatctattcagccatcatcagccgcaattttc
ctattatcggagtgaaagagaagacattcgagcaacttcacaagaaatgtctagaaaaga
aagttctttatgtggaccctgagttcccaccggatgagacctctctcttttatagccaga
agttccccatccagttcgtctggaagagacctccggaaattgcgagaatccccgattta
tcattgatggagccaacagaactgacatctgtcaaggagagctaggggactgctggtttc
tcgcagccattgcctgcctgaccctgaaccagcaccttcttttccgagtcataccccatg
atcaaagtttcatcgaaaactacgcagggatcttccacttccagttctggcgctatggag
agtgggtggacgtggttatagatgactgcctgccaacgtacaacaatcaactggttttca
ccaagtccaaccaccgcaatgagttctggagtgctctgctggagaaggcttatgctaagc
tccatggttcctacgaagctctgaaaggtgggaacaccacagaggccatggaggacttca
caggaggggtggcagagtttttgagatcagggatgctcctagtgacatgtacaagatca
tgaagaaagccatcgagagaggctccctcatgggctgctccattgatgatggcacgaaca
tgacctatggaacctctccttctggtctgaacatgggggagttgattgcacggatggtaa
ggaatatggataactcactgctccaggactcagacctcgacccccagaggctcagatgaaa
gaccgacccggacaatcattccggttcagtatgagacaagaatggcctgcgggctggtca
gaggtcacgcctactctgtcacggggctggatgaggtcccgttcaaaggtgagaaagtga
agctggtgcggctgcggaatccgtggggccaggtggagtggaacggttcttggagtgata
gatggaaggactggagctttgtggacaaagatgagaaggcccgtctgcagcaccaggtca
ctgaggatggagagttctggatgtcctatgaggatttcatctaccatttcacaaagttgg
agatctgcaacctcacggccgatgctctgcagtctgacaagcttcagacctggacagtgt
ctgtgaacgagggccgctgggtacggggttgctctgccggaggctgccgcaacttcccag
atactttctggaccaaccctcagtaccgtctgaagctcctggaggaggacgatgaccctg
atgactcggaggtgatttgcagcttcctggtggccctgatgcagaagaaccggcggaagg
accggaagctaggggccagtctcttcaccattggcttcgccatctacgaggttcccaaag
agatgcacgggaacaagcagcacctgcagaaggacttcttcctgtacaacgcctccaagg
ccaggagcaaaacctacatcaacatgcgggaggtgtcccagcgcttccgcctgcctccca
gcgagtacgtcatcgtgccctccacctacgagccccaccaggaggggaattcatcctcc
gggtcttctctgaaaagaggaacctctctgaggaagttgaaaataccatctccgtggatc
ggccagtgaaaaagaaaaaaaccaagcccatcatcttcgtttcggacagagcaaacagca
acaaggagctgggtgtggaccaggagtcagaggagggcaaaggcaaaacaagccctgata
agcaaaagcagtccccacagccacagcctggcagctctgatcaggaaagtgaggaacagc
aacaattccggaacattttcaagcagatagcaggagatgacatggagatctgtgcagatg
agctcaagaaggtccttaacacagtcgtgaacaaacacaaggacctgaagacacacgggt
tcacactggagtcctgccgtagcatgattgcgctcatggatacagatggctctggaaagc
tcaacctgcaggagttccaccacctctggaacaagattaaggcctggcagaaaattttca
aacactatgacacagaccagtccggcaccatcaacagctacgagatgcgaaatgcagtca
acgacgcaggattccacctcaacaaccagctctatgacatcattaccatgcggtacgcag
acaaacacatgaacatcgactttgacagtttcatctgctgcttcgttaggctggagggca
tgttcagagcttttcatgcatttgacaaggatggagatggtatcatcaagctcaacgttc
tggagtggctgcagctcaccatgtatgcctgagcggccgcggggatccagacatgataag
atacattgatgagtttggacaaaccacaactagaatgcagtgaaaaaaatgctttatttg
taaaatttgtgatgctattgctttatttgtaaccattataagctgcaataaacaagttaa
caacaacaattgcattcattttatgtttcaggttcagggggaggtgtgggaggttttttc
ggatcctctagagtcgaccagagcatggctacgtagataagtagcatggcgggttaatca
ttaactacaaggaacccctagtgatggagttggccactccctctctgcgcgctcgctcgc
tcactgaggccgggcgaccaaaggtcgcccgacgcccgggctttgcccgggcggcctcag
tgagcgagcgagcgcgcagctgcattaatgaatcggccaacgcgcggggagaggcggttt
gcgtattgggcgctcttccgcttcctcgctcactgactcgctgcgctcggtcgttcggct
gcggcgagcggtatcagctcactcaaaggcggtaatacggttatccacagaatcagggga
taacgcaggaaagaacatgtgagcaaaaggccagcaaaaggccaggaaccgtaaaaaggc
cgcgttgctggcgtttttccataggctccgcccccctgacgagcatcacaaaaatcgacg
ctcaagtcagaggtggcgaaacccgacaggactataaagataccaggcgtttccccctgg
aagctccctcgtgcgctctcctgttccgaccctgccgcttaccggatacctgtccgcctt
tctcccttcgggaagcgtggcgctttctcatagctcacgctgtaggtatctcagttcggt

gtaggtcgttcgctccaagctgggctgtgtgcacgaaccccccgttcagcccgaccgctg
cgccttatccggtaactatcgtcttgagtccaacccggtaagacacgacttatcgccact
ggcagcagccactggtaacaggattagcagagcgaggtatgtaggcggtgctacagagtt
cttgaagtggtggcctaactacggctacactagaaggacagtatttggtatctgcgctct
gctgaagccagttaccttcggaaaaagagttggtagctcttgatccggcaaacaaaccac
cgctggtagcggtggtttttttgtttgcaagcagcagattacgcgcagaaaaaaaggatc
tcaagaagatcctttgatcttttctacggggtctgacgctcagtggaacgaaaactcacg
ttaagggattttggtcatgagattatcaaaaaggatcttcacctagatccttttaaatta
aaaatgaagttttaaatcaatctaaagtatatatgagtaaaaatattccggaattgccag
ctggggcgccctctggtaaggttgggaagccctgcaaagtaaactggatggctttcttgc
cgccaaggatctgatggcgcaggggatcaagatctgatcaagagacaggatgaggatcgt
ttcgcatgattgaacaagatggattgcacgcaggttctccggccgcttgggtggagaggc
tattcggctatgactgggcacaacagacaatcggctgctctgatgccgccgtgttccggc
tgtcagcgcaggggcgcccggttctttttgtcaagaccgacctgtccggtgccctgaatg
aactgcaggacgaggcagcgcggctatcgtggctggccacgacgggcgttccttgcgcag
ctgtgctcgacgttgtcactgaagcgggaagggactggctgctattgggcgaagtgccgg
ggcaggatctcctgtcatcccaccttgctcctgccgagaaagtatccatcatggctgatg
caatgcggcggctgcatacgcttgatccggctacctgcccattcgaccaccaagcgaaac
atcgcatcgagcgagcacgtactcggatggaagccggtcttgtcgatcaggatgatctgg
acgaagagcatcaggggctcgcgccagccgaactgttcgccaggctcaaggcgcgcatgc
ccgacggcgaggatctcgtcgtgacccatggcgatgcctgcttgccgaatatcatggtgg
aaaatggccgcttttctggattcatcgactgtggccggctgggtgtggcggaccgctatc
aggacatagcgttggctacccgtgatattgctgaagagcttggcggcgaatgggctgacc
gcttcctcgtgctttacggtatcgccgctcccgattcgcagcgcatcgccttctatcgcc
ttcttgacgagttcttctgaaccggtaatattattgaagcatttatcagggttattgtct
catgagcggatacatatttgaatgtatttagaaaaataaacaaataggggttccgcgcac
atttccccgaaaagtgccacctgacgtctaagaaaccattattatcatgacattaaccta
taaaaataggcgtatcacgaggccctttcgtctcgcgcgtttcggtgatgacggtgaaaa
cctctgacacatgcagctcccggagacggtcacagcttgtctgtaagcggatgccgggag
cagacaagcccgtcagggcgcgtcagcgggtgttggcgggtgtcggggctggcttaacta
tgcggcatcagagcagattgtactgagagtgcaccatatgcggtgtgaaataccgcacag
atgcgtaaggagaaaataccgcatcaggaacttccaacatccaataaatcatacaggcaa
ggcaaagaattagcaaaattaagcaataaagcctcagagcataaagctaaatcggttgta
ccaaaaacattatgaccctgtaatacttttgcgggagaagcctttatttcaacgcaagga
taaaaatttttagaaccctcatatattttaaatgcaatgcctgagtaatgtgtaggtaaa
gattcaaacgggtgagaaaggccggagacagtcaaatcaccatcaatatgatattcaacc
gttctagctgataaattcatgccggagagggtagctatttttgagaggtctctacaaagg
ctatcaggtcattgcctgagagtctggagcaaacaagagaatcgatgaacggtaatcgta
aaactagcatgtcaatcatatgtaccccggttgataatcagaaaagccccaaaaacagga
agattgtataagcaaatatttaaattgtaaacgttaatattttgttaaaattcgcgttaa
atttttgttaaatcagctcattttttaaccaataggccgaaatcggcaaaatcccttata
aatcaaaagaatagaccgagatagggttgagtgttgttccagtttggaacaagagtccac
tattaaagaacgtggactccaacgtcaaagggcgaaaaaccgtctatcagggcgatggcc
cactacgtgaaccatcaccctaatcaagttttttggggtcgaggtgccgtaaatcactaa
atcggaaccctaaagggagcccccgatttagagcttgacggggaaagccggcgaacgtgg
cgagaaaggaagggaagaaagcgaaaggagcgggcgctagggcgctggcaagtgtagcgg
tcacgctgcgcgtaaccaccacacccgccgcgcttaatgcgccgctacagggcgcgtact
atggttgctttgacgagcacgtataacgtgctttcctcgttagaatcagagcggagcta
aacaggaggccgattaaagggattttagacaggaacggtacgccagaatcctgagaagtg
tttttataatcagtgaggccaccgagtaaaagagtctgtccatcacgcaaattaaccgtt
gtcgcaatacttctttgattagtaataacatcacttgcctgagtagaagaactcaaacta
tcggccttgctggtaatatccagaacaatattaccgccagccattgcaacggaatcgcca
ttcgccattcaggctgcgcaactgttgggaagggcgatcggtgcgggcctcttcgctat

While the present disclosure provides specific embodiments, it is understood that variations and modifications will occur to those skilled in the art. Accordingly, only such limitations as appear in the claims should be placed on the invention.

All documents referred to in this application are hereby incorporated by reference in their entirety.

Various features and embodiments of the present invention will now be described with reference to the following numbered paragraphs:
1. A method of increasing expression of peroxisome proliferator activated receptor γ coactivator 1 alpha (PGC1α) in a subject comprising administering to the subject a recombinant adeno-associated virus (rAAV), wherein the rAAV comprises a polynucleotide encoding a protein that increases muscular force and/or increases muscular mass, and wherein administration of the rAAV results in an increase in the level of PGC1α compared to a control level of PGC1α.
2. The method of paragraph 1 further comprising the step of measuring the level of PGC1α in the subject.
3. The method of paragraph 1 or 2, wherein the method results in a switch from fast-twitch glycolytic (FTG) fibers to a slow-twitch oxidative (STO) fibers in the subject.
4. A method of treating muscular dystrophy in a subject, comprising
   administering to the subject an rAAV comprising a polynucleotide encoding a protein that increases muscular force and/or increases muscular mass
   measuring the expression level of peroxisome proliferator activated receptor γ coactivator 1 alpha (PGC1α) in a sample obtained from the subject after administration of a rAAV, wherein a change in PGC1α level compared to a control level of PGC1α is indicative of a therapeutic effect of the rAAV and/or the muscle function of the subject.
5. The method of paragraph 4, wherein an increase in PGC1α level is indicative of improved muscle function.
6. The method of paragraph 4 or 5 further comprising obtaining the sample before and/or after administering the rAAV to the subject.
7. A recombinant adeno-associated virus (rAAV) for treating muscular dystrophy in a subject, wherein the rAAV comprises a gene encoding a protein that increases muscular force and/or increases muscular mass, and wherein administration of the rAAV results in a change in the level of peroxisome proliferator activated receptor γ coactivator 1 alpha (PGC1α) as compared to a control level of PGC1α, wherein in a change in the PGC1α level is indicative of a therapeutic effect of the rAAV and/or the muscle function of the subject.
8. Use of a rAAV for the preparation of a medicament for treating muscular dystrophy in a subject, wherein the rAAV comprises a gene encoding a protein that increases muscular force and/or increases muscular mass, and wherein administration of the rAAV results in a change in the level of peroxisome proliferator activated receptor γ coactivator 1 alpha (PGC1α) as compared to a control level of PGC1α, wherein in a change in the PGC1α level is indicative of a therapeutic effect of the rAAV and/or the muscle function of the subject.
9. The composition or use of paragraph 7 or 8, wherein an increase in PGC1α level is indicative of improved muscle function.
10. The composition or use of any one of paragraphs 7-9, wherein the change in PGC1α is determined in a sample obtained before and/or after administering the rAAV to the subject.
11. The method, rAAV or use of any one of paragraphs 1-10, wherein the control level of PGC1α is the level of PGC1α in the subject prior to or without administration of the rAAV, or a known standard level of PGC1α.
12. The method, rAAV or use of any one of paragraphs 1-11 wherein the protein that increases muscular force and/or increases muscular mass is microdystrophin, dystrophin, calpain 3 (CAPN3), dysferlin (DYSF), SGCGA (α-sarcoglycan ), SGCB (β-sarcoglycan ),γ-sarcoglycan, delta-sarcoglycan, telethonin ancotamin 5 (ANO5), GALGT2, myotilin, lamin A/C, caveolin, desmin, telethonin, FKRP, titin, POMT1, GALGT2 and fukutin.
13. The method, rAAV or use of any one of paragraphs 1-12, wherein the muscular dystrophy is Duchenne Muscular Dystrophy (DMD); Becker Muscular Dystrophy; Congenital Muscular Dystrophy (MDC) 1A, 1B, 1C and 1D; Limb Girdle Muscular Dystrophy (LGMD) 1A, 1B, 1C, 1D, 1E, 1F, 1G, 1H, 2A, 2B, 2C, 2D, 2E, 2F, 2G 2H, 2I, 2J, 2K, 2L, 2M, 2N, 2O and 2Q; Ullrich Congenital Muscular Dystrophy; Fukuyama Congenital Muscular Dystrophy; Myotonic Dystrophy; Emery Dreifuss Muscular Dystrophy; Distal Muscular Dystrophy; Centronuclear; and Faciosacpulohumeral Muscular Dystrophy.
14. A method of increasing expression of peroxisome proliferator activated receptor γ coactivator 1 alpha (PGC1α) in a subject suffering from limb girdle muscular dystrophy 2A comprising administering to the subject a recombinant adeno-associated virus (rAAV), wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein administration of the rAAV results in an increase in the level of PGC1α compared to a control level of PGC1α.
15. The method of paragraph 14 further comprising the step of measuring the level of PGC1α in the subject.
16. The method of paragraph 14 or 15, wherein the method results in a switch from fast-twitch glycolytic (FTG) fibers to a slow-twitch oxidative (STO) fibers in the subject.
17. A method of treating limb girdle muscular dystrophy 2A in a subject comprising administering to the subject a recombinant adeno-associated virus (rAAV), wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein the administration of the rAAV results in an improved performance or improved phenotype in the subject compared to performance or phenotype prior to administration of the rAAV,
   wherein the improved performance is one or more of
      an increase in muscle contractibility, or
      an improved performance in the run to exhaustion test, and
   wherein the improved phenotype is one or more of
      an increase in muscle fiber diameter,
      an increase in number of oxidative muscle fibers,
      a switch from fast-twitch glycolytic (FTG) fibers to a slow-twitch oxidative (STO) fibers, or
      an increase in the percentage of oxidative muscle fibers.
18. A method of treating limb girdle muscular dystrophy 2A in a subject comprising administering to the subject a recombinant adeno-associated virus (rAAV), wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein the administration of the rAAV results in an increase in Capn3 mRNA expression of at least 10% compared with the level of Capn3 mRNA expression prior to administration of the rAAV and wherein an increase of at least 10% is indicative of an increase in performance or phenotype in the subject.
19. The method of paragraph 18 wherein the performance is
   an increase in muscle contractibility, or
   an improved performance in the run to exhaustion test.
20. The method of paragraph 18 the phenotype is one or more of
   an increase in number of oxidative muscle fibers,
   a switch from fast-twitch glycolytic (FTG) fibers to a slow-twitch oxidative (STO) fibers,
   an increase in the percentage of oxidative muscle fibers, or
   an increase in muscle fiber diameter.
21. The method of any one of paragraphs 17-19, wherein the improved performance is at least about 20% increase in maximum twitch response or is at least about 10% increase in maximum tetanic response.
22. The method of any one of paragraphs 17-19, wherein the improved performance is at least about 70% improvement in the treadmill run to exhaustion test or at least about 100% increase in the distance to run to exhaustion test.
23. The method of paragraph 20 wherein the muscle fiber comprises one or more of slow twitch oxidative (STO) muscle fiber, fast twitch oxidative (FTO) muscle fiber, and fast twitch glycolytic (FTG) fiber.
24. The method of any one of paragraphs 14-23, wherein the rAAV are administered by intramuscular injection or intravenous injection.
25. A recombinant adeno-associated virus (rAAV) for increasing expression of peroxisome proliferator activated receptor γ coactivator 1 alpha (PGC1α) in a subject suffering from limb girdle muscular dystrophy 2A, wherein the rAAV comprises a polynucleotide which comprises a first AAV inverted terminal repeat (ITR), a promoter, a nucleotide sequence encoding a protein with calpain 3 (CAPN3) activity and a second AAV ITR.
26. A recombinant adeno-associated virus (rAAV) for treating limb girdle muscular dystrophy 2A in a subject, wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein administration of the rAAV results in an improved performance or improved phenotype in the subject compared to performance or phenotype prior to administration of the rAAV,
   wherein the improved performance is one or more of
      an increase in muscle contractibility, or
      an improved performance in the run to exhaustion test, and
   wherein the improved phenotype is one or more of
      an increase in muscle fiber diameter,
      an increase in number of oxidative muscle fibers,
      a switch from fast-twitch glycolytic (FTG) fibers to a slow-twitch oxidative (STO) fibers, or
      an increase in the percentage of oxidative muscle fibers.
27. A recombinant adeno-associated virus (rAAV) for treating limb girdle muscular dystrophy 2A in a subject, wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein administration of the rAAV results in an increase in Capn3 mRNA expression of at least 10% compared with the level of Capn3 mRNA expression prior to administration of the rAAV and wherein an increase of at least 10% is indicative of an increase in performance or phenotype in the subject.
28. The rAAV of paragraph 27 wherein the performance is
   an increase in muscle contractibility, or
   an improved performance in the run to exhaustion test.
29. The rAAV of paragraph 27 the phenotype is one or more of
   an increase in number of oxidative muscle fibers,
   an increase in the percentage of oxidative muscle fibers, or
   an increase in muscle fiber diameter.
30. The rAAV of any one of paragraphs 26-28, wherein the improved performance is at least about 20% increase in maximum twitch response or is at least about 10% increase in maximum tetanic response.
31. The rAAV of any one of paragraphs 26-28, wherein the improved performance is at least about 70% improvement in the treadmill run to exhaustion test or at least about100% increase in the distance to run to exhaustion test.
32. The rAAV of any one of paragraphs 26-29, wherein the muscle fiber comprises one or more of slow twitch oxidative (STO) muscle fiber, fast twitch oxidative (FTO) muscle fiber, and fast twitch glycolytic (FTG) fiber.
33. The rAAV of any one of paragraphs 25-32, wherein the rAAV are formulated for administration by intramuscular injection or intravenous injection.
34. Use of a recombinant adeno-associated virus (rAAV) for the preparation of a medicament for increasing expression of peroxisome proliferator activated receptor γ coactivator 1 alpha (PGC1α) in a subject suffering from limb girdle muscular dystrophy 2A, wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein administration of the rAAV results in an increase in peroxisome proliferator activated receptor γ coactivator 1 alpha (PGC1α) level compared to a control level of PGC1α.
35. Use of a recombinant adeno-associated virus (rAAV) for the preparation of a medicament for increasing PGC1α level in a subject suffering from limb girdle muscular dystrophy 2A, wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein administration of the rAAV results in an improved performance or improved phenotype in the subject compared to performance or phenotype prior to administration of the rAAV,
   wherein the improved performance is one or more of
      an increase in muscle contractibility, or
      an improved performance n the run to exhaustion test, and
   wherein the improved phenotype is one or more of
      an increase in muscle fiber diameter,
      an increase in number of oxidative muscle fibers,
      a switch from fast-twitch glycolytic (FTG) fibers to a slow-twitch oxidative (STO) fibers, or
      an increase in the percentage of oxidative muscle fibers.
36. Use of a recombinant adeno-associated virus (rAAV) for the preparation of a medicament for treating limb girdle muscular dystrophy 2A, wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein administration of the rAAV results in an increase in Capn3 mRNA expression of at least 10% compared with the level of Capn3 mRNA expression prior to administration of the rAAV and wherein an increase of at least 10% is indicative of an increase in performance or phenotype in the subject.
37. The use of paragraph 36 wherein the performance is
   an increase in muscle contractibility, or
   an improved performance in the run to exhaustion test.
38. The use of paragraph 36 wherein the phenotype is one or more of
   an increase in number of oxidative muscle fibers,
   an increase in the percentage of oxidative muscle fibers, or
   an increase in muscle fiber diameter.
39. The use of any one of paragraphs 34-37, wherein the improved performance is at least about 20% increase in maximum twitch response or is at least about 10% increase in maximum tetanic response.
40. The use of any one of paragraphs 34-37, wherein the improved performance is at least about 70% improvement in the treadmill run to exhaustion test or at least about100% increase in the distance to run to exhaustion test.
41. The use of paragraph 35-38, wherein the muscle fiber comprises one or more of slow twitch oxidative (STO) muscle fiber, fast twitch oxidative (FTO) muscle fiber, and fast twitch glycolytic (FTG) fiber.
42. The use of any one of paragraphs 34-41, wherein the rAAV is formulated for by intramuscular injection or intravenous injection.
43. The method, rAAV or use of any one of paragraphs 14-42, wherein the nucleotide sequence encoding the protein with CAPN3 activity is at least 90%, 91%, 92%, 93%, 94%, 95%, 96%, 97%, 98%, or 99% identical to SEQ ID NO: 2.
44. The method, rAAV or use of any one of paragraphs 14-42, wherein the nucleotide sequence encoding the protein with CAPN3 activity is at least 95% identical to SEQ ID NO: 2.
45. The method, rAAV or use of any one of paragraphs 14-42, wherein the nucleotide sequence encoding the protein with CAPN3 activity comprises the sequence of SEQ ID NO: 2.
46. The method, rAAV or use of any one of paragraphs 14-42, wherein the protein with CAPN3 activity comprises an amino acid sequence at least 90%, 91%, 92%, 93%, 94%, 95%, 96%, 97%, 98%, or 99% identical to SEQ ID NO: 5.
47. The method, rAAV or use of any one of paragraphs 14-42, wherein the protein with CAPN3 activity comprises the amino acid sequence of SEQ ID NO: 5.
48. The method, rAAV or use of any one of paragraphs 14-42, wherein the polynucleotide comprises a sequence which is at least 90%, 91%, 92%, 93%, 94%, 95%, 96%, 97%, 98%, or 99% identical to SEQ ID NO: 1.
49. The method, rAAV or use of any one of paragraphs 14-42, wherein the polynucleotide comprises a sequence at least 95% identical to SEQ ID NO: 1.
50. The method, rAAV or use of any one of paragraphs 14-42, wherein the polynucleotide comprises the sequence of SEQ ID NO: 1.
51. The method of any one of paragraphs 14-50, wherein the promoter is a muscle-specific promoter.
52. The method, rAAV or use of paragraph 51, wherein the muscle-specific promoter comprises one or more of a human skeletal actin gene element, a cardiac actin gene element, a desmin promoter, a skeletal alpha-actin (ASKA) promoter, a troponin I (TNNI2) promoter, a myocyte-specific enhancer binding factor mef binding element, a muscle creatine kinase (MCK) promoter, a truncated MCK (tMCK) promoter, a myosin heavy chain (MHC) promoter, a hybrid a-myosin heavy chain enhancer-/MCK enhancer-promoter (MHCK7) promoter, a C5-12 promoter, a murine creatine kinase enhancer element, a skeletal fast-twitch troponin c gene element, a slow-twitch cardiac troponin c gene element, a slow-twitch troponin i gene element, hypoxia- inducible nuclear factor (HIF)-response element (HRE), a steroid-inducible element, and a glucocorticoid response element (gre).
53. The method, rAAV or use of paragraph 51, wherein the muscle-specific promoter is an MCK promoter, a tMCK promoter, or an MHCK7 promoter.
54. The method, rAAV or use of paragraph 51, wherein the muscle-specific promoter is a truncated MCK promoter comprising the nucleotide sequence of SEQ ID NO: 3.
55. The method, rAAV or use of any one of paragraphs 14-54, wherein the first and second AAV inverted terminal repeats are AAV2 inverted terminal repeats.
56. The method, rAAV or use of any one of paragraphs 14-55, wherein the rAAV comprises one or more of AAV-1, AAV-2, AAV-3, AAV-4, AAV-5, AAV-6, AAV-7, AAV-8, AAV-9, AAV-10, AAV-11, AAV-12, AAV-13, AAV rh.74 and AAV rh.10 capsid proteins.
57. The method, rAAV or use of any one of paragraphs 14-55, wherein the rAAV comprises an rh.74 capsid protein or an AAV9 capsid protein.
58. The method, rAAV or use of any one of paragraphs 14-57, wherein the subject is a pediatric subject, an adolescent subject or a young adult subject.
59. The method, rAAV or use of any one of paragraphs 14-57, wherein the subject is a middle aged adult or elderly subject.
60. The method, rAAV or use of any one of paragraphs 14-57, wherein the subject is 4 to 15 years of age.
61. The method, rAAV or use of any one of paragraphs 14-57, wherein the subject is 15 to 55 years of age.
62. The method, rAAV or use of any one of paragraphs 14-57, wherein the subject over 55 years of age.
63. A polynucleotide comprising the nucleotide sequence of that is at least 90% 95% or 99% identical to the nucleotide sequence of SEQ ID NO: 13.
64. A polynucleotide of paragraph 63 wherein the polynucleotide comprises the nucleotide sequence of SEQ ID NO: 13.
65. A method of generating a recombinant AAV rAAV.tMCK.CAPN3, comprising transferring a plasmid to a cell, wherein the plasmid comprises a nucleotide sequence that is at least 90%, 95%, or 99% identical to SEQ ID NO: 13.
66. The method of paragraph 65, wherein the plasmid comprises a nucleotide sequence of SEQ ID NO: 13.
67. The method of paragraph 65 or 66, further comprising transferring a packaging plasmid and/or a helper virus to the cell.
68. The method of any one of paragraphs 65-67, wherein the cell comprises a stably integrated AAV cap gene.
69. The method of any one of paragraphs 65-68, wherein the cell comprises a stably integrated AAV rep gene.
70. A cell, comprising a plasmid that comprises a nucleotide sequence that is at least 90%, 95%, or 99% identical to SEQ ID NO: 13.
71. The cell of paragraph 70, wherein the plasmid that comprises a nucleotide sequence of SEQ ID NO: 13.
72. The cell of paragraph 70 or 71, comprising a nucleotide sequence of SEQ ID NO: 13.
73. The cell of any one of paragraphs 70-72, wherein the cell is a bacterial cell, an insect cell, a mosquito cell, or a mammalian cell.
74. The cell of any one of paragraphs 70-73, wherein the cell is *E. coli.*
75. The cell of any one of paragraphs 70-73, wherein the cell is an HEK 293 cell, a HeLa cell or a *Spodoptera frugiperda* (Sf9) insect cell.

## Claims

1. A recombinant adeno-associated virus (rAAV) for use in treating muscular dystrophy in a subject, wherein the rAAV comprises a gene encoding a protein that increases muscular force and/or increases muscular mass, and wherein administration of the rAAV results in a change in the level of peroxisome proliferator activated receptor γ coactivator 1 alpha (PGC1α) as compared to a control level of PGC1α, wherein in a change in the PGC1α level is indicative of a therapeutic effect of the rAAV and/or the muscle function of the subject.

2. The rAAV for use according to claim 1, wherein an increase in PGC1α level is indicative of improved muscle function.

3. The rAAV for use according to claim 1 or 2, wherein the change in PGC1α is determined in a sample obtained before and/or after administering the rAAV to the subject.

4. The rAAV for use according to any one of claims 1-3, wherein the control level of PGC1α is the level of PGC1α in the subject prior to or without administration of the rAAV, or a known standard level of PGC1α.

5. The rAAV for use according to any one of claims 1-4, wherein the protein that increases muscular force and/or increases muscular mass is microdystrophin, dystrophin, calpain 3 (CAPN3), dysferlin (DYSF), SGCGA (α-sarcoglycan ), SGCB (β-sarcoglycan ),γ-sarcoglycan, delta-sarcoglycan, telethonin ancotamin 5 (ANO5), GALGT2, myotilin, lamin A/C, caveolin, desmin, telethonin, FKRP, titin, POMT1, GALGT2 and fukutin.

6. The rAAV for use according to any one of claims 1-5, wherein the muscular dystrophy is Duchenne Muscular Dystrophy (DMD); Becker Muscular Dystrophy; Congenital Muscular Dystrophy (MDC) 1A, 1B, 1C and 1D; Limb Girdle Muscular Dystrophy (LGMD) 1A, 1B, 1C, 1D, 1E, 1F, 1G, 1H, 2A, 2B, 2C, 2D, 2E, 2F, 2G 2H, 2I, 2J, 2K, 2L, 2M, 2N, 2O and 2Q; Ullrich Congenital Muscular Dystrophy; Fukuyama Congenital Muscular Dystrophy; Myotonic Dystrophy; Emery Dreifuss Muscular Dystrophy; Distal Muscular Dystrophy; Centronuclear; and Faciosacpulohumeral Muscular Dystrophy.

7. A recombinant adeno-associated virus (rAAV) for use in increasing expression of peroxisome proliferator activated receptor γ coactivator 1 alpha (PGC1α) in a subject suffering from limb girdle muscular dystrophy 2A, wherein the rAAV comprises a polynucleotide which comprises a first AAV inverted terminal repeat (ITR), a promoter, a nucleotide sequence encoding a protein with calpain 3 (CAPN3) activity and a second AAV ITR.

8. A recombinant adeno-associated virus (rAAV) for use in treating limb girdle muscular dystrophy 2A in a subject, wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein administration of the rAAV results in an improved performance or improved phenotype in the subject compared to performance or phenotype prior to administration of the rAAV,
wherein the improved performance is one or more of
an increase in muscle contractibility, or
an improved performance in the run to exhaustion test, and
wherein the improved phenotype is one or more of
an increase in muscle fiber diameter,
an increase in number of oxidative muscle fibers,
a switch from fast-twitch glycolytic (FTG) fibers to a slow-twitch oxidative (STO) fibers, or
an increase in the percentage of oxidative muscle fibers.

9. A recombinant adeno-associated virus (rAAV) for use in treating limb girdle muscular dystrophy 2A in a subject, wherein the rAAV comprises a polynucleotide which comprises a first AAV inverted terminal repeat (ITR), a promoter, a nucleotide sequence encoding a protein with calpain 3 (CAPN3) activity and a second AAV ITR, and wherein administration of the rAAV results in an increase in Capn3 mRNA expression of at least 10% compared with the level of Capn3 mRNA expression prior to administration of the rAAV and wherein an increase of at least 10% is indicative of an increase in performance or phenotype in the subject.

10. The rAAV for use according to claim 9, wherein the performance is
an increase in muscle contractibility, or
an improved performance in the run to exhaustion test.

11. The rAAV for use according to claim 9, wherein the phenotype is one or more of
an increase in number of oxidative muscle fibers,
an increase in the percentage of oxidative muscle fibers, or
an increase in muscle fiber diameter.

12. The rAAV for use according to any one of claims 8-11, wherein the improved performance is
at least about 20% increase in maximum twitch response,
at least about 10% increase in maximum tetanic response,
at least about 70% improvement in the treadmill run to exhaustion test or
at least about 100% increase in the distance to run to exhaustion test.

13. The rAAV for use according to any one of claims 8-12, wherein the muscle fiber comprises one or more of slow twitch oxidative (STO) muscle fiber, fast twitch oxidative (FTO) muscle fiber, and fast twitch glycolytic (FTG) fiber.

14. The rAAV for use according to any one of claims 1-13, wherein the rAAV are administered by intramuscular injection or intravenous injection.

15. The rAAV for use according to any one of claims 8-14, wherein the nucleotide sequence encoding the protein with CAPN3 activity
a) is at least 90%, 91%, 92%, 93%, 94%, 95%, 96%, 97%, 98%, or 99% identical to SEQ ID NO: 2,
b) is at least 95% identical to SEQ ID NO: 2; or
c) comprises the sequence of SEQ ID NO: 2.

16. The rAAV for use according to any one of claims 8-14, wherein the protein with CAPN3 activity comprises an amino acid sequence:
a) at least 90%, 91%, 92%, 93%, 94%, 95%, 96%, 97%, 98%, or 99% identical to SEQ ID NO: 5,
b) at least 95% identical to SEQ ID NO: 5; or
c) of SEQ ID NO: 5.

17. The rAAV for use according to any one of claims 8-14, wherein the polynucleotide comprises
a) a sequence which is at least 90%, 91%, 92%, 93%, 94%, 95%, 96%, 97%, 98%, or 99% identical to SEQ ID NO: 1;
b). a sequence at least 95% identical to SEQ ID NO: 1, or
c) the sequence of SEQ ID NO: 1.

18. The rAAV for use according to any one of claims 8-17, wherein the promoter is a muscle-specific promoter.

19. The rAAV for use according to claim 18, wherein the muscle-specific promoter comprises one or more of a human skeletal actin gene element, a cardiac actin gene element, a desmin promoter, a skeletal alpha-actin (ASKA) promoter, a troponin I (TNNI2) promoter, a myocyte-specific enhancer binding factor (MEF) binding element, a muscle creatine kinase (MCK) promoter, a truncated MCK (tMCK) promoter, a myosin heavy chain (MHC) promoter, a hybrid a-myosin heavy chain enhancer-/MCK enhancer-promoter (MHCK7) promoter, a C5-12 promoter, a murine creatine kinase enhancer element, a skeletal fast-twitch troponin C gene element, a slow-twitch cardiac troponin C gene element, a slow-twitch troponin I gene element, hypoxia- inducible nuclear factor (HIF)-response element (HRE), a steroid-inducible element, and a glucocorticoid response element (GRE).

20. The rAAV for use according to any one of claims 8-19, wherein the first and second AAV inverted terminal repeats are AAV2 inverted terminal repeats.

21. The rAAV for use according to any one of claims 1-20, wherein the rAAV comprises one or more of AAV-1, AAV-2, AAV-3, AAV-4, AAV-5, AAV-6, AAV-7, AAV-8, AAV-9, AAV-10, AAV-11, AAV-12, AAV-13, AAV rh.74 and AAV rh.10 capsid proteins.

22. The rAAV for use according to any one of claims 1-21, wherein the subject is
a) a pediatric subject, an adolescent subject or a young adult subject,
b) middle aged adult or elderly subject;
c) 4 to 15 years of age;
d) 15 to 55 years of age; or
e) over 55 years of age.
